# EUROPEAN PATENT SPECIFICATION

(11) **EP 4 240 489 B1**
(45) Date of publication and mention of the grant of the patent: **09.12.2026**
(21) Application number: 21889899.7
(22) Date of filing: 02.11.2021
(51) Int. Cl.: A61P 35/00, C07D 417/14, A61K 31/517

(54) **KRAS G12D INHIBITORS**
KRAS-G12D-HEMMER
INHIBITEURS DE KRAS G12D

(30) Priority: 03.11.2020 US 202063109310 P
(43) Date of publication of application: 13.09.2023
(73) Proprietor: Mirati Therapeutics, Inc., Princeton, New Jersey 08543 (US); Array Biopharma, Inc., Boulder, CO 80301 (US)
(72) Inventor: MARX, Matthew Arnold, San Diego, CA 92121 (US); LAWSON, John David, San Diego, CA 92121 (US); DOERNER BARBAUR, Patrick Michael, Boulder, CO 80301 (US); BLAKE, James Francis, Boulder, CO 80301 (US); FELL, Jay Bradford, Boulder, CO 80301 (US); FISCHER, John Peter, Boulder, CO 80301 (US); NEWHOUSE, Bradley J., Boulder, CO 80301 (US); NGUYEN, Phong, Boulder, CO 80301 (US); O'LEARY, Jacob M., Boulder, CO 80301 (US); PAJK, Spencer, Boulder, CO 80301 (US); RODRIGUEZ, Martha E., Boulder, CO 80301 (US); TANG, Tony Pisal, Boulder, CO 80301 (US)
(74) Representative: Mewburn Ellis LLP
(86) International application number: PCT/US2021/057672
(87) International publication number: WO 2022/098625

(56) References cited:
- WO-A1-2016/164675
- WO-A1-2020/146613
- WO-A1-2021/031952
- WO-A1-2021/041671
- WO-A1-2021/051034
- WO-A1-2021/106231
- WO-A2-2018/218070
- WO-A2-2020/097537
- US-A1- 2021 024 501

## Description

### FIELD OF THE INVENTION

The present invention relates to compounds that inhibit KRas G12D. In particular, the present invention relates to compounds that inhibit the activity of KRas G12D, pharmaceutical compositions comprising the compounds and the compounds and the pharmaceutical compositions for use in therapy.

### BACKGROUND OF THE INVENTION

Kirsten Rat Sarcoma 2 Viral Oncogene Homolog ("KRas") is a small GTPase and a member of the Ras family of oncogenes. KRas serves as a molecular switch cycling between inactive (GDP-bound) and active (GTP-bound) states to transduce upstream cellular signals received from multiple tyrosine kinases to downstream effectors to regulate a wide variety of processes, including cellular proliferation (e.g., see Alamgeer et al., (2013) Current Opin Pharmcol. 13:394-401).

The role of activated KRas in malignancy was observed over thirty years ago (e.g., see Santos et al., (1984) Science 223:661-664). Aberrant expression of KRas accounts for up to 20% of all cancers and oncogenic KRas mutations that stabilize GTP binding and lead to constitutive activation of KRas and downstream signaling have been reported in 25 -30% of lung adenocarcinomas. (e.g., see Samatar and Poulikakos (2014) Nat Rev Drug Disc 13(12): 928-942 doi: 10.103 8/nrd428). Single nucleotide substitutions that result in missense mutations at codons 12 and 13 of the KRas primary amino acid sequence comprise approximately 40% of these KRas driver mutations in lung adenocarcinoma. KRAS G12D mutation is present in 25.0% of all pancreatic ductal adenocarcinoma patients, 13.3% of all colorectal carcinoma patients, 10.1% of all rectal carcinoma patients, 4.1% of all non-small cell lung carcinoma patients and 1.7% of all small cell lung carcinoma patients (e.g., see The AACR Project GENIE Consortium, (2017) Cancer Discovery;7(8):818-831. Dataset Version 4).

The well-known role of KRas in malignancy and the discovery of these frequent mutations in KRas in various tumor types made KRas a highly attractive target of the pharmaceutical industry for cancer therapy. Notwithstanding thirty years of large-scale discovery efforts to develop inhibitors of KRas for treating cancer, no KRas inhibitor has yet demonstrated sufficient safety and/or efficacy to obtain regulatory approval (e.g., see McCormick (2015) Clin Cancer Res. 21 (8):1797-1801).

Compounds that inhibit KRas activity are still highly desirable and under investigation, including those that disrupt effectors such as guanine nucleotide exchange factors (e.g., see Sun et al., (2012) Agnew Chem Int Ed Engl. 51(25):6140-6143 doi: 10.1002/anie201201358) as well recent advances in the covalent targeting of an allosteric pocket of KRas G12C (e.g., see Ostrem et al., (2013) Nature 503:548-551 and Fell et al., (2018) ACS Med. Chem. Lett. 9:1230-1234). Clearly there remains a continued interest and effort to develop inhibitors of KRas, particularly inhibitors of activating KRas mutants, especially KRas G12D. WO 2021041671 A1 and WO 2021106231 A1 describe compounds for inhibiting KRas G12D

Thus, there is a need to develop new KRas G12D inhibitors that demonstrate sufficient efficacy for treating KRas G12D-mediated cancer.

### SUMMARY OF THE INVENTION

The present invention is set out in the appended claims. The present disclosure contains some subject matter that is not explicitly claimed. The claimed compounds, pharmaceutically acceptable salts thereof, and pharmaceutical compositions thereof may be for use in the methods of treatment of the human or animal body by surgery, therapy or diagnosis disclosed herein. In one aspect, compounds are provided that inhibit KRas G12D activity.

In certain embodiments, the compounds are represented by Formula (I): 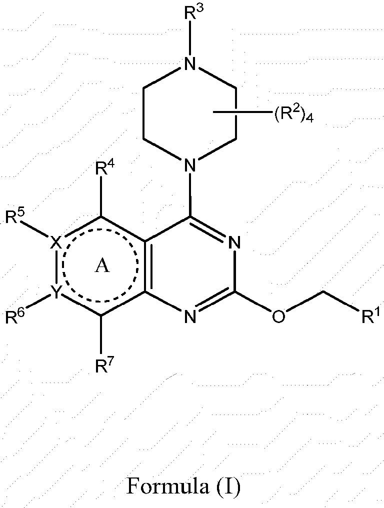 or a pharmaceutically acceptable salt thereof, wherein:
A is phenyl, heteroaryl or heterocyclyl;
X is N, C or CH;
Y is N, C or CH;
where if X is N then Y is not N, and where if Y is N then X is not N; 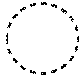
represents one or more optionally-present double bonds;
R¹ is heterocyclyl, optionally substituted with one or more substituents independently selected from: hydroxy, halogen, C1-C3 haloalkyl, C1-C3 alkyl, C1-C3 alkoxy and cyano;
two R² are each independently hydrogen, hydroxy, halogen, C1-C3 haloalkyl, C1-C3 alkyl, C1-C3 alkoxy, (C1-C3 alkoxy)-C1-C3 alkyl, C1-C3 alkyl-N(R⁸)₂, cyano, C1-C3 cyanoalkyl, C2 - C4 cyanoalkenyl, C1 - C3 hydroxyalkyl, HC(=O)-, -CO₂R⁸, or-CO₂N(R⁸)₂, where R⁸ is hydrogen or C1-C3 alkyl, and
two R² on the same carbon atom join to form a cycloalkyl ring, or two R² join to form an ethylene bridge to form a [3.2.1] or [2.2.2] ring system, where said ethylene bridge forms a [2.2.2] ring system if X is N and ring A is heteroaryl, and where said ethylene bridge forms a [2.2.2] ring system if Y is N and ring A is heterocyclyl;
R³ is hydrogen or C1-C3 alkyl optionally substituted with one or more substituents independently selected from: halogen, C1-C3 alkoxy and cyano;
R⁴ is hydrogen;
R⁵ is absent or is selected from hydrogen, halogen, -O-phenyl and -O-pyridyl, where said phenyl and said pyridyl are optionally substituted with one or more substituents independently selected from: hydroxy, halogen, C1-C3 haloalkyl, C1-C3 alkyl, C1-C3 alkoxy and cyano;
R⁶ is phenyl, naphthyl or indazolyl, optionally substituted with one or more substituents independently selected from: hydroxy, halogen, C1-C3 haloalkyl, C1-C3 alkyl, C1-C3 alkoxy and cyano; and
R⁷ is hydrogen or halogen.

In certain embodiments, the compounds of Formula (I) are represented by Formula (I-A): 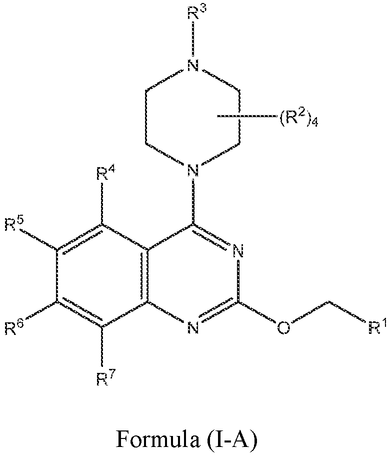 or a pharmaceutically acceptable salt thereof, wherein R¹, R², R³, R⁴, R⁵, R⁶ and R⁷ represent the chemical moieties as described above in Formula (I).

In certain embodiments, the compounds of Formula (I) are represented by Formula (I-B): 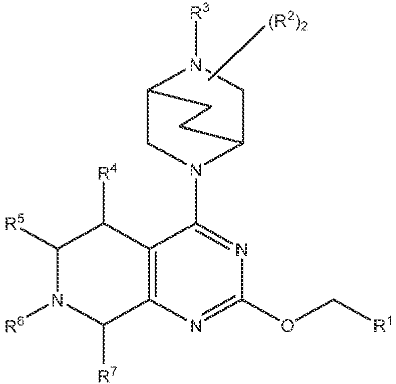 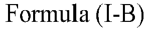 or a pharmaceutically acceptable salt thereof, wherein R¹, R², R³, R⁴, R⁵, R⁶ and R⁷ represent the chemical moieties as described above in Formula (I).

In another aspect of the invention, pharmaceutical compositions are provided comprising a therapeutically effective amount of a compound of the present invention or a pharmaceutically acceptable salt thereof and a pharmaceutically acceptable excipient.

In yet another aspect, methods for inhibiting KRas G12D activity in a in a cell, comprising contacting the cell with a compound of Formula (I), or a pharmaceutically acceptable salt thereof, or a pharmaceutical composition thereof as defined herein. In one embodiment, the contacting is in vitro. In one embodiment, the contacting is in vivo.

Also provided herein is a method of inhibiting cell proliferation, in vitro or in vivo, the method comprising contacting a cell with an effective amount of a compound of Formula (I), or a pharmaceutically acceptable salt thereof, or a pharmaceutical composition thereof as defined herein.

Also provided are methods for treating cancer in a patient comprising administering a therapeutically effective amount of a compound or pharmaceutical composition of the present invention or a pharmaceutically acceptable salt thereof to a patient in need thereof.

Also provided herein is a method of treating a KRas G12D-associated disease or disorder in a patient in need of such treatment, the method comprising administering to the patient a therapeutically effective amount of a compound of Formula (I), or a pharmaceutically acceptable salt thereof, or a pharmaceutical composition thereof as defined herein.

Also provided herein is a compound of Formula (I), or a pharmaceutically acceptable salt thereof, or a pharmaceutical composition thereof as defined herein for use in therapy.

Also provided herein is a compound of Formula (I), or a pharmaceutically acceptable salt thereof or a pharmaceutical composition thereof as defined herein for use in the treatment of cancer.

Also provided herein is a compound of Formula (I), or a pharmaceutically acceptable salt thereof for use in the inhibition of KRas G12D.

Also provided herein is a compound of Formula (I), or a pharmaceutically acceptable salt thereof or a pharmaceutical composition thereof as defined herein, for use in the treatment of a KRas G12D-associated disease or disorder.

Also provided herein is the use of a compound of Formula (I), or a pharmaceutically acceptable salt thereof, as defined herein in the manufacture of a medicament for the treatment of cancer.

Also provided herein is a use of a compound of Formula (I), or a pharmaceutically acceptable salt thereof, as defined herein in the manufacture of a medicament for the inhibition of activity of KRas G12D.

Also provided herein is the use of a compound of Formula (I), or a pharmaceutically acceptable salt thereof, as defined herein, in the manufacture of a medicament for the treatment of a KRas G12D-associated disease or disorder.

Also provided herein is a method for treating cancer in a patient in need thereof, the method comprising (a) determining that the cancer is associated with a KRas G12D mutation (i.e., a KRas G12D-associated cancer); and (b) administering to the patient a therapeutically effective amount of a compound of Formula (I), or a pharmaceutically acceptable salt thereof, or a pharmaceutical composition thereof.

Also provided herein is a process for preparing a compound of Formula (I), or a pharmaceutically acceptable salt thereof.

Also provided herein is a compound of Formula (I), or a pharmaceutically acceptable salt thereof obtained by a process of preparing the compound as defined herein.

### DETAILED DESCRIPTION OF THE INVENTION

The present invention relates to inhibitors of KRas G12D. In particular, the present invention relates to compounds that inhibit the activity of KRas G12D, pharmaceutical compositions comprising a therapeutically effective amount of the compounds and methods of use therefor.

### DEFINITIONS

Unless defined otherwise, all technical and scientific terms used herein have the same meaning as is commonly understood by one of skill in the art to which this invention belongs.

As used herein, "KRas G12D" refers to a mutant form of a mammalian KRas protein that contains an amino acid substitution of an aspartic acid for a glycine at amino acid position 12. The assignment of amino acid codon and residue positions for human KRas is based on the amino acid sequence identified by UniProtKB/Swiss-Prot P01116: Variantp.Gly12Asp.

As used herein, a "KRas G12D inhibitor" refers to compounds of the present invention that are represented by Formula (I), as described herein. These compounds are capable of negatively modulating or inhibiting all or a portion of the enzymatic activity of KRas G12D.

A "KRas G12D-associated disease or disorder" as used herein refers to diseases or disorders associated with or mediated by or having a KRas G12D mutation. A non-limiting example of a KRas G12D-associated disease or disorder is a KRas G12D-associated cancer.

As used herein, the term "subject," "individual," or "patient," used interchangeably, refers to any animal, including mammals such as mice, rats, other rodents, rabbits, dogs, cats, swine, cattle, sheep, horses, primates, and humans. In some embodiments, the patient is a human. In some embodiments, the subject has experienced and/or exhibited at least one symptom of the disease or disorder to be treated and/or prevented. In some embodiments, the subject has been identified or diagnosed as having a cancer having a KRas G12D mutation (e.g., as determined using a regulatory agency-approved, e.g., FDA-approved, assay or kit). In some embodiments, the subject has a tumor that is positive for a KRas G12D mutation (e.g., as determined using a regulatory agency-approved assay or kit). The subject can be a subject with a tumor(s) that is positive for a KRas G12D mutation (e.g., identified as positive using a regulatory agency-approved, e.g., FDA-approved, assay or kit). The subject can be a subject whose tumors have a KRas G12D mutation (e.g., where the tumor is identified as such using a regulatory agency-approved, e.g., FDA-approved, kit or assay). In some embodiments, the subject is suspected of having a KRas G12D gene-associated cancer. In some embodiments, the subject has a clinical record indicating that the subject has a tumor that has a KRas G12D mutation (and optionally the clinical record indicates that the subject should be treated with any of the compositions provided herein).

In some embodiments of any of the methods or uses described herein, an assay is used to determine whether the patient has KRas G12D mutation using a sample (e.g., a biological sample or a biopsy sample (e.g., a paraffin-embedded biopsy sample) from a patient (e.g., a patient suspected of having a KRas G12D-associated cancer, a patient having one or more symptoms of a KRas G12D-associated cancer, and/or a patient that has an increased risk of developing a KRas G12D-associated cancer) can include, for example, next generation sequencing, immunohistochemistry, fluorescence microscopy, break apart FISH analysis, Southern blotting, Western blotting, FACS analysis, Northern blotting, and PCR-based amplification (e.g., RT-PCR and quantitative real-time RT-PCR). As is well-known in the art, the assays are typically performed, e.g., with at least one labelled nucleic acid probe or at least one labelled antibody or antigen-binding fragment thereof.

The term "regulatory agency" is a country's agency for the approval of the medical use of pharmaceutical agents with the country. For example, a non-limiting example of a regulatory agency is the U.S. Food and Drug Administration (FDA).

The terms "C1-C6 alkyl", "C1-C4 alkyl" and "C1-C3 alkyl" as employed herein refers to straight and branched chain aliphatic groups having from 1-6 carbon atoms, or 1-4 carbon atoms, or 1-3 carbon atoms, respectively. Examples of alkyl groups include, without limitation, methyl, ethyl, propyl, isopropyl, butyl, isobutyl, sec-butyl, tert-butyl, pentyl, and hexyl.

The term "alkenyl" as used herein means an unsaturated straight or branched chain aliphatic group with one or more carbon-carbon double bonds, having from 2 to 12 carbon atoms. As such, "alkenyl" encompasses C2, C3, C4, C5, C6, C7, C8, C9, C10, C11 and C12 groups. Examples of alkenyl groups include, without limitation, ethenyl, propenyl, butenyl, pentenyl, and hexenyl. "C2-C4 alkenyl" for example refers to ethene, propene and butene(s).

The terms "C1-C3 haloalkyl", "C1-C4 haloalkyl" and C1-C6 haloalkyl" refer to a C1-C3 alkyl, a C1-C4 alkyl or a C1-C6 alkyl, respectively, as defined herein in which one or more hydrogen has been replaced by a halogen. Examples include trifluoromethyl, difluoromethyl and fluoromethyl.

An "C1-C3 alkylene," group is a C1-C3 alkyl group, as defined hereinabove, that is positioned between and serves to connect two other chemical groups. Exemplary alkylene groups include, without limitation, methylene, ethylene and propylene.

The terms "C1-C3 alkoxy" and "C1 - C4 alkoxy" refer to -OC1-C3 alkyl and -OC1-C4 alkyl, respectively, wherein the alkyl portion is as defined herein above.

The term "cycloalkyl" as employed herein includes saturated and partially unsaturated cyclic hydrocarbon groups having 3 to 12 carbons, for example 3 to 8 carbons, and as a further example 3 to 6 carbons, wherein the cycloalkyl group additionally is optionally substituted with one or more R⁶ groups as defined herein. Examples of cycloalkyl groups include, without limitation, cyclopropyl, cyclobutyl, cyclopentyl, cyclopentenyl, cyclohexyl, cyclohexenyl, cycloheptyl, and cyclooctyl. The term "cycloalkyl" also includes bridged cycloalkyls, such as bicyclo[1.1.1]pentanyl.

As used herein, the terms "C1-C3 hydroxyalkyl" and "C1-C4 hydroxyalkyl" refer to -C1-C3 alkylene-OH and -C1-C4 alkylene-OH, respectively

A "heterocyclyl" or "heterocyclic" group is a ring structure having from 3 to 12 atoms, for example 4 to 8 atoms, wherein one or more atoms are selected from the group consisting of N, O, and S wherein the ring N atom may be oxidized to N-O, and the ring S atom may be oxidized to SO or SO₂, the remainder of the ring atoms being carbon. The heterocyclyl may be a monocyclic, a bicyclic, a spirocyclic or a bridged ring system. Examples of heterocyclic groups include, without limitation, epoxy, azetidinyl, aziridinyl, tetrahydrofuranyl, tetrahydropyranyl, pyrrolidinyl, pyrrolidinonyl, piperidinyl, piperazinyl, imidazolidinyl, imidazopyridinyl, thiazolidinyl, dithianyl, trithianyl, dioxolanyl, oxazolidinyl, oxazolidinonyl, decahydroquinolinyl, piperidonyl, 4-piperidinonyl, quinuclidinyl, thiomorpholinyl, thiomorpholinyl 1,1 dioxide, morpholinyl, azepanyl, oxazepanyl, azabicyclohexanyls, azabicycloheptanyl, azabicyclooctanyls, azabicyclononanyls (e.g., octahydroindolizinyl), azaspiroheptanyls, dihydro-1H,3H,5H-oxazolo[3,4-c]oxazolyl, tetrahydro-1'H,3'H-spiro[cyclopropane-1,2'-pyrrolizine], hexahydro-1H-pyrrolizinyl, hexahydro-1H-pyrrolo[2,1-c][1,4]oxazinyl, octahydroindolizinyl, oxaazaspirononanyls, oxaazaspirooctanyls, diazaspirononanyls, oxaazabiocycloheptanyls, hexahydropyrrolizinyl 4(1H)-oxide, tetrahydro-2H-thiopyranyl 1-oxide and tetrahydro-2H-thiopyranyl 1,1-dioxide. The heterocyclic group is also optionally substituted. For example, the heterocyclic group may optionally substituted with one or more substituents independently selected from: hydroxy, halogen, C1-C3 haloalkyl, C1-C3 alkyl, C1-C3 alkoxy and cyano.

As used herein, the term "heteroaryl" refers to groups having 5 to 14 ring atoms, preferably 5, 6, 9, or 10 ring atoms; having 6, 10, or 14 π electrons shared in a cyclic array having one to three heteroaromatic rings; and having, in addition to carbon atoms, from one to three heteroatoms in at least one ring selected from the group consisting of N, O, and S. Examples of heteroaryl groups include acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazolinyl, carbazolyl, 4aH-carbazolyl, carbolinyl, chromanyl, chromenyl, cinnolinyl, 6,7-dihydro-5H-pyrrolo[1,2-a]imidazole, furanyl, furazanyl, imidazolinyl, imidazolyl, 1H-indazolyl, indolenyl, indolinyl, indolizinyl, indolyl, 3H-indolyl, isobenzofuranyl, isochromanyl, isoindazolyl, isoindolinyl, isoindolyl, isoquinolinyl, isothiazolyl, isoxazolyl, methylenedioxyphenyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, pyrimidinyl, phenanthridinyl, phenanthrolinyl, phenazinyl, phenothiazinyl, phenoxathiinyl, phenoxazinyl, phthalazinyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolinyl, 2H-pyrrolyl, pyrrolyl, quinazolinyl, quinolinyl, 4H-quinolizinyl, quinoxalinyl, quinuclidinyl, tetrahydroisoquinolinyl, tetrahydroquinolinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl. "Heteroaryl" also refers to bicyclic ring systems having, in addition to carbon atoms, from one to three heteroatoms in at least one aromatic ring selected from the group consisting of N, O, and S in which one ring in the bicyclic ring system may be saturated or partially saturated. "Heteroaryl" ring systems are optionally substituted as defined herein.

As used herein, "an effective amount" of a compound is an amount that is sufficient to negatively modulate or inhibit the activity of KRas G12D. Such amount may be administered as a single dosage or may be administered according to a regimen, whereby it is effective.

As used herein, a "therapeutically effective amount" of a compound is an amount that is sufficient to ameliorate, or in some manner reduce a symptom or stop or reverse progression of a condition, or negatively modulate or inhibit the activity of KRas G12D. Such amount may be administered as a single dosage or may be administered according to a regimen, whereby it is effective.

As used herein, treatment means any manner in which the symptoms or pathology of a condition, disorder or disease are ameliorated or otherwise beneficially altered. Treatment also encompasses any pharmaceutical use of the compositions herein.

As used herein, amelioration of the symptoms of a particular disorder by administration of a particular pharmaceutical composition refers to any lessening, whether permanent or temporary, lasting or transient that can be attributed to or associated with administration of the composition.

### COMPOUNDS

In one aspect, compounds are provided represented by Formula (I): 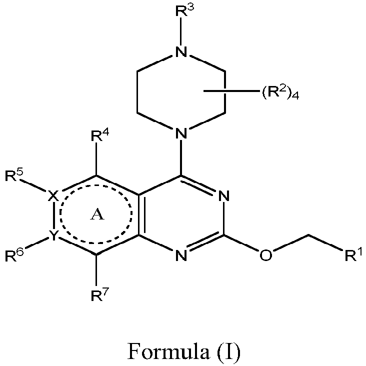 or a pharmaceutically acceptable salt thereof, wherein:
A is phenyl, heteroaryl or heterocyclyl;
X is N, C or CH;
Y is N, C or CH;
where if X is N then Y is not N, and where if Y is N then X is not N; 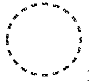
represents one or more optionally-present double bonds;
R¹ is heterocyclyl, optionally substituted with one or more substituents independently selected from: hydroxy, halogen, C1-C3 haloalkyl, C1-C3 alkyl, C1-C3 alkoxy and cyano;
two R² are each independently hydrogen, hydroxy, halogen, C1-C3 haloalkyl, C1-C3 alkyl, C1-C3 alkoxy, (C1-C3 alkoxy)-C1-C3 alkyl, C1-C3 alkyl-N(R⁸)₂, cyano, C1-C3 cyanoalkyl, C2 - C4 cyanoalkenyl, C1 - C3 hydroxyalkyl, HC(=O)-, -CO₂R⁸, or -CO₂N(R⁸)₂, where R⁸ is hydrogen or C1-C3 alkyl, and
two R² on the same carbon atom join to form a cycloalkyl ring, or two R² join to form an ethylene bridge to form a [3.2.1] or [2.2.2] ring system, where said ethylene bridge forms a [2.2.2] ring system if X is N and ring A is heteroaryl, and where said ethylene bridge forms a [2.2.2] ring system if Y is N and ring A is heterocyclyl;
R³ is hydrogen or C1-C3 alkyl optionally substituted with one or more substituents independently selected from: halogen, C1-C3 alkoxy and cyano;
R⁴ is hydrogen;
R⁵ is absent or is selected from hydrogen, halogen, -O-phenyl and -O-pyridyl, where said phenyl and said pyridyl are optionally substituted with one or more substituents independently selected from: hydroxy, halogen, C1-C3 haloalkyl, C1-C3 alkyl, C1-C3 alkoxy and cyano;
R⁶ is phenyl, naphthyl or indazolyl, optionally substituted with one or more substituents independently selected from: hydroxy, halogen, C1-C3 haloalkyl, C1-C3 alkyl, C1-C3 alkoxy and cyano; and
R⁷ is hydrogen or halogen.

In certain embodiments, the compounds of Formula (I) are represented by Formula (I-A): 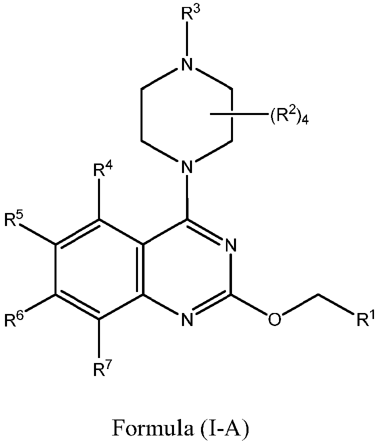 or a pharmaceutically acceptable salt thereof, wherein R¹, R², R³, R⁴, R⁵, R⁶ and R⁷ represent the chemical moieties as described above in Formula (I).

In certain embodiments, the compounds of Formula (I) are represented by Formula (I-B): 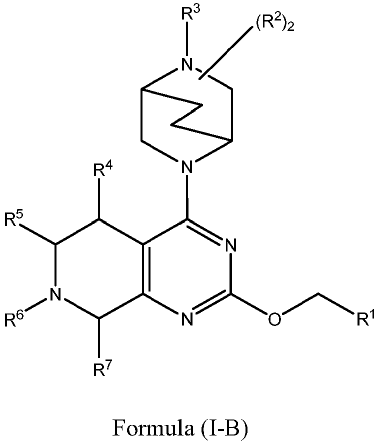 or a pharmaceutically acceptable salt thereof, wherein R¹, R², R³, R⁴, R⁵, R⁶ and R⁷ represent the chemical moieties as described above in Formula (I).

In certain embodiments R¹ is heterocyclyl.

In certain of those embodiments, R¹ is heterocyclyl optionally substituted with one or more substituents independently selected from: hydroxy, halogen, C1-C3 haloalkyl, C1-C3 alkyl, C1-C3 alkoxy and cyano.

In certain of these embodiments, the R¹ heterocyclyl is hexahydro-1H-pyrrolizinyl, optionally substituted with a halogen. In certain of these embodiments, the halogen is fluorine. In certain of these embodiments, the substituted heterocycle is 2-fluorohexahydro-1H-pyrrolizinyl.

In certain of these embodiments the R¹ heterocyclyl is pyrrolidine, optionally substituted with C1-C3 alkyl. In certain of these embodiments, the C1-C3 alkyl is methyl. In certain of these embodiments, the substituted heterocycle is 1-methylpyrrolidine.

In certain embodiments, each R² is independently hydrogen, hydroxy, halogen, C1-C3 haloalkyl, C1 - C3 alkyl, C1-C3 alkoxy, (C1-C3 alkoxy)-C1-C3 alkyl, , C1-C3 alkyl-N(R⁸)2, cyano, *C1-C3* cyanoalkyl, C2 - C4 cyanoalkenyl, C1 - C3 hydroxyalkyl, HC(=O)-, -CO₂R⁸, or - CO₂N(R⁸)₂, where two R² join to form an ethylene bridge to form a [3.2.1] or [2.2.2] ring system, where said ethylene bridge forms a [2.2.2] ring system if X is N and ring A is heteroaryl, and where said ethylene bridge is in a [2.2.2] ring system if Y is N and ring A is heterocyclyl.

In certain embodiments two R² on the same carbon atom join to form a cycloalkyl ring, for instance a cyclopropyl ring.

In certain embodiments two R² join to form a C1-C3 alkyl bridge to form a [3.2.1] or [2.2.2] ring system, where said C1-C3 alkyl bridge forms a [2.2.2] ring system if X is N and ring A is heteroaryl, and where said C1-C3 alkyl bridge forms a [2.2.2] ring system if Y is N and ring A is heterocyclyl. In certain of those embodiments, the C1-C3 alkyl bridge forms a [2.2.2] ring system, and in certain of those embodiments X is N and A is heteroaryl, and the C1-C3 alkyl bridge forms a [2.2.2] ring system. In certain other embodiments Y is N and ring A is heterocyclyl, and the C1-C3 alkyl bridge forms a [2.2.2] ring system. In certain other embodiments X and Y are both C or CH, and the C1-C3 alkyl bridge forms a [3.2.1] or a [2.2.2] ring system.

In certain embodiments, X is N, C or CH. In certain embodiments Y is N, C or CH. In embodiments X and Y cannot both be N in the same compound, and therefore in certain embodiments when X is N then Y is not N, while in certain other embodiments when Y is N then X is not N.

In certain embodiments R³ is hydrogen or C1-C3 alkyl optionally substituted with one or more substituents independently selected from: halogen, C1-C3 alkoxy and cyano.

In certain embodiments R³ is hydrogen.

In certain embodiments R³ is C1-C3 alkyl optionally substituted with one or more substituents independently selected from: halogen, C1-C3 alkoxy and cyano.

In certain of these embodiments R³ is methyl. In certain of these embodiments R³ is ethyl. In certain embodiments where R³ is methyl or ethyl, R³ is substituted with halogen. In certain of these embodiments the halogen is chlorine. In certain of these embodiments the halogen is fluorine.

In certain embodiments, R⁴ is hydrogen.

In certain embodiments R⁵ is absent or is selected from hydrogen, halogen, -O-phenyl and -O-pyridyl, where said phenyl and said pyridyl are optionally substituted with one or more substituents independently selected from: hydroxy, halogen, C1-C3 haloalkyl, C1-C3 alkyl, C1-C3 alkoxy and cyano.

In certain embodiments R⁵ is -O-phenyl. In certain of these embodiments, said phenyl is substituted with halogen, C1-C3 alkoxy, or both halogen and C1-C3 alkoxy. In certain of these embodiments said phenyl is substituted with a halogen such as fluorine or chlorine, and methoxy. In certain of these embodiments said phenyl is substituted with a halogen such as fluorine or chlorine.

In certain embodiments R⁵ is -O-phenyl where said phenyl is substituted with cyano.

In certain embodiments R⁵ is absent.

In certain embodiments R⁵ is hydrogen.

In certain embodiments, R⁵ is -O-pyridyl.

In certain embodiments, R⁶ is phenyl, naphthyl or indazolyl, optionally substituted with one or more substituents independently selected from: hydroxy, halogen, C1-C3 haloalkyl, C1-C3 alkyl, C1-C3 alkoxy and cyano.

In certain embodiments, R⁶ is phenyl. In certain of these embodiments, said phenyl is substituted with halogen, hydroxy, or both halogen and hydroxy.

In certain embodiments, R⁶ is naphthyl. In certain of these embodiments, said naphthyl is substituted with halogen, hydroxy, or both halogen and hydroxy.

In certain embodiments, R⁶ is indazolyl. In certain of these embodiments, said indazolyl is substituted with one or more C1-C3 alkyl.

In certain embodiments, R⁷ is hydrogen.

In certain embodiments, R⁷ is halogen.

In certain embodiments the present invention provides a pharmaceutical composition comprising a therapeutically effective amount of any of the compounds described herein, or a pharmaceutically acceptable salt thereof, and a pharmaceutically acceptable excipient.

Embodiments also include a method for inhibiting KRas G12D activity in a cell, comprising contacting the cell in which inhibition of Kras G12D activity is desired with an effective amount of a compound of any compound described herein, or a pharmaceutically acceptable salt thereof, or related pharmaceutical compositions described herein.

There is an embodiment which is a method for treating cancer comprising administering to a patient having cancer a therapeutically effective amount of a compound described herein, or a pharmaceutically acceptable salt thereof, or a pharmaceutical composition described herein. In certain embodiments that cancer is a Kras G12D-associated cancer. In certain embodiments, that cancer is non-small cell lung cancer, small cell lung cancer, colorectal cancer, rectal cancer or pancreatic cancer.

In certain embodiments, a compound for cancer treatment described herein is provided in a therapeutically effective amount of between about 0.01 to 100 mg/kg per day. In certain other embodiments, the therapeutically effective amount of the compound is between about 0.1 to 50 mg/kg per day.

There is an embodiment which is a method for treating cancer in a patient in need thereof, the method comprising (a) determining that the cancer is associated with a Kras G12D mutation (e.g., a Kras G12D-associated cancer); and (b) administering to the patient a therapeutically effective amount of a compound as described herein, or a pharmaceutically acceptable salt thereof, or a pharmaceutical composition described herein.

Nonlimiting examples of compounds of Formula (I) are: 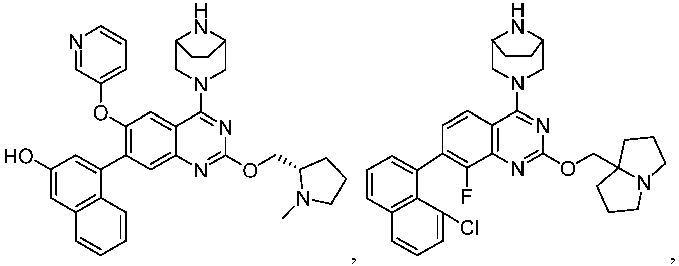 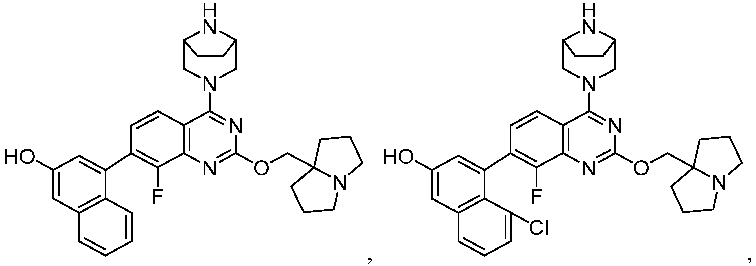 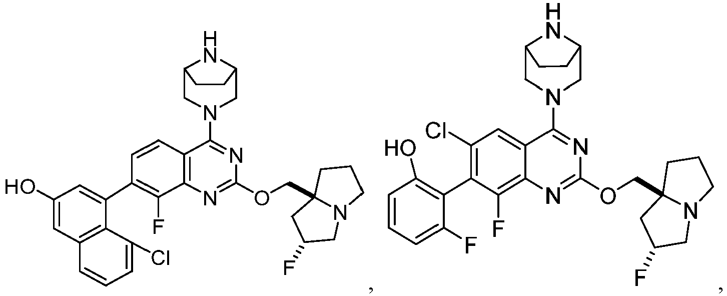 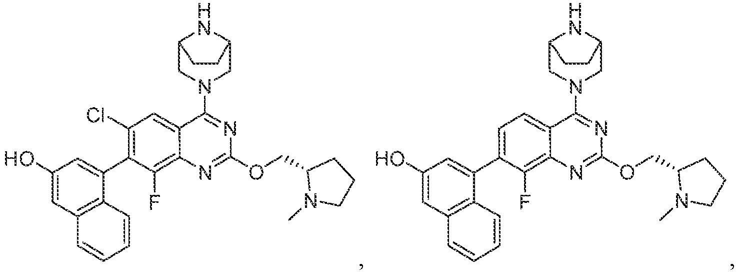 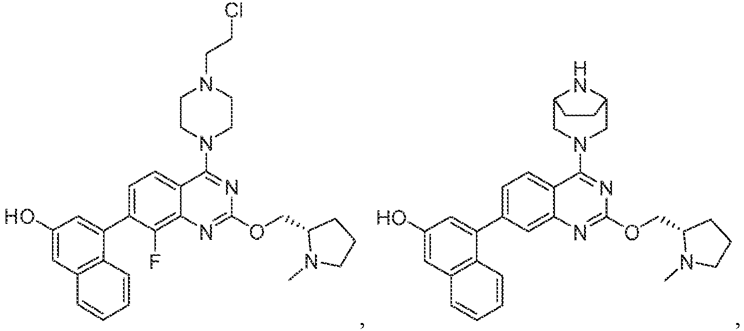 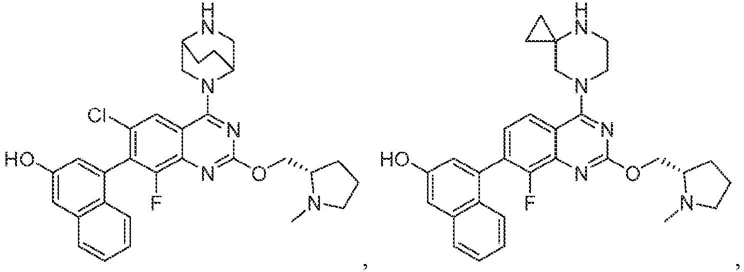 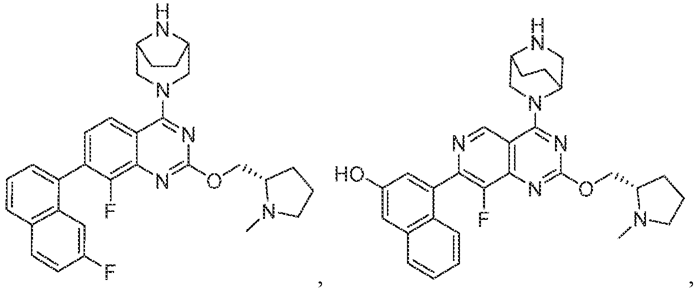 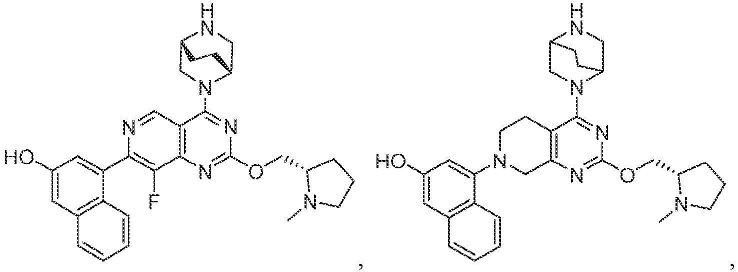 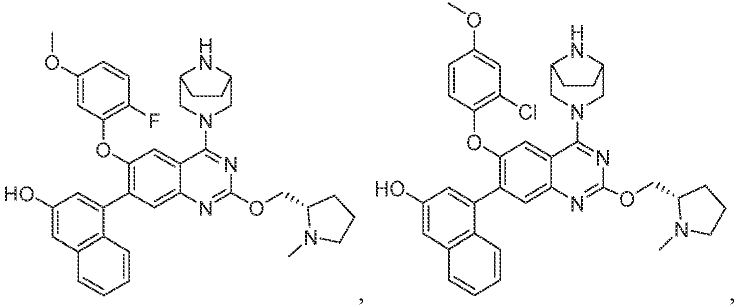 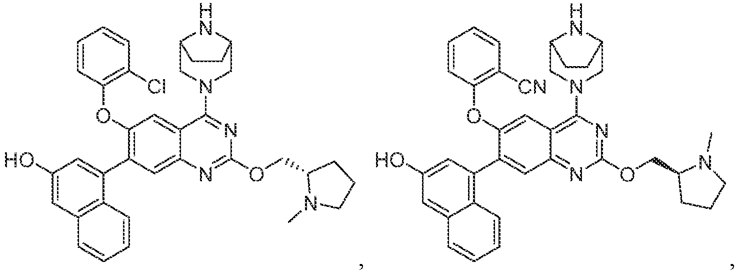 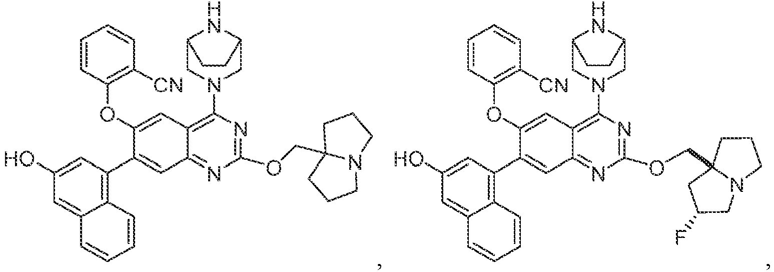 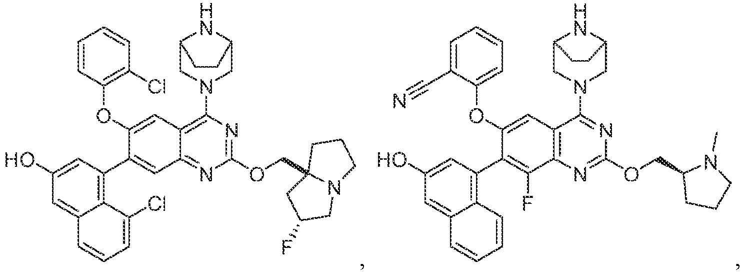 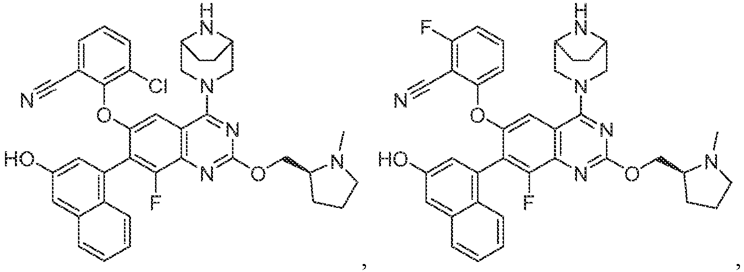 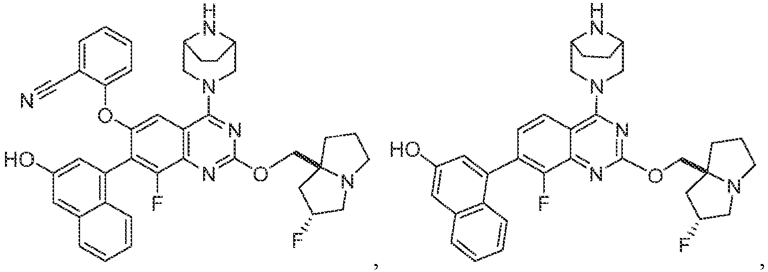 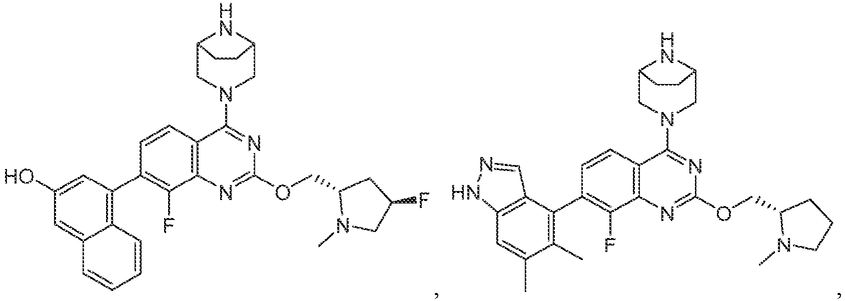 and 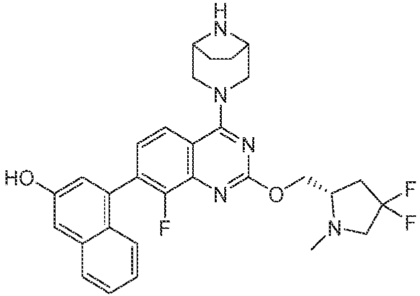 and pharmaceutically acceptable salts thereof.

### PHARMACEUTICAL COMPOSITIONS

In another aspect, the invention provides pharmaceutical compositions comprising a Kras G12D inhibitor according to the invention and a pharmaceutically acceptable carrier, excipient, or diluent. Compounds of the invention may be formulated by any method well known in the art and may be prepared for administration by any route, including, without limitation, parenteral, oral, sublingual, transdermal, topical, intranasal, intratracheal, or intrarectal. In certain embodiments, compounds of the invention are administered intravenously in a hospital setting. In one embodiment, administration may be by the oral route.

The characteristics of the carrier will depend on the route of administration. As used herein, the term "pharmaceutically acceptable" means a non-toxic material that is compatible with a biological system such as a cell, cell culture, tissue, or organism, and that does not interfere with the effectiveness of the biological activity of the active ingredient(s). Thus, compositions according to the invention may contain, in addition to the inhibitor, diluents, fillers, salts, buffers, stabilizers, solubilizers, and other materials well known in the art. The preparation of pharmaceutically acceptable formulations is described in, e.g., Remington's Pharmaceutical Sciences, 18th Edition, ed. A. Gennaro, Mack Publishing Co., Easton, Pa., 1990.

As used herein, the term pharmaceutically acceptable salt refers to salts that retain the desired biological activity of the above-identified compounds and exhibit minimal or no undesired toxicological effects. Examples of such salts include, but are not limited to acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acid, naphthalenedisulfonic acid, and polygalacturonic acid. The compounds can also be administered as pharmaceutically acceptable quaternary salts known by those skilled in the art, which specifically include the quaternary ammonium salt of the formula -NR+Z-, wherein R is hydrogen, alkyl, or benzyl, and Z is a counterion, including chloride, bromide, iodide, -O-alkyl, toluenesulfonate, methylsulfonate, sulfonate, phosphate, or carboxylate (such as benzoate, succinate, acetate, glycolate, maleate, malate, citrate, tartrate, ascorbate, benzoate, cinnamoate, mandeloate, benzyloate, and diphenylacetate).

The active compound is included in the pharmaceutically acceptable carrier or diluent in an amount sufficient to deliver to a patient a therapeutically effective amount without causing serious toxic effects in the patient treated. In one embodiment, a dose of the active compound for all of the above-mentioned conditions is in the range from about 0.01 to 300 mg/kg, for example 0.1 to 100 mg/kg per day, and as a further example 0.5 to about 25 mg per kilogram body weight of the recipient per day. A typical topical dosage will range from 0.01-3% wt/wt in a suitable carrier. The effective dosage range of the pharmaceutically acceptable derivatives can be calculated based on the weight of the parent compound to be delivered. If the derivative exhibits activity in itself, the effective dosage can be estimated as above using the weight of the derivative, or by other means known to those skilled in the art.

### METHODS OF USE

The pharmaceutical compositions comprising compounds of the present invention may be used in the methods of use described herein.

In yet another aspect, there are methods for inhibiting Kras G12D activity in a cell, comprising contacting the cell in which inhibition of Kras G12D activity is desired with an effective amount of a compound of Formula (I), pharmaceutically acceptable salts thereof, or pharmaceutical compositions containing the compound or pharmaceutically acceptable salt thereof. In one embodiment, the contacting is in vitro. In one embodiment, the contacting is in vivo.

As used herein, the term "contacting" refers to the bringing together of indicated moieties in an in vitro system or an in vivo system. For example, "contacting" a Kras G12D with a compound provided herein includes the administration of a compound provided herein to an individual or patient, such as a human, having Kras G12D, as well as, for example, introducing a compound provided herein into a sample containing a cellular or purified preparation containing the Kras G12D.

In one embodiment, a cell in which inhibition of Kras G12D activity is desired is contacted with an effective amount of a compound of Formula (I) or pharmaceutically acceptable salt thereof to negatively modulate the activity of Kras G12D.

By negatively modulating the activity of Kras G12D, the methods described herein are designed to inhibit undesired cellular proliferation resulting from enhanced Kras G12D activity within the cell. The cells may be contacted in a single dose or multiple doses in accordance with a particular treatment regimen to effect the desired negative modulation of Kras G12D. The ability of compounds to bind Kras G12D may be monitored in vitro using well known methods, including those described in Examples A and B below. In addition, the inhibitory activity of exemplary compounds in cells may be monitored, for example, by measuring the inhibition of Kras G12D activity of the amount of phosphorylated ERK, for example using the method described in Example C below.

In another aspect, methods of treating cancer in a patient in need thereof, comprising administering to said patient a therapeutically effective amount of a compound of Formula (I) or a pharmaceutically acceptable salt thereof, or a pharmaceutical composition comprising the compound or pharmaceutically acceptable salt thereof are provided.

The compositions and methods provided herein may be used for the treatment of a Kras G12D-associated cancer in a patient in need thereof, comprising administering to said patient a therapeutically effective amount of a compound of Formula (I), a pharmaceutically acceptable salt thereof, or a pharmaceutical composition comprising the compound or pharmaceutically acceptable salt thereof are provided. In one embodiment, the Kras G12D-associated cancer is lung cancer.

The compositions and methods provided herein may be used for the treatment of a wide variety of cancers including tumors such as lung, prostate, breast, brain, skin, cervical carcinomas, testicular carcinomas, etc. More particularly, cancers that may be treated by the compositions and methods herein include, but are not limited to tumor types such as astrocytic, breast, cervical, colorectal, endometrial, esophageal, gastric, head and neck, hepatocellular, laryngeal, lung, oral, ovarian, prostate and thyroid carcinomas and sarcomas. More specifically, these compounds can be used to treat: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Biliary tract: gall bladder carcinoma, ampullary carcinoma, cholangiocarcinoma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); Hematologic: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); Skin: malignant melanoma, basal cell carcinoma, squamous cell carcinoma, Kaposi's sarcoma, moles dysplastic nevi, lipoma, angioma, dermatofibroma, keloids, psoriasis; and Adrenal glands: neuroblastoma. In certain embodiments, the cancer is non-small cell lung cancer, small cell lung cancer, colorectal cancer, rectal cancer or pancreatic cancer. In certain embodiments, the cancer is non-small cell lung cancer.

The concentration and route of administration to the patient will vary depending on the cancer to be treated. The compounds, pharmaceutically acceptable salts thereof and pharmaceutical compositions comprising such compounds and salts also may be co-administered with other anti-neoplastic compounds, e.g., chemotherapy, or used in combination with other treatments, such as radiation or surgical intervention, either as an adjuvant prior to surgery or postoperatively.

Also provided herein is a compound of Formula (I) or a pharmaceutically acceptable salt thereof, or a pharmaceutical composition thereof as defined herein for use in therapy.

Also provided herein is a compound of Formula (I) or a pharmaceutically acceptable salt thereof, or a pharmaceutical composition thereof as defined herein for use in the treatment of cancer.

Also provided herein is a compound of Formula (I) or a pharmaceutically acceptable salt thereof, or a pharmaceutical composition thereof for use in the inhibition of Kras G12D.

Also provided herein is a compound of Formula (I) or a pharmaceutically acceptable salt thereof, or a pharmaceutical composition thereof as defined herein, for use in the treatment of a Kras G12D-associated disease or disorder.

Also provided herein is the use of a compound of Formula (I) or a pharmaceutically acceptable salt thereof, as defined herein in the manufacture of a medicament for the treatment of cancer.

Also provided herein is a use of a compound of Formula (I) or a pharmaceutically acceptable salt thereof, as defined herein in the manufacture of a medicament for the inhibition of activity of Kras G12D.

Also provided herein is the use of a compound of Formula (I) or a pharmaceutically acceptable salt thereof, as defined herein, in the manufacture of a medicament for the treatment of a Kras G12D-associated disease or disorder.

Also provided herein is a method for treating cancer in a patient in need thereof, the method comprising (a) determining that cancer is associated with a Kras G12D mutation (e.g., a Kras G12D-associated cancer) (e.g., as determined using a regulatory agency-approved, e.g., FDA-approved, assay or kit); and (b) administering to the patient a therapeutically effective amount of a compound of Formula (I), or a pharmaceutically acceptable salt thereof, or a pharmaceutical composition thereof.

One skilled in the art will recognize that, both in vivo and in vitro trials using suitable, known and generally accepted cell and/or animal models are predictive of the ability of a test compound to treat or prevent a given disorder.

One skilled in the art will further recognize that human clinical trials including first-inhuman, dose ranging and efficacy trials, in healthy patients and/or those suffering from a given disorder, may be completed according to methods well known in the clinical and medical arts.

### REACTION SCHEMES AND EXAMPLES

The compounds of the present invention may be prepared from commercially available reagents using the synthetic methods and reaction schemes described herein, or using other reagents and conventional methods well known to those skilled in the art.

The compounds of the present invention may have one or more chiral center and may be synthesized as stereoisomeric mixtures, isomers of identical constitution that differ in the arrangement of their atoms in space. The compounds may be used as mixtures or the individual components/isomers may be separated using commercially available reagents and conventional methods for isolation of stereoisomers and enantiomers well-known to those skilled in the art, e.g., using CHIRALPAK^{®} (Sigma-Aldrich) or CHIRALCEL^{®} (Diacel Corp) chiral chromatographic HPLC columns according to the manufacturer's instructions. Alternatively, compounds of the present invention may be synthesized using optically pure, chiral reagents and intermediates to prepare individual isomers or enantiomers. Unless otherwise indicated, all chiral (enantiomeric and diastereomeric) and racemic forms are contemplated herein. Unless otherwise indicated, whenever the specification, including the claims, refers to compounds of the invention, the term "compound" is to be understood to encompass all chiral (enantiomeric and diastereomeric) and racemic forms.

The compounds of the present invention may be in anhydrous, solvated or hydrated forms.

The following Intermediates are intended to illustrate further certain embodiments of the invention.

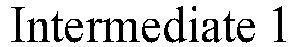

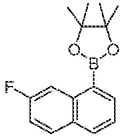

### 2-(7-fluoronaphthalen-1-yl)-4,4,5,5-tetramethyl-1,3,2-dioxaborolane

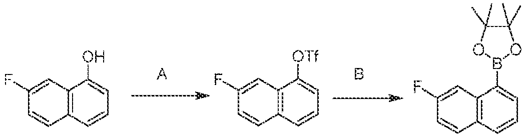

Step A. 7-fluoronaphthalen-1-yl trifluoromethanesulfonate. To a solution of 7-fluoronaphthalen-1-ol (0.50 g, 3.1 mmol) in DMA (15 ml) were added N-ethyl-N-isopropylpropan-2-amine (0.54 ml, 3.1 mmol) and then N-phenyl-bis(trifluoromethanesulfonimide) (1.7 g, 4.6 mmol) at rt. The mixture was stirred at rt for 18 h. The reaction mixture was diluted with aq. Sat. NaHCO₃, and extracted with EtOAc. The organic layer was filtered. The filtrate was evaporated *in vacuo* and purified by chromatography eluting with 0-50% EtOAc/hexane to give 7-fluoronaphthalen-1-yl trifluoromethanesulfonate (0.91 g, 99% yield).

Step B. 2-(7-fluoronaphthalen-1-yl)-4,4,5,5-tetramethyl-1,3,2-dioxaborolane. To a solution of 7-fluoronaphthalen-1-yl trifluoromethanesulfonate (0.50 g, 1.7 mmol) in dioxane (9 ml) were added potassium acetate (0.47 g, 5.1 mmol) and 4,4,4',4',5,5,5',5'-octamethyl-2,2'-bi(1,3,2-dioxaborolane) (1.3 g, 5.1 mmol).The reaction was sparged with N₂ for 15 minutes, followed by addition of PdCl₂(dppf) (0.12 g, 0.17 mmol) and the reaction mixture was heated to 95°C for 18 hrs. The reaction was concentrated *in vacuo* and taken up in DCM. The slurry was filtered through GF/F paper and the filtrate was concentrated *in vacuo.* The residue was purified by flash chromatography using 10→100% DCM/hexane as eluent to give 2-(7-fluoronaphthalen-1-yl)-4,4,5,5-tetramethyl-1,3,2-dioxaborolane (0.18 g, 66%).

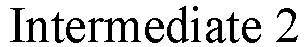

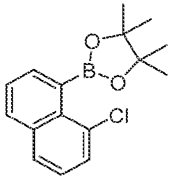

### 2-(8-chloronaphthalen-1-yl)-4,4,5,5-tetramethyl-1,3,2-dioxaborolane

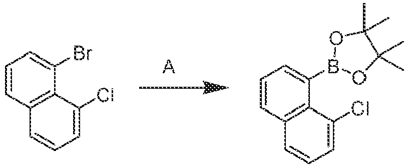

Step A. 2-(8-chloronaphthalen-1-yl)-4,4,5,5-tetramethyl-1,3,2-dioxaborolane. To a solution of 1-bromo-8-chloronaphthalene (20 g, 83 mmol) in dioxane (415 mL) were added KOAc (24.4 g, 248 mmol) and 4,4,4',4',5,5,5',5'-octamethyl-2,2'-bi(1,3,2-dioxaborolane) (63 g, 248 mmol).The reaction was degassed with Argon for 15 minutes followed by addition of PdCl₂(dppf) (6.1 g, 8.3 mmol) and the reaction was heated to 95 °C for 18 hours. The mixture was filtered, and the filtrate was partitioned between water (400 mL) and EtOAc (400 mL). The aqueous layer was extracted with EtOAc (2 x 200 mL) and the combined organic phases were washed with brine (200 mL), dried over Na₂SO₄, filtered and concentrated. The residue was filtered through a silica gel plug eluting with hexanes to 10% EtOAc/hexanes, and further purified by silica gel column eluting with 0-8% EtOAc / hexanes. Clean fractions from both lots were combined and concentrated to afford 2-(8-chloronaphthalen-1-yl)-4,4,5,5-tetramethyl-1,3,2-dioxaborolane (15 g, 62% yield). ¹H NMR (400 MHz, (CDCl₃) δ 7.86 (dd, *J =* 8.0, 1.2 Hz, 1H), 7.75 (dd, *J =* 7.7, 1.2 Hz, 1H), 7.66 (dd, *J* = 7.0, 1.2 Hz, 1H), 7.57 (dd, *J =* 7.5, 1.1 Hz, 1H), 7.50 (dd, *J =* 7.1, 6.9 Hz, 1H), 7.36 (dd, *J* = 8.2, 7.4 Hz, 1H), 1.44 (s, 12H).

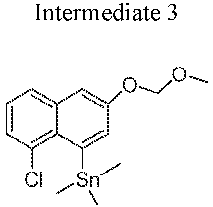

### (8-chloro-3-(methoxymethoxy)naphthalen-1-yl)trimethylstannane

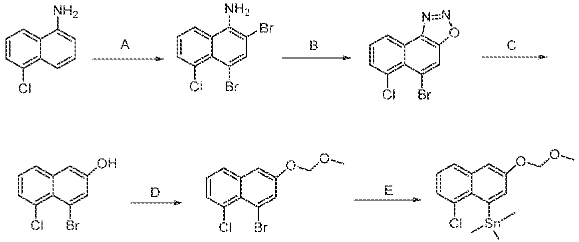

Step A. 2,4-dibromo-5-chloronaphthalen-1-amine: To a solution of 5-Chloronaphthalen-1-amine (1000 mg, 5.63 mmol) in chloroform (30 ml) was added bromine (0.58 ml, 11.3 mmol) in chloroform (30 ml) dropwise. The mixture was heated at 50° C overnight. Additional bromine (0.58 ml, 11.3 mmol) in 30 ml of chloroform was added dropwise at room temperature and the mixture was warmed to 50° C for 4 more hours. The reaction was cooled to rt and concentrated *in vacuo.* Water was added to the residue and the aqueous layer was extracted three times with ethyl acetate. Pooled organic layers were dried over magnesium sulfate, filtered, and concentrated. The residue was purified by silica gel column to give 2,4-dibromo-5-chloronaphthalen-1-amine as a brown solid. ¹H NMR 400 MHz, (CDCl₃) δ 7.94 (s, 1H), 7.76 (d, 1H, J= 8.0 Hz), 7.64 (d, 1H, J= 8.0 Hz), 7.36 (t, 1H, J= 8.0 Hz), 4.46 (bs, 2H).

Step B. 5-bromo-6-chloronaphtho[1,2-d][1,2,3]oxadiazole: 2,4-dibromo-5-chloronaphthalen-1-amine (900 mg, 2.68 mmol) was dissolved in acetic acid (22 ml) and propionic acid (2.2 ml) and cooled in an ice bath followed by addition of sodium nitrite (278 mg, 4.02 mmol) and the reaction was stirred at 0 °C for one hour and rt for one hour. Water was added to the reaction and the aqueous layer was extracted three times with ethyl acetate. The pooled organic layers were dried over magnesium sulfate, filtered, and concentrated. Crude product was purified by silica gel column to give 5-bromo-6-chloronaphtho[1,2-d][1,2,3]oxadiazole as a brown/yellow solid. ¹H NMR 400 MHz, (CDCl₃) δ 7.45-7.38 (m, 2H), 7.31 (s, 1H), 7.22 (dd, 1H, *J =* 8.0, 4.0 Hz).

Step C. 4-bromo-5-chloronaphthalen-2-ol: 5-bromo-6-chloronaphtho[1,2-d][1,2,3]oxadiazole (282 mg, 0.995 mmol) was dissolved in ethanol (15 ml) and THF (15 ml) at 0° C. Sodium borohydride (86.5 mg, 2.29 mmol) was added and warmed up to rt over 2 hours. The solvent was removed, and water was added to the residue. The mixture was acidified with 2 M HCl (aq.) and extracted two times with ethyl acetate. Pooled organic layers were dried over magnesium sulfate, filtered, and concentrated. Crude product was purified by silica gel column to give 4-bromo-5-chloronaphthalen-2-ol as a yellow solid. ¹H NMR 500 MHz, (CDCl₃) δ 7.61-7.58 (m, 2H), 7.48 (d, 1H, J= 10.0 Hz), 7.30 (d, 1H, J= 10.0 Hz), 7.15 (s, 1H), 5.02 (s, 1H).

Step D. 1-bromo-8-chloro-3-(methoxymethoxy)naphthalene: To a solution of 4-bromo-5-chloronaphthalen-2-ol (203 mg, 0.788 mmol) in THF (3900 µL) at 0° C was added sodium hydride (47.3 mg, 1.18 mmol). The mixture was stirred at 0° C for 30 minutes followed by addition of chloromethyl methyl ether (77.8 µL, 1.02 mmol) and the mixture was warmed to room temperature over 2 hours. The reaction was concentrated *in vacuo.* The residue was partitioned between EtOAc and water and the layers were separated. The aqueous layer was extracted with additional ethyl acetate. Pooled organic layers were dried over magnesium sulfate, filtered, and concentrated. The crude material was purified by silica gel column to give 1-bromo-8-chloro-3-(methoxymethoxy)naphthalene as solid. ¹H NMR 500 MHz, (CDCl₃) δ 7.68 (s, 1H), 7.65 (d, 1H, J= 10.0 Hz), 7.49 (d, 1H, J= 10.0 Hz), 7.36 (s, 1H), 7.29 (t, 1H, J= 10.0 Hz), 5.26 (s, 2H), 3.51 (s, 3H).

Step E. (8-chloro-3-(methoxymethoxy)naphthalen-1-yl)trimethylstannane: A mixture of 1-bromo-8-chloro-3-(methoxymethoxy)naphthalene (200 mg, 0.663 mmol), 1,1,1,2,2,2-hexamethyldistannane (0.69 ml, 3.32 mmol) and toluene (4.1 ml) was sparged with argon for 5 minutes. Tetrakis(triphenylphosphine) Pd(0) (76.6 mg, 0.0663 mmol) was added and the reaction was sparged with argon for a few more minutes. The mixture was heated at 110 °C overnight. The reaction was diluted with water and the aqueous layer extracted 2X with hexane. The pooled organic layers were washed with brine, dried over magnesium sulfate, filtered, and concentrated. Crude product was purified by silica gel column to give (8-chloro-3-(methoxymethoxy)naphthalen-1-yl)trimethylstannane as an oil. ¹H NMR 500 MHz, (CDCl₃) δ 7.67 (d, 1H, J= 10.0 Hz), 7.56 (s, 1H), 7.46 (d, 1H, J= 10.0 Hz), 7.37 (s, 1H), 7.31 (t, 1H, J= 10.0 Hz), 5.30 (s, 2H), 3.53 (s, 3H), 0.42 (s, 9H).

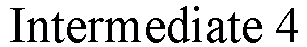

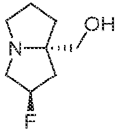

### ((2R,7aS)-2-fluorotetrahydro-1H-pyrrolizin-7a(5H)-yl)methanol (racemic, trans)

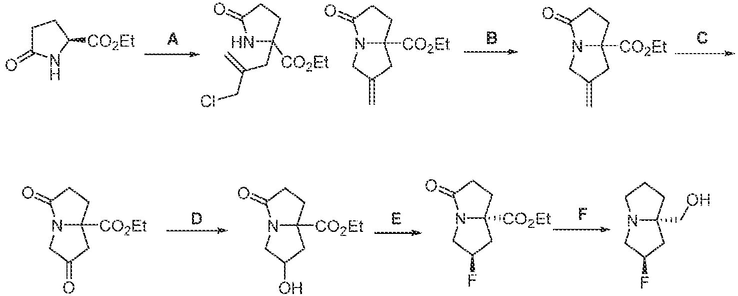

Step A. Ethyl 2-(2-(chloromethyl)allyl)-5-oxopyrrolidine-2-carboxylate. To a stirred solution of ethyl (S)-5-oxopyrrolidine-2-carboxylate (5.7 g, 36.3 mmol) and 3-chloro-2-(chloromethyl)prop-1-ene (16.8 ml, 145 mmol) in 36 mL of THF at -40 °C under nitrogen was added LiHMDS (76.2 ml, 76.2 mmol) (1M in THF) by slow cannulation. After 15 minutes, the cooling bath was removed. The reaction was warmed to room temperature and stirred for 2 hours. The reaction was quenched with saturated ammonium chloride solution (20 mL) and then partially concentrated to about 60 mL. The residual material was partitioned between ethyl acetate (100 mL) and water (100 mL) and the layers were separated. The organics were washed 1 x 100 mL with brine, dried over MgSO₄, filtered and concentrated. The crude product was purified by flash chromatography eluting with an ethyl acetate/hexanes gradient (20% to 80% ethyl acetate). The crude product (5.55 g total) contained a mixture (approximately 2.7:1) of ethyl 2-(2-(chloromethyl)allyl)-5-oxopyrrolidine-2-carboxylate and ethyl 2-methylene-5-oxotetrahydro-1H-pyrrolizine-7a(5H)-carboxylate (product of the step B) and was carried on crude without further purification.

Step B. Ethyl 2-methylene-5-oxotetrahydro-1H-pyrrolizine-7a(5H)-carboxylate. To a stirred suspension of NaH (139 mg, 3.47 mmol) in 40 mL of THF at 0 °C under nitrogen was added a 2.7:1 mixture of ethyl 2-(2-(chloromethyl)allyl)-5-oxopyrrolidine-2-carboxylate and ethyl 2-methylene-5-oxotetrahydro-1H-pyrrolizine-7a(5H)-carboxylate (710 mg, 2.89 mmol) as a solution in 20 mL of THF by syringe. After the addition was complete, the mixture was heated to reflux overnight. The reaction was cooled to room temperature and quenched with water (20 mL). The majority of the THF was removed by rotary evaporation and the residual solution was partitioned between ethyl acetate (50 mL) and water (50 mL). The organics were dried over MgSO₄, filtered and concentrated to yield ethyl 2-methylene-5-oxotetrahydro-1H-pyrrolizine-7a(5H)-carboxylate an orange oil which was carried on crude without further purification.

Step C. Ethyl 2,5-dioxotetrahydro-1H-pyrrolizine-7a(5H)-carboxylate. To a stirred solution of crude ethyl 2-methylene-5-oxotetrahydro-1H-pyrrolizine-7a(5H)-carboxylate (1.1 g, 5.26 mmol) in 14 mL of dichloromethane at -78 °C was added ozone gas via a pipet inserted into the solution. Ozone was continuously passed through the solution until a light blue color appeared (about 15 minutes). The ozone generator was turned off and oxygen was then passed through the reaction for about 5 minutes. The ozone generator was disconnected and nitrogen gas was passed through the solution for another 5 minutes. Polymer-bound triphenylphosphine (3.50 g, 10.5 mmol) was added neat as a solid at - 78 °C. The reaction was warmed to rt and slowly stirred overnight. The reaction mixture was filtered and concentrated to yield 1g of a light yellow oil which was carried on crude without further purification.

Step D. Ethyl 2-hydroxy-5-oxotetrahydro-1H-pyrrolizine-7a(5H)-carboxylate. Ethyl 2,5-dioxotetrahydro-1H-pyrrolizine-7a(5H)-carboxylate (1.08 g, 5.113 mmol) was charged to a 50 mL round bottom flask equipped with a stir bar and nitrogen inlet with methanol (17 ml, 5.1 mmol). To the stirring solution was added sodium borohydride neat (0.14 g, 3.8 mmol). After 5 minutes the mixture was quenched slowly with 10% aqueous K₂CO₃ and the aqueous layer was extracted with 5 portions of 25% IPA/DCM. The combined organics were dried over Na₂SO₄ and concentrated *in vacuo* to yield 969 mg of a white solid which was carried on crude without further purification.

Step E. Ethyl 2-fluoro-5-oxotetrahydro-1H-pyrrolizine-7a(5H)-carboxylate. To a stirred solution of crude ethyl 2-hydroxy-5-oxotetrahydro-1H-pyrrolizine-7a(5H)-carboxylate (4.8:1 cis:trans isomers) (1 g, 4.69 mmol) in dichloromethane (14.2 ml, 4.69 mmol) at -78 °C was added Deoxo-Fluor (0.86 ml, 4.7 mmol) neat by syringe. The reaction was stirred overnight and warmed to rt. The mixture was then partitioned between 25% IPA/DCM and water and the layers were separated. The aqueous layer was washed 3x with 25% IPA/DCM and the organics were combined and dried over Na₂SO₄. The crude product was concentrated purified by flash chromatography eluting with an ethyl acetate/hexanes gradient (0% to 60% ethyl acetate) to yield ethyl 2-fluoro-5-oxotetrahydro-1H-pyrrolizine-7a(5H)-carboxylate as a clear oil containing a single racemic trans diastereomer (210 mg, 0.98 mmol, 21%). ¹H NMR (400 MHz, CDCl₃) δ 5.30, (m, 1H), 4.21 (m, 2H), 3.16 (m, 1H), 2.73 (m, 4H), 2.45 (m, 1H), 2.19 (m, 2H), 1.28 (m, 3H).

Step F. (2-fluorotetrahydro-1H-pyrrolizin-7a(5H)-yl)methanol. Ethyl 2-fluoro-5-oxotetrahydro-1H-pyrrolizine-7a(5H)-carboxylate (.21 g, 0.990 mmol) and dry THF (2 ml) were charged to a 25 mL pear shaped flask equipped with a stir bar. The mixture was cooled to 0 °C and LAH (1M in THF) (2.97 ml, 2.97 mmol) was added dropwise. The vessel was equipped with a cold-water condenser and heated to 70 °C for 4 hours. The mixture was diluted with ethyl ether, cooled to 0 °C and quenched with 110 µL DI water. 110 µL of 15% aqueous NaOH was added to the mixture, followed by 330 µL of DI water. The vessel was warmed to room temperature and stirred for 15 minutes. To the mixture was added anhydrous magnesium sulfate. The mixture was stirred for 15 minutes before being filtered and concentrated *in vacuo.* LCMS (MM-ES+APCI, Pos): m/z 160.2 (M+H).

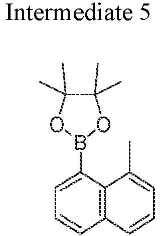

### 4,4,5,5-tetramethyl-2-(8-methylnaphthalen-1-yl)-1,3,2-dioxaborolane

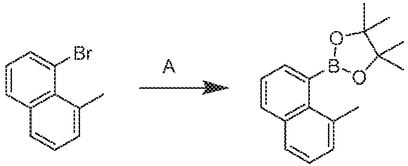

Step A. 4,4,5,5-tetramethyl-2-(8-methylnaphthalen-1-yl)-1,3,2-dioxaborolane. To a solution of 1-bromo-8-methylnaphthalene (0.700 g, 3.17 mmol) in dioxane (15.8 ml) was added potassium acetate (0.932 g, 9.50 mmol) and 4,4,4',4',5,5,5',5'-octamethyl-2,2'-bi(1,3,2-dioxaborolane) (2.41 g, 9.50 mmol) and the reaction sparged with N₂ for 15 minutes, followed by the addition of PdCl₂(dppf) (0.232 g, 0.317 mmol). The reaction was heated to 95°C for 18 hrs. The reaction was concentrated *in vacuo* and taken up in DCM. The slurry was filtered through GF/F filter paper and the organics was concentrated *in vacuo.* The material was chromatographed twice using 102→100% Ethyl acetate/hexane as eluent to give 4,4,5,5-tetramethyl-2-(8-methylnaphthalen-1-yl)-1,3,2-dioxaborolane (576 mg, 2.15 mmol, 68 % yield). HPLC (5-95% ACN/H₂O+0.1%TFA) 3.701 min.

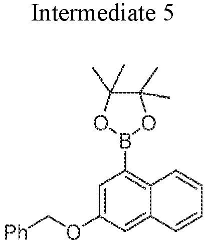

### 2-(3-(benzyloxy) naphthalen-1-yl)-4,4,5,5-tetramethyl-1,3,2-dioxaborolane

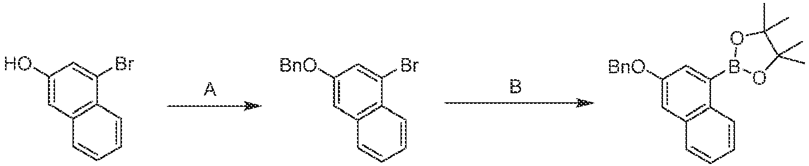

Step A. 3-(benzyloxy)-1-bromonaphthalene. A solution of 4-bromonaphthalen-2-ol (5.0 g, 22 mmol) in DMF (50 mL) was treated with sodium hydride (0.99 g, 60%, 25 mmol) and heated to 50 °C for 1 hour under N₂. After the mixture was cooled to room temperature, benzyl bromide (3.5 mL, 29 mmol) was added, followed by tetrabutylammonium iodide (0.82 g, 2.2 mmol). The mixture was stirred for 16 hours and then partitioned between water (200 mL) and EtOAc (200 mL). The aqueous layer was extracted with EtOAc (2 x 100 mL) and the combined organic phases were washed with water (4 x 100 mL) and brine (50 mL), dried over Na₂SO₄, filtered and concentrated. The residue was purified by silica column chromatography eluting with 0-15% EtOAc/hexanes. The impure product was chromatographed a second time eluting with 0-5% EtOAc/hexanes to afford 3-(benzyloxy)-1-bromonaphthalene (6.2 g, 88% yield).

Step B. 2-(3-(benzyloxy) naphthalen-1-yl)-4,4,5,5-tetramethyl-1,3,2-dioxaborolane. 3-(Benzyloxy)-1-bromonaphthalene (1.2 g, 3.9 mmol), 4,',4',4',5,',5',5'-octamethyl-',2'-bi(1,3,2-dioxaborolane) (3.0 g, 12 mmol) and potassium acetate (1.16 g, 11.8 mmol) were combined in dioxanes (20 mL) and purged with Argon for 5 minutes. PdCl₂(dppf) (0.29 g, 0.39 mmol) was added. The reaction was heated to 95 °C for 6 hours, and then stirred at room temperature for 16 hours. The mixture was partitioned between water (100 mL) and EtOAc (50 mL), and the aqueous layer was extracted with EtOAc (2 x 30 mL). The combined organic phases were washed with brine (30 mL), dried over Na₂SO₄, filtered and concentrated *in vacuo.* The residue was purified by silica column chromatography eluting with 0-15% EtOAc/hexanes to afford 2-(3-(benzyloxy)naphthalen-1-yl)-4,4,5,5-tetramethyl-1,3,2-dioxaborolane (1.3 g, 88% yield) ¹H NMR (400 MHz, (CDCl₃) δ 8.66 (d, *J =* 8.3 Hz, 1H), 7.85 (d, *J =* 2.3 Hz, 1H), 7.49 (d, *J =* 8.2 Hz, 2H), 7.35 (m, 7H), 5.19 (s, 2H), 1.41 (s, 12H).

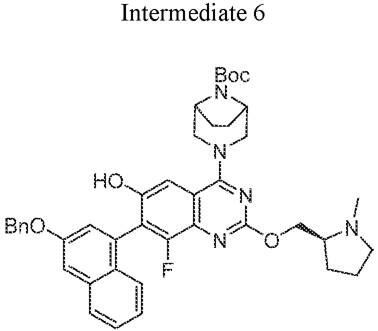

### tert-butyl (1R,5S)-3-(7-(3-(benzyloxy)naphthalen-1-yl)-8-fluoro-6-hydroxy-2-(((S)-1-methylpyrrolidin-2-yl)methoxy)quinazolin-4-yl)-3,8-diazabicyclo[3.2.1]octane-8-carboxylate

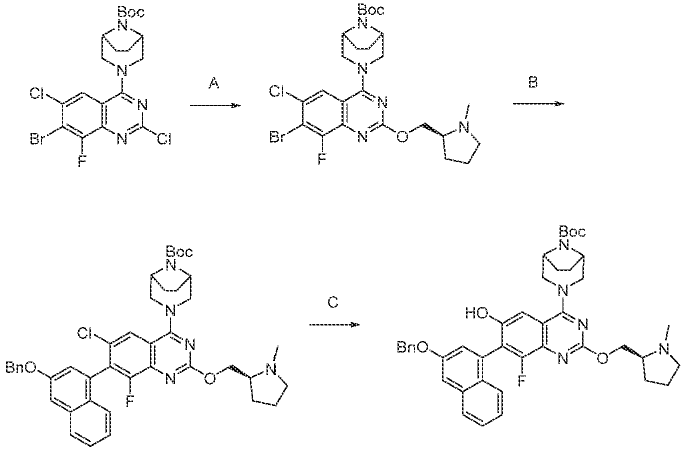

The Step A: tert-butyl (1R,5S)-3-(7-bromo-6-chloro-8-fluoro-2-(((S)-1-methylpyrrolidin-2-yl)methoxy)quinazolin-4-yl)-3,8-diazabicyclo[3.2.1]octane-8-carboxylate. A solution of tert-butyl (1R,5S)-3-(7-bromo-2,6-dichloro-8-fluoroquinazolin-4-yl)-3,8-diazabicyclo[3.2.1]octane-8-carboxylate (1.0 g, 2.0 mmol), DIEA (1.3g, 10 mmol) and (S)-(1-methylpyrrolidin-2-yl)methanol (1.1g, 10 mmol) in 1,4-dioxane (20 mL) was heated at 100°C for 18 hours. The reaction mixture was diluted with EtOAc and washed with 10% aq. K₂CO₃ (3x). The organic phase was dried over Na₂SO₄, filtered, concentrated under reduced pressure, and purified by silica gel chromatography (0-10% DCM/MeOH with 0.1% NH₄OH) to afford tert-butyl (1R,5S)-3-(7-bromo-6-chloro-8-fluoro-2-(((S)-1-methylpyrrolidin-2-yl)methoxy)quinazolin-4-yl)-3,8-diazabicyclo[3.2.1]octane-8-carboxylate (0.35g, 30 %). LCMS (MM-ES+APCI, Pos): m/z 583.4 (M+H).

Step B: tert-butyl (1R,5S)-3-(7-(3-(benzyloxy)naphthalen-1-yl)-6-chloro-8-fluoro-2-(((S)-1-methylpyrrolidin-2-yl)methoxy)quinazolin-4-yl)-3,8-diazabicyclo[3.2.1]octane-8-carboxylate. A solution of tert-butyl (1R,5S)-3-(7-bromo-6-chloro-8-fluoro-2-(((S)-1-methylpyrrolidin-2-yl)methoxy)quinazolin-4-yl)-3,8-diazabicyclo[3.2.1]octane-8-carboxylate (0.30 g, 0.51 mmol), 2-(3-(benzyloxy)naphthalen-1-yl)-4,4,5,5-tetramethyl-1,3,2-dioxaborolane (0.24 g, 0.67 mmol), Pd(Ph₃P)₄ (0.12 g, 0.10 mmol) in 1,4-dioxane (5.0 mL) was added aqueous K₂CO₃ (1.0 mL, 2.0 mmol, 2.0 M). The reaction was sparged with argon for 5 minutes, sealed and heated at 100°C for 18 hours. The reaction was diluted with 1,4 dioxane (25 mL), filtered through celite, concentrated and purified by reverse phase chromatography (5-95% ACN/H₂O with 0.1%TFA). The fractions containing product were diluted with saturated aqueous NaHCO₃ (15 mL) and extracted with 4:1 DCM/IPA (3 x 15 mL). The extracts were combined, dried over Na₂SO₄, filtered and concentrated to afford tert-butyl (1R,5S)-3-(7-(3-(benzyloxy)naphthalen-1-yl)-6-chloro-8-fluoro-2-(((S)-1-methylpyrrolidin-2-yl)methoxy)quinazolin-4-yl)-3,8-diazabicyclo[3.2.1]octane-8-carboxylate (0.33 g, 76 %). LCMS (MM-ES+APCI, Pos): m/z 738.3 (M+H).

Step C: tert-butyl (1R,5S)-3-(7-(3-(benzyloxy)naphthalen-1-yl)-8-fluoro-6-hydroxy-2-(((S)-1-methylpyrrolidin-2-yl)methoxy)quinazolin-4-yl)-3,8-diazabicyclo[3.2.1]octane-8-carboxylate. A solution of tert-butyl (1R,5S)-3-(7-(3-(benzyloxy)naphthalen-1-yl)-6-chloro-8-fluoro-2-(((S)-1-methylpyrrolidin-2-yl)methoxy)quinazolin-4-yl)-3,8-diazabicyclo[3.2.1]octane-8-carboxylate (0.12 g, 0.14 mmol), di-tert-buty'(2',4',6'-triisopropyl-[',1'-biphenyl]-2-yl)phosphane (72 mg, 0.17 mmol), KOH (32 mg, 0.56 mmol) and Pd(dba)₂ (39 mg, 42 µmol) in water (2.5 mL) and 1,4-dioxane (2.5 mL) was sparged with argon and heated at 100°C for 3 hours. The reaction was cooled to ambient temperature, filtered, concentrated and purified by reverse phase chromatography (5-95% ACN/H₂O with 0.1% TFA modifier). Fractions containing product were lyophilized to afford tert-butyl (1R,5S)-3-(7-(3-(benzyloxy)naphthalen-1-yl)-8-fluoro-6-hydroxy-2-(((S)-1-methylpyrrolidin-2-yl)methoxy)quinazolin-4-yl)-3,8-diazabicyclo[3.2.1]octane-8-carboxylate (67 mg, 57 %) as a yellow solid. LCMS (MM-ES+APCI, Pos): m/z 720.4 (M+H).

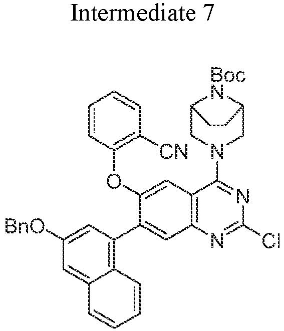

### tert-butyl (1R,5S)-3-(7-(3-(benzyloxy)naphthalen-1-yl)-2-chloro-6-(2-cyanophenoxy)quinazolin-4-yl)-3,8-diazabicyclo[3.2.1]octane-8-carboxylate

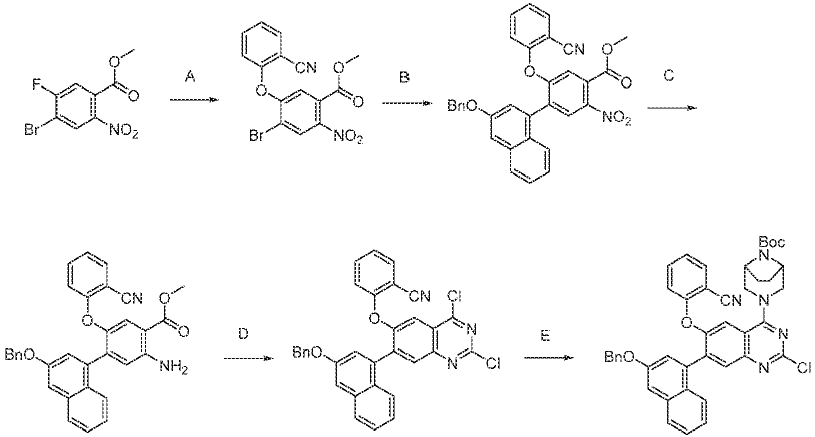

Step A: methyl 4-bromo-5-(2-cyanophenoxy)-2-nitrobenzoate. To a solution of methyl 4-bromo-5-fluoro-2-nitrobenzoate (0.75 g, 2.7 mmol), 2-hydroxybenzonitrile (0.32 g, 2.6 mmol) in DMSO (7 mL) was added potassium carbonate (0.75 mg, 5.4 mmol). The reaction mixture was sparged with nitrogen for 5 minutes and then heated to 100°C for 5 hours. The reaction mixture was cooled to ambient temperature and diluted with water (0.10 mL) and brine (0.10 L). The aqueous phase was extracted with MTBE (3x 50 mL). The organic phases were combined and washed with brine (50 mL), dried over Na₂SO₄, filtered and concentrated. The reaction mixture was purified by flash chromatography (0-50% hexanes/EtOAc) to afford methyl 4-bromo-5-(2-cyanophenoxy)-2-nitrobenzoate (0.57 g, 56 %) as a yellow solid. ¹H NMR (400 MHz, CDCl₃) δ 8.31 (s, 1H), 7.8-7.75 (m, 1H), 7.67-7.61 (m, 1H), 7.39-7.33 (m, 1H), 7.13 (s, 1H), 7.00 (d, 1H), 3.89 (s, 3H).

Step B: methyl 4-(3-(benzyloxy)naphthalen-1-yl)-5-(2-cyanophenoxy)-2-nitrobenzoate. To a solution of methyl 4-bromo-5-(2-cyanophenoxy)-2-nitrobenzoate (0.57 g, 1.5 mmol), 2-(3-(benzyloxy)naphthalen-1-yl)-4,4,5,5-tetramethyl-1,3,2-dioxaborolane (0.64 g, 1.8 mmol) and Pd(PPh₃)₄ (0.26 g, 0.22 mmol) in 1,4-dioxane (15 mL) was added an aqueous solution of potassium carbonate (2.2 mL, 4.5 mmol). The reaction mixture was sparged with argon for 5 minutes, sealed and heated at 100°C for 18 hours. The reaction mixture was cooled to ambient temperature, diluted with EtOAc (50 mL) and filtered through a pad of celite. The organic phase was dried over Na₂SO₄, filtered and concentrated to afford a red solid. The crude reaction mixture was purified by flash chromatography (0-45% hexanes/EtOAc) to afford methyl 4-(3-(benzyloxy)naphthalen-1-yl)-5-(2-cyanophenoxy)-2-nitrobenzoate (0.68 g, 86 %) as a clear yellow oil. LCMS (MM-ES+APCI, Pos): m/z 531.2 (M+H).

Step C: methyl 2-amino-4-(3-(benzyloxy)naphthalen-1-yl)-5-(2-cyanophenoxy)benzoate. A solution of methyl 4-(3-(benzyloxy)naphthalen-1-yl)-5-(2-cyanophenoxy)-2-nitrobenzoate (0.89 g, 1.7 mmol) in THF (12 mL) was stirred while zinc powder (0.90 g, 14 mmol) was added. A solution of saturated aqueous ammonium chloride (2.0 mL) was added to the reaction mixture. The reaction was stirred for 18 hours under nitrogen atmosphere at ambient temperature. The reaction was filtered through GF/F filter paper and the filtrate was concentrated. The residue was purified by flash chromatography eluting with (0-50% EtOAc/hexanes) to afford methyl 2-amino-4-(3-(benzyloxy)naphthalen-1-yl)-5-(2-cyanophenoxy)benzoate (0.56 g, 67 %). LCMS (MM-ES+APCI, Pos): m/z 501.2 (M+H).

Step D: 2-((7-(3-(benzyloxy)naphthalen-1-yl)-2,4-dichloroquinazolin-6-yl)oxy)benzonitrile. To a solution of methyl 2-amino-4-(3-(benzyloxy)naphthalen-1-yl)-5-(2-cyanophenoxy)benzoate (0.50 g, 1.0 mmol) in THF (10 mL) was added 2,2,2-trichloroacetyl isocyanate (0.12 mL, 1.0 mmol) at 0°C. The reaction mixture was stirred for 5 minutes and warmed to ambient temperature over 30 minutes. The reaction mixture was concentrated under reduce pressure. The solids were suspended in methanol (10 mL) and to this suspension was added a solution of NH₃ in methanol (2.9 mL, 20 mmol, 7.0 M). The reaction mixture was stirred at ambient temperature overnight and concentrated under reduced pressure. The white solid was dissolved in POCl₃ (6.0 mL, 0.60 mmol) and DIEA (0.14 g, 0.20 mL, 1.1 mmol) was added. The reaction mixture was heated to 100°C for 18 hours. The reaction mixture was concentrated under reduced pressure. The residue was dissolved in EtOAc (50 mL) and washed with aqueous 1 M Na₂CO₃ (3 x 10 mL) and brine (10 mL). The organic phase was dried over Na₂SO₄, filtered and concentrated under reduced pressure. The residue was purified by flash chromatography (0-55% hexanes/EtOAc) to afford 2-((7-(3-(benzyloxy)naphthalen-1-yl)-2,4-dichloroquinazolin-6-yl)oxy)benzonitrile (0.30 g, 54 %) as a yellow solid. LCMS (MM-ES+APCI, Pos): m/z 548.1 (M+H).

Step E: tert-butyl (1R,5S)-3-(7-(3-(benzyloxy)naphthalen-1-yl)-2-chloro-6-(2-cyanophenoxy)quinazolin-4-yl)-3,8-diazabicyclo[3.2.1]octane-8-carboxylate. To a solution of 2-((7-(3-(benzyloxy)naphthalen-1-yl)-2,4-dichloroquinazolin-6-yl)oxy)benzonitrile (0.29 g, 0.53 mmol) and N-ethyl-N-isopropylpropan-2-amine (0.28 ml, 1.6 mmol) in 1,4-dioxane (6 mL) was added tert-butyl (1R,5S)-3,8-diazabicyclo[3.2.1]octane-8-carboxylate (0.11 mg, 0.53 mmol). The reaction mixture was stirred for 18 hours at ambient temperature. The reaction mixture was concentrated under reduced pressure and purified by flash chromatography (0-55% hexanes/EtOAc) to afford tert-butyl (1R,5S)-3-(7-(3-(benzyloxy)naphthalen-1-yl)-2-chloro-6-(2-cyanophenoxy)quinazolin-4-yl)-3,8-diazabicyclo[3.2.1]octane-8-carboxylate (0.37 g, 96 %) as a white solid. LCMS (MM-ES+APCI, Pos): m/z 724.2 (M+H).

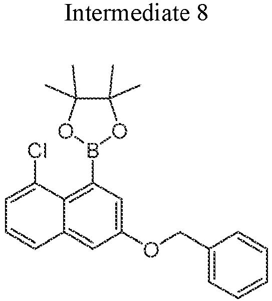

### 2-(3-(benzyloxy)-8-chloronaphthalen-1-yl)-4,4,5,5-tetramethyl-1,3,2-dioxaborolane

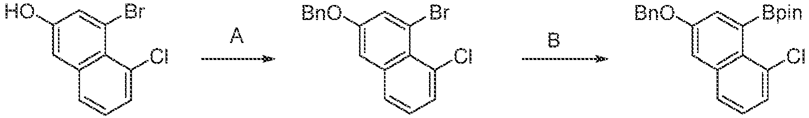

Step A. 3-(benzyloxy)-1-bromo-8-chloronaphthalene. A solution of 4-bromo-5-chloronaphthalen-2-ol (1 g, 3.9 mmol) in DMF (16 mL) was cooled in an ice/water and sodium hydride (0.16 g, 4.0 mmol) was added to the mixture. The reaction was stirred until gas evolution ceased then (bromomethyl)benzene (0.50 ml, 4.3 mmol) and tetrabutylammonium iodide (0.14 g, 0.39 mmol) were added and the reaction stirred at ambient temperature for 16 hours. The reaction was diluted with water (100 mL) and extracted with EtOAc (3 x 50 mL). The organics were combined, dried over sodium sulfate, filtered and concentrated to afford a yellow oil that was purified by silica gel chromatography eluting with 0-20% hexanes/EtOAc to afford 3-(benzyloxy)-1-bromo-8-chloronaphthalene (1.3 g, 99 % yield). 1H NMR (400 MHz, CDCl₃) δ 7.71 (d, 1H), 7.63 (d, 1H), 7.50-7.33(m, 6H), 7.29 (t, 1H), 7.19 (d, 1H), 5.16 (d, 2H).

Step B. 2-(3-(benzyloxy)-8-chloronaphthalen-1-yl)-4,4,5,5-tetramethyl-1,3,2-dioxaborolane. A solution of 3-(benzyloxy)-1-bromo-8-chloronaphthalene (1.0 g, 2.9 mmol), 4,',4',4',5,',5',5'-octamethyl-',2'-bi(1,3,2-dioxaborolane) (2.2 g, 8.6 mmol), potassium acetate (0.85 g, 8.6 mmol), and [1,1'-Bis(diphenylphosphino)ferrocene]dichloropalladium(II) (0.47 g, 0.58 mmol) in 1,4-dioxane (14 mL) was sparged with argon for 5 minutes, then sealed, and heated to 100°C for 16 hours. The reaction mixture was cooled to ambient temperature, poured into water (100 mL), and extracted with DCM (3 x 50 mL). The organics were combined, dried over sodium sulfate, filtered, concentrated and purified by silica gel chromatography eluting with 0-70% hexanes/DCM to afford 2-(3-(benzyloxy)-8-chloronaphthalen-1-yl)-4,4,5,5-tetramethyl-1,3,2-dioxaborolane (0.73 g, 65 % yield). 1H NMR (400 MHz, CDCl₃) δ 7.62 (d, 1H), 7.50-7.46 (m, 2 H), 7.43-7.28 (m, 6H), 7.21 (d, 1H), 5.18 (s, 2H), 1.43 (s, 12H).

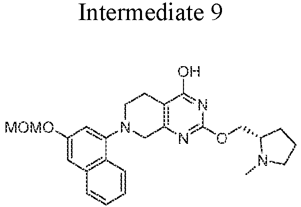

### (S)-7-(3-(methoxymethoxy)naphthalen-1-yl)-2-((1-methylpyrrolidin-2-yl)methoxy)-5,6,7,8-tetrahydropyrido[3,4-d]pyrimidin-4-ol

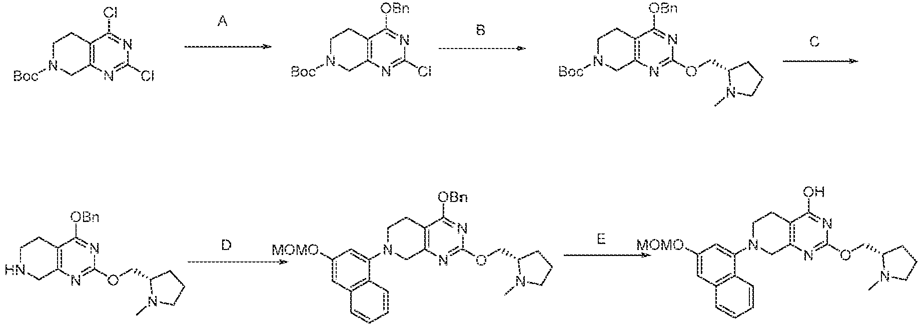

Step A. tert-butyl 4-(benzyloxy)-2-chloro-5,8-dihydropyrido[3,4-d]pyrimidine-7(6H)-carboxylate. To a stirred mixture of tert-butyl 2,4-dichloro-5,8-dihydropyrido[3,4-d]pyrimidine-7(6H)-carboxylate (25 g, 82 mmol) in dioxane (240 mL) were added phenylmethanol (26 mL, 250 mmol) and cesium carbonate (53 g, 160 mmol) and the mixture was heated to 80 °C for 8 hours. The reaction mixture was cooled to r.t., concentrated in vacuo to ~75 mL, and partitioned between ethyl acetate (200 mL) and water (200 mL). The organic layer was washed with brine (200 mL), dried over MgSO₄, filtered, and concentrated in vacuo. A portion of the residue was chromatographed on silica gel in 0-20% of ethyl acetate/hexanes to give pure crystalline material. The remaining crude product was diluted with ~20% ethyl acetate/hexanes, seeded with the crystals, and sonicated. The solid was filtered off, washed with 30% ethyl acetate/hexanes, and dried under a stream of N₂ to give the product. The mother liquor was concentrated, dried under high vacuum, and chromatographed on silica gel eluting with 0→20% EtOAc/hexane to give the product. Combined yield (7.3 g, 56%). LCMS (MM-ES+APCI, Pos): m/z 376.2 (M+H).

Step B. tert-butyl (S)-4-(benzyloxy)-2-((1-methylpyrrolidin-2-yl)methoxy)-5,8-dihydropyrido[3,4-d]pyrimidine-7(6H)-carboxylate. Synthesized according to Example 1, Step B, substituting tert-butyl 4-(benzyloxy)-2-chloro-5,8-dihydropyrido[3,4-d]pyrimidine-7(6H)-carboxylate (1.2 g, 3.3 mmol) in place of tert-butyl (1R,5S)-3-(7-benzyl-2-chloro-5,6,7,8-tetrahydropyrido[3,4-d]pyrimidin-4-yl)-3,8-diazabicyclo[3.2.1]octane-8-carboxylate to yield the title product as a yellow solid (1.0 g, 67%). LCMS (MM-ES+APCI, Pos): m/z 455.3 (M+H).

Step C. (S)-4-(benzyloxy)-2-((1-methylpyrrolidin-2-yl)methoxy)-5,6,7,8-tetrahydropyrido[3,4-d]pyrimidine. To a stirred solution of tert-butyl (S)-4-(benzyloxy)-2-((1-methylpyrrolidin-2-yl)methoxy)-5,8-dihydropyrido[3,4-d]pyrimidine-7(6H)-carboxylate (1.0 g, 2.2 mmol) in dichloromethane (6.6 mL) was added TFA (6.8 mL, 88 mmol). After 1 hour at r.t. the reaction was concentrated under a stream of nitrogen and under high vacuum. The residue was dissolved in dichloromethane (10 mL) and treated with a 20% sodium carbonate solution (10 mL). The layers were separated and the aqueous phase was extracted with 10% methanol in dichloromethane (2 x 10 mL). The combined organic layers were dried over MgSO₄, filtered, and concentrated to a yellow oil that was used as crude in the next reaction. LCMS (MM-ES+APCI, Pos): m/z 355.2 (M+H).

Step D. (S)-4-(benzyloxy)-7-(3-(methoxymethoxy)naphthalen-1-yl)-2-((1-methylpyrrolidin-2-yl)methoxy)-5,6,7,8-tetrahydropyrido[3,4-d]pyrimidine. A mixture of Cs₂CO₃ (5.9 g, 18 mmol), (S)-4-(benzyloxy)-2-((1-methylpyrrolidin-2-yl)methoxy)-5,6,7,8-tetrahydropyrido[3,4-d]pyrimidine (2.1 g, 6.0 mmol), RuPhos (0.28 mg, 0.60 mmol), RuPhos-Pd-G4 precatalyst (0.51 mg, 0.60 mmol), 3-(methoxymethoxy)naphthalen-1-yl trifluoromethanesulfonate (3.0 g, 9.0 mmol) and 1,4-dioxane (20 mL) was degassed and stirred under N₂ at 75 °C for 1.5 hr. The reaction mixture was cooled and partitioned between EtOAc (50 mL) and water (20 mL). The organic layer was washed with 0.5M NaHCO₃ and brine (15 mL each), dried over Na₂SO₄, and evaporated *in vacuo.* The residue was chromatographed on silica gel eluting with 4% MeOH/DCM + 0.4% NH₄OH as a modifier to give the product (2.4 g, 74%). LCMS (MM-ES+APCI, Pos): m/z 541.3 (M+H).

Step E. (S)-7-(3-(methoxymethoxy)naphthalen-1-yl)-2-((1-methylpyrrolidin-2-yl)methoxy)-5,6,7,8-tetrahydropyrido[3,4-d]pyrimidin-4-ol. (S)-4-(benzyloxy)-7-(3-(methoxymethoxy)naphthalen-1-yl)-2-((1-methylpyrrolidin-2-yl)methoxy)-5,6,7,8-tetrahydropyrido[3,4-d]pyrimidine was reduced with hydrogen gas in methanol with 20% palladium (II) hydroxide on carbon to give the title compound. LCMS (MM-ES+APCI, Pos): m/z 451.2 (M+H).

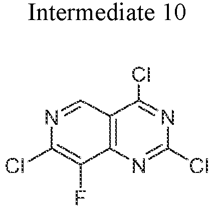

### 2,4,7-trichloro-8-fluoropyrido[4,3-d]pyrimidine

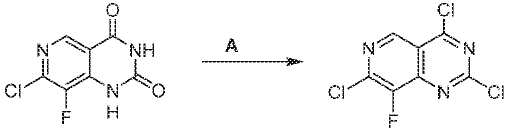

Step A. 2,4,7-trichloro-8-fluoropyrido[4,3-d]pyrimidine. To a solution of 7-chloro-8-fluoropyrido[4,3-d]pyrimidine-2,4(1H,3H)-dione (100 g, 463 mmol, 1.00 *eq*) in toluene (500 mL) were added POCl₃ (213 g, 1.39 mol, 129 mL, 3.00 *eq*) and DIEA (179 g, 1.39 mol, 242 mL, 3.00 *eq*) at 0 °C. The mixture was stirred at 110 °C for 5 h. The reaction was distilled in vacuum (80 °C, water pump) to give 2,4,7-trichloro-8-fluoropyrido[4,3-d]pyrimidine (100 g, 396.10 mmol, 85.39% yield) as brown oil.

### EXAMPLE 1

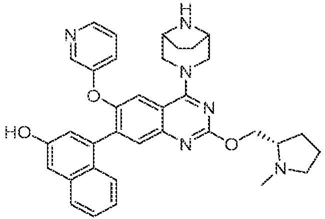

### 4-(4-((1R,5S)-3,8-diazabicyclo[3.2.1]octan-3-yl)-2-(((S)-1-methylpyrrolidin-2-yl)methoxy)-45hosphordin-3-yloxy)quinazolin-7-yl)naphthalen-2-ol

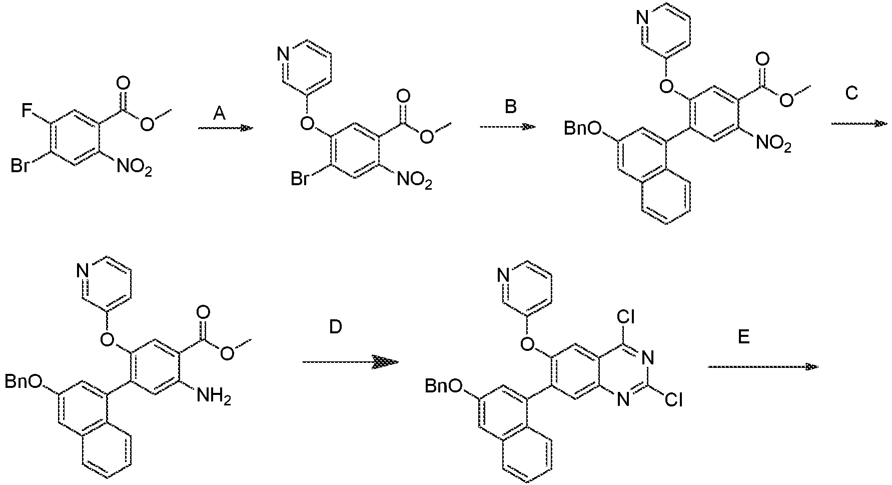

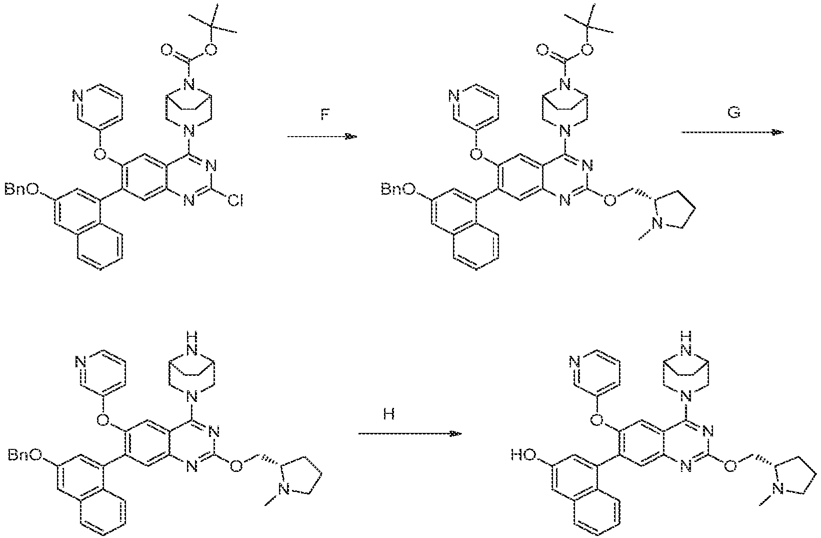

Step A: methyl 4-bromo-2-nitro-46hosphordin-3-yloxy)benzoate. A mixture of methyl 4-bromo-5-fluoro-2-nitrobenzoate (2.5 g, 9.0 mmol 46 hosphordin-3-ol (1.0 g, 11 mmol), and potassium carbonate (2.5 g, 18 mmol) in 90 ml of DMSO was warmed to 80°C for 16 hrs and cooled to room temperature. The mixture was diluted with water/EtOAc and extracted with EtOAc. The organics were washed with brine (2X), dried over sodium sulfate and concentrated under reduced pressure. The crude product was purified by flash chromatography eluting with 5→95% EtOAc/Hex to give methyl 4-bromo-2-nitro-46hosphordin-3-yloxy)benzoate (1.5 g, 49%). LCMS (MM-ES+APCI, Pos): m/z: 353 [M+H].

Step B: methyl 4-(3-(benzyloxy)naphthalen-1-yl)-2-nitro- 46 hosphordin-3-yloxy)benzoate. A mixture of methyl 4-bromo-2-nitro-46hosphordin-3-yloxy)benzoate (1.5 g, 4.4 mmol), 2-(3-(benzyloxy)naphthalen-1-yl)-4,4,5,5-tetramethyl-1,3,2-dioxaborolane (2.0 g, 5.7 mmol), Pd(Ph₃P)₄ (0.50 g, 0.44 mmol), aqueous potassium carbonate (3 eq. of 2M solution) in 44 mL of dioxane was purged with argon for 5 minutes. The mixture was warmed to 100°C for 16 hrs and cooled to room temperature. The mixture was diluted with EtOAc, and washed with 10% aq. potassium carbonate. The organics were dried over sodium sulfate and concentrated under reduced pressure. The residue was purified by chromatography eluting with 5→90% EtOAc/Hex to afford 1.7 g of methyl 4-(3-(benzyloxy)naphthalen-1-yl)-2-nitro-47hosphordin-3-yloxy)benzoate. LCMS (MM-ES+APCI, Pos): m/z: 507.1 [M+H].

Step C: methyl 2-amino-4-(3-(benzyloxy)naphthalen-1-yl)- 47 hosphordin-3-yloxy)benzoate. A mixture of methyl 4-(3-(benzyloxy)naphthalen-1-yl)-2-nitro-47hosphordin-3-yloxy)benzoate (1.7 g, 3.4 mmol), 35 mL of THF, 35 mL of saturated ammonium chloride and Zn dust (2.2 g, 34 mmol) was stirred at room temperature overnight. The mixture was diluted with water/EtOAc and filtered through GF/F filter paper. The filtrate was extracted with EtOAc. The organics were dried over sodium sulfate and concentrated under reduced pressure to give methyl 2-amino-4-(3-(benzyloxy)naphthalen-1-yl)-47hosphordin-3-yloxy)benzoate (1.5 g, 93%). LCMS (MM-ES+APCI, Pos): m/z: 477.1 [M+H].

Step D: 7-(3-(benzyloxy)naphthalen-1-yl)-2,4-dichloro- 47 hosphordin-3-yloxy)quinazoline. A round bottom flask equipped with a stir bar and rubber septum was charged with methyl 2-amino-4-(3-(benzyloxy)naphthalen-1-yl)-47hosphordin-3-yloxy)benzoate (1.6 g, 3.4 mmol), dry THF (35 mL) and this mixture was chilled to 0°C. To this was added 2,2,2-trichloroacetyl isocyanate (0.64 g, 3.4 mmol). The mixture was stirred at 0°C for 5 minutes and then warmed to rt. After 30 minutes, the mixture was concentrated under reduced pressure and the crude material taken up in methanol (35 mL) and 30 eq. of 7M NH₃ in methanol added. The mixture was stirred at room temperature overnight and concentrated under reduced pressure. The crude material was taken up in POCl₃ (20 mL), 1 eq. of DIEA was added and the mixture warmed to reflux overnight. The POCl₃ was removed by distillation. The crude material was taken up in EtOAc. The mixture was washed with 10% aq. K₂CO₃, filtered, dried over sodium sulfate and concentrated under reduced pressure to give 7-(3-(benzyloxy)naphthalen-1-yl)-2,4-dichloro-47hosphordin-3-yloxy)quinazoline (0.78 g, 44%) LCMS (MM-ES+APCI, Pos): m/z: 524.1 [M+H].

Step E: tert-butyl (1R,5S)-3-(7-(3-(benzyloxy)naphthalen-1-yl)-2-chloro-47hosphordin-3-yloxy)quinazolin-4-yl)-3,8-diazabicyclo[3.2.1]octane-8-carboxylate. A mixture of 7-(3-(benzyloxy)naphthalen-1-yl)-2,4-dichloro-47hosphordin-3-yloxy)quinazoline (0.78 g, 1.5 mmol), tert-butyl 3,8-diazabicyclo[3.2.1]octane-8-carboxylate (0.41 g, 1.9 mmol), DIEA (0.38 g, 3.0 mmol) in 15 mL of dioxane was stirred at room temperature for 45 minutes. The mixture was diluted with EtOAc and washed with 10% aq. potassium carbonate. The organics were dried over sodium sulfate and concentrated under reduced pressure. The residue was purified by chromatography eluting with 0→60% EtOAc/DCM afforded tert-butyl (1R,5S)-3-(7-(3-(benzyloxy)naphthalen-1-yl)-2-chloro- 48 hosphordin-3-yloxy)quinazolin-4-yl)-3,8-diazabicyclo[3.2.1]octane-8-carboxylate (0.36g, 35%). LCMS (MM-ES+APCI, Pos): m/z: 700.2 [M+H].

Step F: tert-butyl (1R,5S)-3-(7-(3-(benzyloxy)naphthalen-1-yl)-2-(((S)-1-methylpyrrolidin-2-yl)methoxy)- 48 hosphordin-3-yloxy)quinazolin-4-yl)-3,8-diazabicyclo[3.2.1]octane-8-carboxylate. A mixture of tert-butyl (1R,5S)-3-(7-(3-(benzyloxy)naphthalen-1-yl)-2-chloro- 48 hosphordin-3-yloxy)quinazolin-4-yl)-3,8-diazabicyclo[3.2.1]octane-8-carboxylate (0.36 g, 0.51 mmol), (S)-(1-methylpyrrolidin-2-yl)methanol (0.18 g, 1.5 mmol), cesium carbonate (0.50 g, 1.5 mmol), and RuPhos Pd G3 (0.043 g, 0.051 mmol) in 5 mL of dioxane was warmed to 100°C for 16 hrs and cooled to room temperature. The mixture was diluted with EtOAc, and washed with 10% aq. potassium carbonate. The organics were dried over sodium sulfate and concentrated under reduced pressure. The residue was purified by chromatography eluting with 0→20% MeOH/DC to afforded tert-butyl (1R,5S)-3-(7-(3-(benzyloxy)naphthalen-1-yl)-2-(((S)-1-methylpyrrolidin-2-yl)methoxy)- 48 hosphordin-3-yloxy)quinazolin-4-yl)-3,8-diazabicyclo[3.2.1]octane-8-carboxylate (0.26 g, 65%). %). LCMS (MM-ES+APCI, Pos): m/z: 779.2 [M+H].

Step G: 7-(3-(benzyloxy)naphthalen-1-yl)-4-((1R,5S)-3,8-diazabicyclo[3.2.1]octan-3-yl)-2-(((S)-1-methylpyrrolidin-2-yl)methoxy)-48 hosphordin-3-yloxy)quinazoline. To tert-butyl (1R,5S)-3-(7-(3-(benzyloxy)naphthalen-1-yl)-2-(((S)-1-methylpyrrolidin-2-yl)methoxy)-48hosphordin-3-yloxy)quinazolin-4-yl)-3,8-diazabicyclo[3.2.1]octane-8-carboxylate (0.26 g, 0.33 mmol) in 3 mL of DCM was added 3 mL of TFA and the mixture was stirred at room temperature for one hour. The mixture was diluted with EtOAc, and washed with 10% aqueous potassium carbonate. The organics were dried over sodium sulfate and concentrated under reduced pressure. Reverse phase purification and neutralization of the product fractions afforded 7-(3-(benzyloxy)naphthalen-1-yl)-4-((1R,5S)-3,8-diazabicyclo[3.2.1]octan-3-yl)-2-(((S)-1-methylpyrrolidin-2-yl)methoxy)-48hosphordin-3-yloxy)quinazoline (0.17 g, 74%) of. LCMS (MM-ES+APCI, Pos): m/z: 679.3 [M+H].

Step H: 4-(4-((1R,5S)-3,8-diazabicyclo[3.2.1]octan-3-yl)-2-(((S)-1-methylpyrrolidin-2-yl)methoxy)- 49 hosphordin-3-yloxy)quinazolin-7-yl)naphthalen-2-ol. A mixture of 7-(3-(benzyloxy)naphthalen-1-yl)-4-((1R,5S)-3,8-diazabicyclo[3.2.1]octan-3-yl)-2-(((S)-1-methylpyrrolidin-2-yl)methoxy)-49hosphordin-3-yloxy)quinazoline (0.066 g, 0.097 mmol), 1 ml of methanol and 66 mgs of Pearlman's catalyst was subjected to a balloon of hydrogen for 2 hours. The mixture was filtered through a syringe fitted with an Acro filter disc, and the filtrate was concentrated under reduced pressure to give 4-(4-((1R,5S)-3,8-diazabicyclo[3.2.1]octan-3-yl)-2-(((S)-1-methylpyrrolidin-2-yl)methoxy)- 49 hosphordin-3-yloxy)quinazolin-7-yl)naphthalen-2-ol (38 mgs, 67%). LCMS (MM-ES+APCI, Pos): m/z: 589.3 [M+H].

### EXAMPLE 2

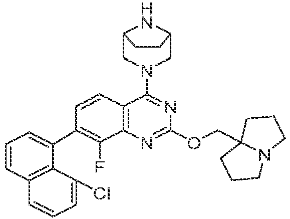

### 4-((1R,5 S)-3,8-diazabicyclo[3.2.1]octan-3-yl)-7-(8-chloronaphthalen-1-yl)-8-fluoro-2-((tetrahydro-1H-pyrrolizin-7a(5H)-yl)methoxy)quinazoline

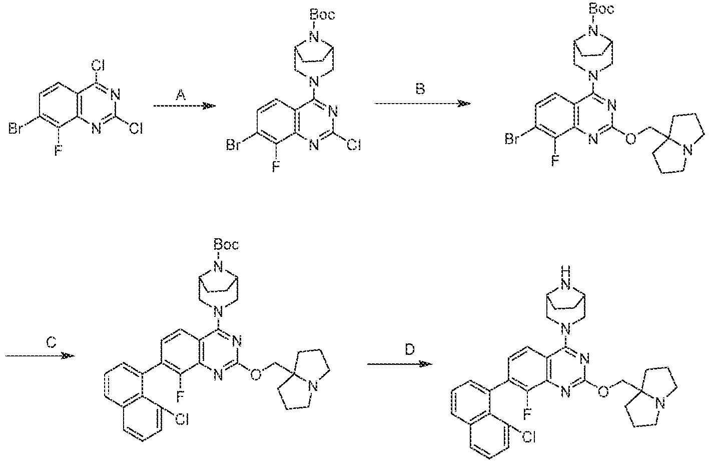

Step A. Tert-butyl (1R,5S)-3-(7-bromo-2-chloro-8-fluoroquinazolin-4-yl)-3,8-diazabicyclo[3.2.1]octane-8-carboxylate. A mixture of 7-bromo-2,4-dichloro-8-fluoroquinazoline (0.20 g, 0.68 mmol) and 8-Boc-3,8-diazabicyclo[3.2.1]octane (0.14 g, 0.68 mmol) in DMA (3 ml) was treated with DIEA (0.24 ml, 1.4 mmol) at rt. The mixture was stirred at rt for 18 hours. Water was added and the slurry filtered. The solid was purified by silica gel column eluting with 20% EtOAc/Hex to afford desired product as a white solid (0.23 g, 71%). LCMS (MM-ES+APCI, Pos): m/z 471.1 (M+H).

Step B. Tert-butyl (1R,5S)-3-(7-bromo-8-fluoro-2-(((S)-1-methylpyrrolidin-2-yl)methoxy)quinazolin-4-yl)-3.8-diazabicyclo[3.2.1]octane-8-carboxylate. A mixture of tert-butyl (1R,5S)-3-(7-bromo-2-chloro-8-fluoroquinazolin-4-yl)-3,8-diazabicyclo[3.2.1]octane-8-carboxylate (0.11 g, 0.24 mmol), tetrahydro-1H-pyrrolizine-7a(5H)-methanol (0.10 g, 0.72 mmol), cesium carbonate (0.23 g, 0.72 mmol) in 1,4-dioxane (2 ml) was degassed with argon and heated at 80°C overnight. The reaction was cooled to room temperature, diluted with water and extracted two times with ethyl acetate. The pooled organics were dried over magnesium sulfate, filtered, and concentrated. Crude material was purified by silica gel column eluting with 0→25% MeOH/DCM to afford desired product as a white solid (20 mg, 15%). LCMS (MM-ES+APCI, Pos): m/z 576.2 (M+H).

Step C. Tert-butyl (1R,5S)-3-(7-(8-chloronaphthalen-1-yl)-8-fluoro-2-((tetrahydro-1H-pyrrolizin-7a(5H)-yl)methoxy)quinazolin-4-yl)-3,8-diazabicyclo[3.2.1]octane-8-carboxylate. A mixture of 2-(8-chloronaphthalen-1-yl)-4,4,5,5-tetramethyl-1,3,2-dioxaborolane (30 mg, 0.10 mmol), tert-butyl (1R,5S)-3-(7-bromo-8-fluoro-2-((tetrahydro-1H-pyrrolizin-7a(5H)-yl)methoxy)quinazolin-4-yl)-3,8-diazabicyclo[3.2.1]octane-8-carboxylate (20 mg, 0.035 mmol), potassium carbonate (24 mg, 0.17 mmol), and Pd(Ph₃P)₄ (4.0 mg, 0.004 mmol) in toluene (0.5 mL), ethanol (0.1 mL), and water (50 □L) was sparged with argon for 5 minutes and heated at 85°C for 4 hours. Water was added and the aqueous layer was extracted two times with ethyl acetate. Pooled organics were dried over magnesium sulfate, filtered, and concentrated. Crude material was purified by silica gel column eluting with 0→25% MeOH/DCM to give desired product (2 mg, 9%). LCMS (MM-ES+APCI, Pos): m/z 658.2 (M+H).

Step D. 4-((1R,5S)-3,8-diazabicyclo[3.2.1]octan-3-yl)-7-(8-chloronaphthalen-1-yl)-8-fluoro-2-((tetrahydro-1H-pyrrolizin-7a(5H)-yl)methoxy)quinazoline. Tert-butyl (1R,5S)-3-(7-(8-chloronaphthalen-1-yl)-8-fluoro-2-((tetrahydro-1H-pyrrolizin-7a(5H)-yl)methoxy)quinazolin-4-yl)-3,8-diazabicyclo[3.2.1]octane-8-carboxylate (2 mg, 0.003 mmol) was treated with dichloromethane/ trifluoroacetic acid (3:1, 1mL) and the mixture was stirred at room temperature for one hour. The solvent was removed in vacuo and the residue was purified by prep-HPLC (5→95% ACN/ H₂O with 0.1% TFA) to afford desired product (1.3 mg, 57%) as the TFA salt. LCMS (MM-ES+APCI, Pos): m/z 558.3 (M+H).

### EXAMPLE 3

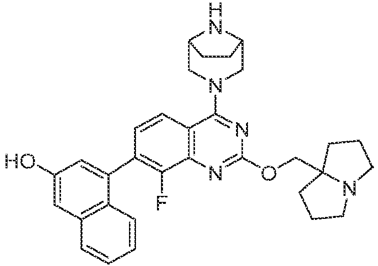

### 4-(4-((1R,5S)-3,8-diazabicyclo[3.2.1]octan-3-yl)-8-fluoro-2-((tetrahydro-1H-pyrrolizin-7a(5H)-yl)methoxy)quinazolin-7-yl)naphthalen-2-ol

Synthesized according to Example 2, Steps A-D substituting 4-(4,4,5,5-tetramethyl-1,3,2-dioxaborolan-2-yl)naphthalen-2-ol in place of 2-(8-chloronaphthalen-1-yl)-4,4,5,5-tetramethyl-1,3,2-dioxaborolane in Step C to afford 4-(4-((1R,5S)-3,8-diazabicyclo[3.2.1]octan-3-yl)-8-fluoro-2-((tetrahydro-1H-pyrrolizin-7a(5H)-yl)methoxy)quinazolin-7-yl)naphthalen-2-ol (27 mg, 95%). LCMS (MM-ES+APCI, Pos): m/z 540.3 (M+H).

### EXAMPLE 4

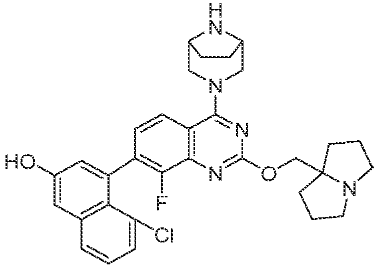

### 4-(4-((1R,5S)-3,8-diazabicyclo[3.2.1]octan-3-yl)-8-fluoro-2-((tetrahydro-1H-pyrrolizin-7a(5H)-yl)methoxy)quinazolin-7-yl)-5-chloronaphthalen-2-ol

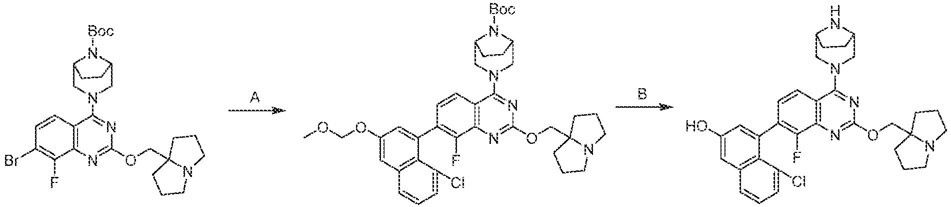

Step A. Tert-butyl (1R,5S)-3-(7-(8-chloro-3-(methoxymethoxy)naphthalen-1-yl)-8-fluoro-2-((tetrahydro-1H-pyrrolizin-7a(5H)-yl)methoxy)quinazolin-4-yl)-3,8-diazabicyclo[3.2.1]octane-8-carboxylate. A mixture of rac-2,2-bis(triphenylphosphine)-',1'-binaphthyl (42 mg, 0.067 mmol), copper(I) iodide (19 mg, 0.10 mmol), tert-butyl (1R,5S)-3-(7-bromo-8-fluoro-2-((tetrahydro-1H-pyrrolizin-7a(5H)-yl)methoxy)quinazolin-4-yl)-3,8-diazabicyclo[3.2.1]octane-8-carboxylate (0.19 g, 0.33 mmol), and (8-chloro-3-(methoxymethoxy)naphthalen-1-yl)trimethylstannane (0.26 g, 0.67 mmol) in toluene (4 ml) was sparged with argon for five minutes. Tetrakis(triphenylphosphine)palladium (0) (77 mg, 0.070 mmol) was added, and the mixture was sparged with argon for a few more minutes. The mixture was heated at 100°C overnight. The reaction was cooled to room temperature, water was added and the aqueous layer was extracted two times with ethyl acetate. Pooled organics were dried over magnesium sulfate, filtered, and concentrated. Crude material was purified by silica gel column eluting with 0→ 20% methanol/dichloromethane with 2% ammonium hydroxide to afford a crude product (0.12 g, 51%). LCMS (MM-ES+APCI, Pos): m/z 718.3 (M+H).

Step B. 4-(4-((1R,5S)-3,8-diazabicyclo[3.2.1]octan-3-yl)-8-fluoro-2-((tetrahydro-1H-pyrrolizin-7a(5H)-yl)methoxy)quinazolin-7-yl)-5-chloronaphthalen-2-ol Synthesized according to Example 2, Step D to afford 4-(4-((1R,5S)-3,8-diazabicyclo[3.2.1]octan-3-yl)-8-fluoro-2-((tetrahydro-1H-pyrrolizin-7a(5H)-yl)methoxy)quinazolin-7-yl)-5-chloronaphthalen-2-ol (16 mg, 12%). LCMS (MM-ES+APCI, Pos): m/z 574.2 (M+H).

### EXAMPLE 5

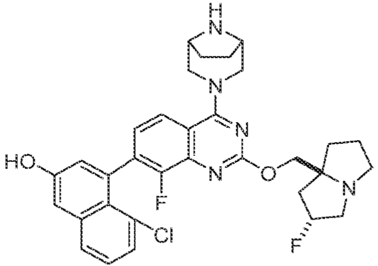

### 4-(4-((1R,5S)-3,8-diazabicyclo[3.2.1]octan-3-yl)-8-fluoro-2-(((2R,7aS)-2-fluorotetrahydro-1H-pyrrolizin-7a(5H)-yl)methoxy)quinazolin-7-yl)-5-chloronaphthalen-2-ol (racemic, trans)

Synthesized according to Example 2, Steps A-B substituting ((2S,7aR)-2-fluorotetrahydro-1H-pyrrolizin-7a(5H)-yl)methanol in place of tetrahydro-1H-pyrrolizine-7a(5H)-methanol in Step B, Example 4, Step A substituting tert-butyl (1R,5S)-3-(7-bromo-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate in place of tert-butyl (1R,5S)-3-(7-bromo-8-fluoro-2-((tetrahydro-1H-pyrrolizin-7a(5H)-yl)methoxy)quinazolin-4-yl)-3,8-diazabicyclo[3.2.1]octane-8-carboxylate and deprotection using Example 2, Step D to afford desired product (25 mg, 11%). LCMS (MM-ES+APCI, Pos): m/z 592.3 (M+H).

### EXAMPLE 6

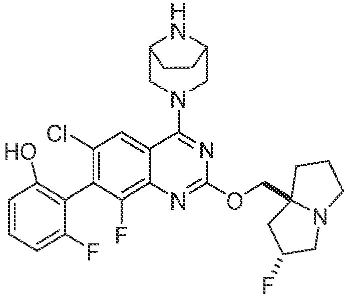

### 2-(4-((1R,5S)-3,8-diazabicyclo[3.2.1]octan-3-yl)-6-chloro-8-fluoro-2-(((2R,7aS)-2-fluorotetrahydro-1H-pyrrolizin-7a(5H)-yl)methoxy)quinazolin-7-yl)-3-fluorophenol (racemic, trans)

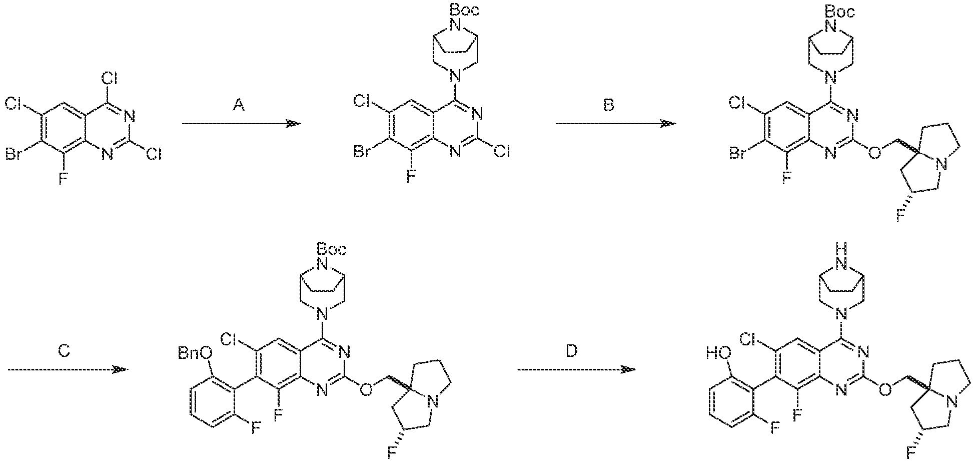

Step A. Tert-butyl (1R,5S)-3-(7-bromo-2,6-dichloro-8-fluoroquinazolin-4-yl)-3,8-diazabicyclo[3.2.1]octane-8-carboxylate. 7-bromo-2,4,6-trichloro-8-fluoroquinazoline (0.70 g, 2.1 mmol), tert-butyl (1R,5S)-3,8-diazabicyclo[3.2.1]octane-8-carboxylate (0.54 g, 2.5 mmol), and N,N-diisopropylethylamine (1.4g, 11 mmol) were combined in 1,4-dioxane (20 mL). After stirred at room temperature for 2 hours, the reaction was concentrated *in vacuo.* The residue was purified by flash chromatography (0-30% EtOAc/hexanes) to afford tert-butyl (1R,5S)-3-(7-bromo-2,6-dichloro-8-fluoroquinazolin-4-yl)-3,8-diazabicyclo[3.2.1]octane-8-carboxylate (0.92 g, 86%). LCMS (MM-ES+APCI, Pos): m/z 507.0 (M+H).

Step B. Tert-butyl (1R,5S)-3-(7-bromo-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate. Tert-butyl (1R,5S)-3-(7-bromo-2,6-dichloro-8-fluoroquinazolin-4-yl)-3,8-diazabicyclo[3.2.1]octane-8-carboxylate (0.25 g, 0.49 mmol), ((2R,7aS)-2-fluorotetrahydro-1H-pyrrolizin-7a(5H)-yl)methanol (0.16 g, 0.99 mmol), and *N,N-*diisopropylethylamine (0.31 g, 2.5 mmol) were dissolved in 1,4-dioxane (5 mL). The solution was sealed in a tube and heated to 90°C for 5 days. The mixture was filtered through Celite and condensed *in vacuo.* Purification by flash chromatography (0-5% MeOH/DCM) afforded tert-butyl (1R,5S)-3-(7-bromo-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (0.11 g, 35%). LCMS (MM-ES+APCI, Pos): m/z 630.2 (M+H).

Step C. Tert-butyl (1R,5S)-3-(7-(2-(benzyloxy)-6-fluorophenyl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate. Tert-butyl (1R,5S)-3-(7-bromo-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (55 mg, 0.087 mmol), tetrakis(triphenylphosphine)palladium(0) (10 mg, 0.0087 mmol), (2-(benzyloxy)-6-fluorophenyl)boronic acid (27 mg, 0.11 mmol), and potassium carbonated (48 mg, 0.35 mmol) were combined in a mixture of 1,4-dioxane (1 mL) and water (0.2 mL). The mixture was degassed with argon before heated to 100°C for 36 hours. The reaction mixture was cooled and condensed *in vacuo.* Purification by prep HPLC (5-95% MeCN/H₂O with 0.1% TFA) followed by partitioning between DCM and saturated aqueous NaHCO₃ afforded tert-butyl (1R,5S)-3-(7-(2-(benzyloxy)-6-fluorophenyl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (20 mg, 31%). LCMS (MM-ES+APCI, Pos): m/z 751.4 (M+H).

Step D. 2-(4-((1R,5S)-3,8-diazabicyclo[3.2.1]octan-3-yl)-6-chloro-8-fluoro-2-(((2R,7aS)-2-fluorotetrahydro-1H-pyrrolizin-7a(5H)-yl)methoxy)quinazolin-7-yl)-3-fluorophenol bis(2,2,2-trifluoroacetate). Tert-butyl (1R,5S)-3-(7-(2-(benzyloxy)-6-fluorophenyl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (20 mg, 0.027 mmol) and pentamethylbenzene (12 mg, 0.080 mmol) were dissolved in DCM (0.5 mL). The solution was cooled to -78°C, and boron trichloride was added as a 1 N solution in DCM (0.053 mL, 0.053 mmol). The mixture was warmed to 0°C over 30 minutes, and then quenched by addition of 1 mL of 1:1 MeCN/MeOH. The solution was condensed *in vacuo.* The residue was purified by prep HPLC (5-95% MeCN/H₂O with 0.1% TFA) followed by lyophilization to afford 2-(4-((1R,5S)-3,8-diazabicyclo[3.2.1]octan-3-yl)-6-chloro-8-fluoro-2-(((2R,7aS)-2-fluorotetrahydro-1H-pyrrolizin-7a(5H)-yl)methoxy)quinazolin-7-yl)-3-fluorophenol bis(2,2,2-trifluoroacetate) (6.0 mg, 33%). LCMS (MM-ES+APCI, Pos): m/z 560.2 (M+H).

### EXAMPLE 7

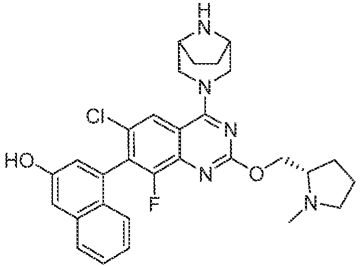

### 4-(4-((1R,5S)-3,8-diazabicyclo[3.2.1]octan-3-yl)-6-chloro-8-fluoro-2-(((S)-1-methylpyrrolidin-2-yl)methoxy)quinazolin-7-yl)naphthalen-2-ol

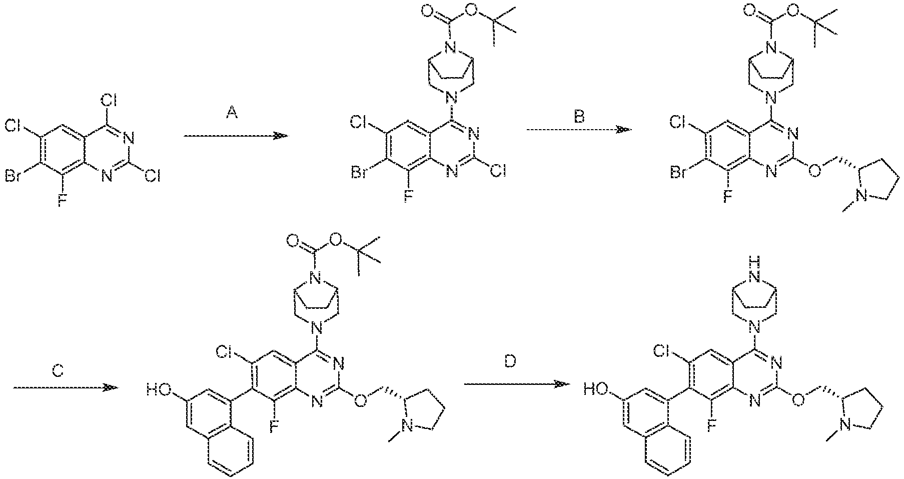

Step A. tert-butyl (1R,5S)-3-(7-bromo-2,6-dichloro-8-fluoroquinazolin-4-yl)-3,8-diazabicyclo[3.2.1]octane-8-carboxylate. A mixture of 7-bromo-2,4,6-trichloro-8-fluoroquinazoline (0.20 g, 0.61 mmol) and 8-Boc-3,8-diazabicyclo[3.2.1]octane (0.13 g, 0.61 mmol) in DMA (3 ml) was treated with DIEA (0.21 ml, 1.2 mmol) at room temperature. The mixture was stirred at rt for 18 h. The mixture was diluted with water and extracted with EtOAc. The organics were separated, filtered through 1PS paper and evaporated in vac to give tert-butyl (1R,5S)-3-(7-bromo-2,6-dichloro-8-fluoroquinazolin-4-yl)-3,8-diazabicyclo[3.2.1]octane-8-carboxylate (0.26 g, 85 % yield). LCMS (MM-ES+APCI, Pos): m/z 635.4 (M+H)⁺.

Step B. tert-butyl (1R,5S)-3-(7-bromo-6-chloro-8-fluoro-2-(((S)-1-methylpyrrolidin-2-yl)methoxy)quinazolin-4-yl)-3,8-diazabicyclo[3.2.1]octane-8-carboxylate. A mixture of tert-butyl (1R,5S)-3-(7-bromo-2,6-dichloro-8-fluoroquinazolin-4-yl)-3,8-diazabicyclo[3.2.1]octane-8-carboxylate (0.26 g, 0.52 mmol) and (S)-(1-methylpyrrolidin-2-yl)methanol (0.12 g, 1.0 mmol) in DMA (5 ml) was treated with DIEA (0.45 ml, 2.6 mmol) at rt. The mixture was stirred at 95°C for 18 h. The mixture was diluted with water and extracted with EtOAc. The organics were filtered through 1PS paper, evaporated in vacuo and purified by chromatography (0-20% MeOH/DCM with 0.5% NH₄OH) to give tert-butyl (1R,5S)-3-(7-bromo-6-chloro-8-fluoro-2-(((S)-1-methylpyrrolidin-2-yl)methoxy)quinazolin-4-yl)-3,8-diazabicyclo[3.2.1]octane-8-carboxylate (43 mg, 14 % yield). LCMS (MM-ES+APCI, Pos): m/z 586.1 (M+H)⁺.

Step C. tert-butyl (1R,5S)-3-(6-chloro-8-fluoro-7-(3-hydroxynaphthalen-1-yl)-2-(((S)-1-methylpyrrolidin-2-yl)methoxy)quinazolin-4-yl)-3,8-diazabicyclo[3.2.1]octane-8-carboxylate. A solution of 4-(4,4,5,5-tetramethyl-1,3,2-dioxaborolan-2-yl)naphthalen-2-ol (30 mg, 0.11 mmol), tert-butyl (1R,5S)-3-(7-bromo-6-chloro-8-fluoro-2-(((S)-1-methylpyrrolidin-2-yl)methoxy)quinazolin-4-yl)-3,8-diazabicyclo[3.2.1]octane-8-carboxylate (43 mg, 0.07 mmol), K₂CO₃ (0.11 ml, 0.22 mmol), Pd(PPh₃)₄ (9 mg, 0.01 mmol) in dioxane (1 ml) was sparged with argon and heated at 95°C for 1 h. The mixture was diluted with aq. saturated NaHCO₃ and extracted with EtOAc. The organics were filtered through 1PS paper, evaporated in vacuo and chromatographed using 0-->20% MeOH/DCM with 0.25% NH₄OH as eluent to give tert-butyl (1R,5S)-3-(6-chloro-8-fluoro-7-(3-hydroxynaphthalen-1-yl)-2-(((S)-1-methylpyrrolidin-2-yl)methoxy)quinazolin-4-yl)-3,8-diazabicyclo[3.2.1]octane-8-carboxylate (25 mg, 52 % yield). LCMS (MM-ES+APCI, Pos): m/z 648.2 (M+H) ⁺.

Step D. 4-(4-((1R,5S)-3,8-diazabicyclo[3.2.1]octan-3-yl)-6-chloro-8-fluoro-2-(((S)-1-methylpyrrolidin-2-yl)methoxy)quinazolin-7-yl)naphthalen-2-ol. To a solution of tert-butyl (1R,5S)-3-(6-chloro-8-fluoro-7-(3-hydroxynaphthalen-1-yl)-2-(((S)-1-methylpyrrolidin-2-yl)methoxy)quinazolin-4-yl)-3,8-diazabicyclo[3.2.1]octane-8-carboxylate (25 mg, 0.04 mmol) in MeOH (0.4 ml) was added 4 M HCl solution in 1,4-dioxane (0.05 ml, 0.2 mmol). The mixture was stirred at rt for 6 hours. The mixture was evaporated in vacuo and purified by reverse prep chromatography (Gilson, 5-95% ACN/H₂O with 0.1% TFA) to afford 4-(4-((1R,5S)-3,8-diazabicyclo[3.2.1]octan-3-yl)-6-chloro-8-fluoro-2-(((S)-1-methylpyrrolidin-2-yl)methoxy)quinazolin-7-yl)naphthalen-2-ol (23 mg, 77 % yield). LCMS (MM-ES+APCI, Pos): m/z 548.2 (M+H)⁺.

### EXAMPLE 8

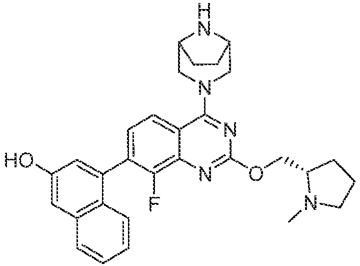

### 4-(4-((1R,5S)-3,8-diazabicyclo[3.2.1]octan-3-yl)-8-fluoro-2-(((S)-1-methylpyrrolidin-2-yl)methoxy)quinazolin-7-yl)naphthalen-2-ol

Synthesized according to Example 7, substituting 7-bromo-2,4-dichloro-8-fluoroquinazoline in place of 7-bromo-2,4,6-trichloro-8-fluoroquinazoline in Step A to give the title compound (7 mg, 12%). LCMS (MM-ES+APCI, Pos): m/z 514.3 (M+H).

### EXAMPLE 9

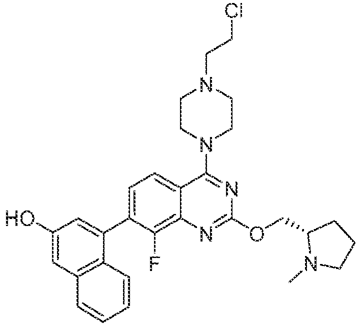

### (S)-4-(4-(4-(2-chloroethyl)piperazin-1-yl)-8-fluoro-2-((1-methylpyrrolidin-2-yl)methoxy)quinazolin-7-yl)naphthalen-2-ol

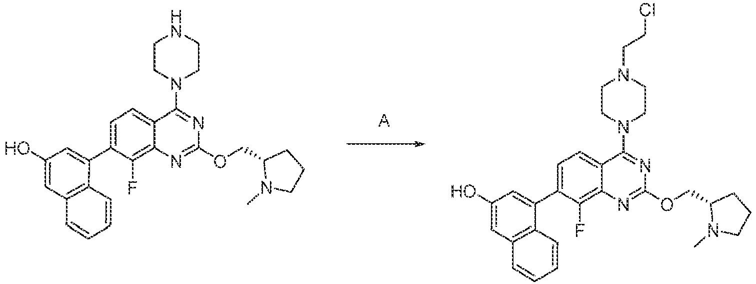

Step A. (S)-4-(4-(4-(2-chloroethyl)piperazin-1-yl)-8-fluoro-2-((1-methylpyrrolidin-2-yl)methoxy)quinazolin-7-yl)naphthalen-2-ol. A solution of (S)-4-(8-fluoro-2-((1-methylpyrrolidin-2-yl)methoxy)-4-(piperazin-1-yl)quinazolin-7-yl)naphthalen-2-ol (15 mg, 0.031 mmol, synthesized according to Example 7, substituting 7-bromo-2,4-dichloro-8-fluoroquinazoline in place of 7-bromo-2,4,6-trichloro-8-fluoroquinazoline and boc piperazine for 8-Boc-3,8-diazabicyclo[3.2.1]octane), and chloroacetaldehyde (8 mg, 0.05 mmol) in THF (0.2 ml) was treated with NaBh(OAc)₃ (13 mg, 0.060 mmol) at rt. The mixture was stirred at rt for 30 min. The mixture was filtered through Acrodisk 0.45 um filter and purified by C18 chromatography (5-95% ACN/H₂O with 0.1% TFA) to give (S)-4-(4-(4-(2-chloroethyl)piperazin-1-yl)-8-fluoro-2-((1-methylpyrrolidin-2-yl)methoxy)quinazolin-7-yl)naphthalen-2-ol (9.0 mg, 38 % yield). LCMS (MM-ES+APCI, Pos): m/z 550.2 (M+H).

### EXAMPLE 10

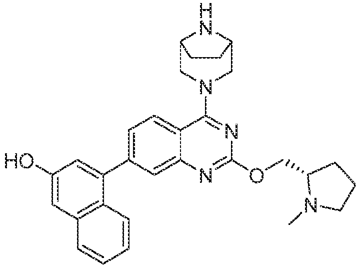

### 4-(4-((1R,5S)-3,8-diazabicyclo[3.2.1]octan-3-yl)-2-(((S)-1-methylpyrrolidin-2-yl)methoxy)quinazolin-7-yl)naphthalen-2-ol

Synthesized according to Example 7, substituting 7-bromo-2,4-dichloroquinazoline kin place of 7-bromo-2,4,6-trichloro-8-fluoroquinazoline to give the tile compound (48 mg, 90%). LCMS (MM-ES+APCI, Pos): m/z 496.3 (M+H).

### EXAMPLE 11

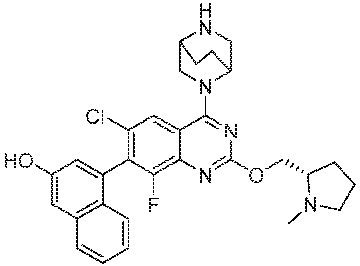

### 4-((7R)-4-(2,5-diazabicyclo[2.2.2]octan-2-yl)-6-chloro-8-fluoro-2-(((S)-1-methylpyrrolidin-2-yl)methoxy)quinazolin-7-yl)naphthalen-2-ol

Synthesized according to Example 7, substituting tert-butyl 2,5-diazabicyclo[2.2.2]octane-2-carboxylate for 8-Boc-3,8-diazabicyclo[3.2.1]octane in step A to give the title compound (8 mg, 28%). LCMS (MM-ES+APCI, Pos): m/z 548.2 (M+H).

### EXAMPLE 12

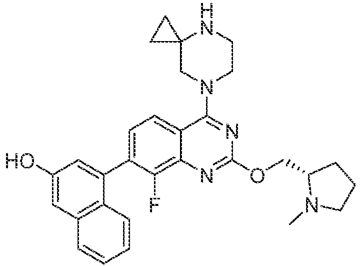

### (S)-4-(8-fluoro-2-((1-methylpyrrolidin-2-yl)methoxy)-4-(4,7-diazaspiro[2.5]octan-7-yl)quinazolin-7-yl)naphthalen-2-ol

Synthesized according to Example 7, substituting 4-Boc-4,7-diazaspiro[2.5]octane in place of 8-Boc-3,8-diazabicyclo[3.2.1]octane and 7-bromo-2,4-dichloro-8-fluoroquinazoline for 7-bromo-2,4,6-trichloro-8-fluoroquinazoline in step A to give the title compound (10 mg, 83%). LCMS (MM-ES+APCI, Pos): m/z 514.3 (M+H).

### EXAMPLE 13

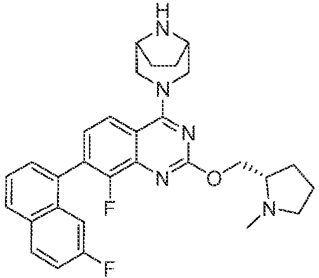

### 4-((1R,5S)-3,8-diazabicyclo[3.2.1]octan-3-yl)-8-fluoro-7-(7-fluoronaphthalen-1-yl)-2-(((S)-1-methylpyrrolidin-2-yl)methoxy)quinazoline

Synthesized according to Example 7, Step A-D substituting 4-Boc-4,7-diazaspiro[2.5]octane in place of 8-Boc-3,8-diazabicyclo[3.2.1]octane and 7-bromo-2,4-dichloro-8-fluoroquinazoline for 7-bromo-2,4,6-trichloro-8-fluoroquinazoline in Step A, in Step C substituting 2-(7-fluoronaphthalen-1-yl)-4,4,5,5-tetramethyl-1,3,2-dioxaborolane in place of 4-(4,4,5,5-tetramethyl-1,3,2-dioxaborolan-2-yl)naphthalen-2-ol and tert-butyl (1R,5S)-3-(7-bromo-8-fluoro-2-(((S)-1-methylpyrrolidin-2-yl)methoxy)quinazolin-4-yl)-3,8-diazabicyclo[3.2.1]octane-8-carboxylate in place of tert butyl (1R,5S)-3-(7-bromo-6-chloro-8-fluoro-2-(((S)-1-methylpyrrolidin-2-yl)methoxy)quinazolin-4-yl)-3,8-diazabicyclo[3.2.1]octane-8-carboxylate (10 mg, 64%). LCMS (MM-ES+APCI, Pos): m/z 516.2 (M+H).

### EXAMPLE 14

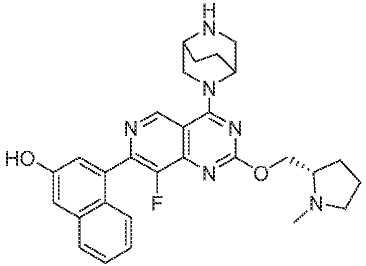

### 4-(4-(2,5-diazabicyclo[2.2.2]octan-2-yl)-8-fluoro-2-(((S)-1-methylpyrrolidin-2-yl)methoxy)pyrido[4,3-d]pyrimidin-7-yl)naphthalen-2-ol

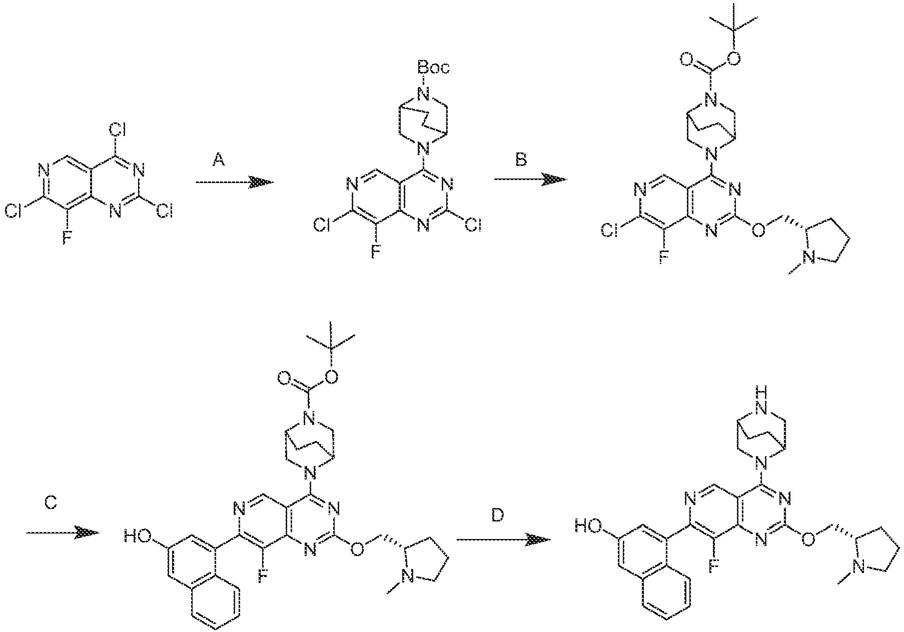

Step A. Tert-butyl 5-(2,7-dichloro-8-fluoropyrido[4,3-d]pyrimidin-4-yl)-2,5diazabicyclo[2.2.2]octane-2-carboxylate. A solution of 2,4,7-trichloro-8-fluoropyrido[4,3-d]pyrimidine (200 mg, 0.63 mmol) in dioxane (4.2 ml) at 0°C was treated with tert-butyl 2,5-diazabicyclo[2.2.2]octane-2-carboxylate (135 mg, 0.63 mmol) and DIEA (0.5 ml, 3.17 mmol). The mixture was stirred at room temperature for 2 h. The reaction mixture was partitioned between EtOAc and water, and the layers were separated. The aqueous layer was extracted with EtOAc twice. The combined organics were filtered through 1PS paper, evaporated in vacuo, and purified by silica gel chromatography eluting with 0-50% EtOAc/hex to give tert-butyl 5-(2,7-dichloro-8-fluoropyrido[4,3-d]pyrimidin-4-yl)-2,5-diazabicyclo[2.2.2]octane-2-carboxylate (58 mg, 21 % yield). LCMS (MM-ES+APCI, Pos): m/z 428.1 (M+H).

Step B. Tert-butyl 5-(7-chloro-8-fluoro-2-(((S)-1-methylpyrrolidin-2-yl)methoxy)pyrido[4,3-d]pyrimidin-4-yl)-2,5-diazabicyclo[2.2.2]octane-2-carboxylate. A mixture of tert-butyl 5-(2,7-dichloro-8-fluoropyrido[4,3-d]pyrimidin-4-yl)-2,5-diazabicyclo[2.2.2]octane-2-carboxylate (57 mg, 0.13 mmol) in dioxane (1.3 ml) was treated with (S)-(1-methylpyrrolidin-2-yl)methanol (31 mg, 0.27 mmol) and cesium carbonate (130 mg, 0.40 mmol) at room temperature. The mixture was stirred at 80°C for 18 h. The mixture was diluted with brine, extracted with EtOAc and the layers were separated. The combined organics were filtered through 1PS paper, evaporated in vacuo, and purified by silica gel chromatography eluting with 0-20% MeOH/DCM with 1% NH₄OH as modifier to give tert-butyl 5-(7-chloro-8-fluoro-2-(((S)-1-methylpyrrolidin-2-yl)methoxy)pyrido[4,3-d]pyrimidin-4-yl)-2,5-diazabicyclo[2.2.2]octane-2-carboxylate (15 mg, 22 % yield). LCMS (MM-ES+APCI, Pos): m/z 507.2 (M+H).

Step C. Tert-butyl 5-(8-fluoro-7-(3-hydroxynaphthalen-1-yl)-2-(((S)-1-methylpyrrolidin-2-yl)methoxy)pyrido[4,3-d]pyrimidin-4-yl)-2,5-diazabicyclo[2.2.2]octane-2-carboxylate. A solution of 4-(4,4,5,5-tetramethyl-1,3,2-dioxaborolan-2-yl)naphthalen-2-ol (21 mg, 0.08 mmol), tert-butyl 5-(7-chloro-8-fluoro-2-(((S)-1-methylpyrrolidin-2-yl)methoxy)pyrido[4,3-d]pyrimidin-4-yl)-2,5-diazabicyclo[2.2.2]octane-2-carboxylate (27 mg, 0.053 mmol), potassium carbonate (0.8 ml, 0.16 mmol), Pd(PPh₃)₄ (6.2 mg, 0.005 mmol) in dioxane (0.5 ml) was sparged with argon and heated at 85°C for 2 h. The mixture was diluted with aq. saturated NaHCO₃, extracted with EtOAc and the layers were separated. The combined organics were filtered through 1PS paper, evaporated and chromatographed eluting with 0-->20% MeOH/DCM with 1% NH₄OH as modifier to give tert-butyl 5-(8-fluoro-7-(3-hydroxynaphthalen-1-yl)-2-(((S)-1-methylpyrrolidin-2-yl)methoxy)pyrido[4,3-d]pyrimidin-4-yl)-2,5-diazabicyclo[2.2.2]octane-2-carboxylate (20 mg, 61 % yield). LCMS (MM-ES+APCI, Pos): m/z 615.3 (M+H).

Step D. 4-(4-(2,5-diazabicyclo[2.2.2]octan-2-yl)-8-fluoro-2-(((S)-1-methylpyrrolidin-2-yl)methoxy)pyrido[4,3-d]pyrimidin-7-yl)naphthalen-2-ol. To a cold 0°C solution of tert-butyl 5-(8-fluoro-7-(3-hydroxynaphthalen-1-yl)-2-(((S)-1-methylpyrrolidin-2-yl)methoxy)pyrido[4,3-d]pyrimidin-4-yl)-2,5-diazabicyclo[2.2.2]octane-2-carboxylate (8 mg, 0.013 mmol) in dichloromethane (0.3 ml) was added trifluoroacetic acid (0.02 ml, 0.26 mmol). The mixture was stirred at room temperature for 1.5 hours. The mixture was evaporated and purified by reverse phase chromatography eluting with 5-95% ACN/H₂O with 0.1% TFA as modifier to afford 4-(4-(2,5-diazabicyclo[2.2.2]octan-2-yl)-8-fluoro-2-(((S)-1-methylpyrrolidin-2-yl)methoxy)pyrido[4,3-d]pyrimidin-7-yl)naphthalen-2-ol as the TFA salt (8 mg, 83 % yield). LCMS (MM-ES+APCI, Pos): m/z 515.2 (M+H).

### EXAMPLE 15

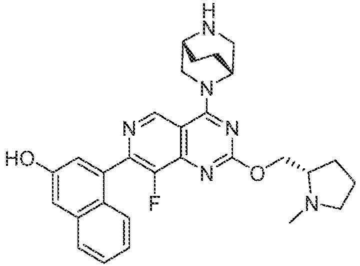

### 4-(4-((1R,4R)-2,5-diazabicyclo[2.2.2]octan-2-yl)-8-fluoro-2-(((S)-1-methylpyrrolidin-2-yl)methoxy)pyrido[4,3-d]pyrimidin-7-yl)naphthalen-2-ol

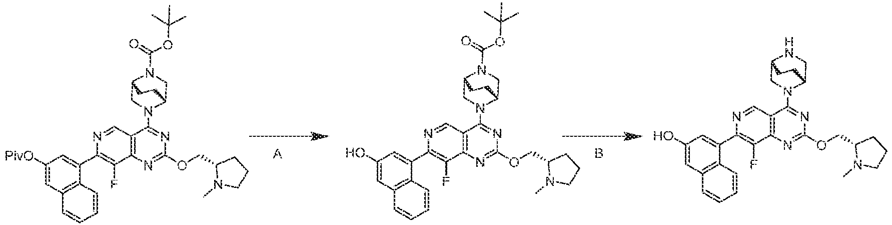

tert-butyl (1R,4R)-5-(8-fluoro-2-(((S)-1-methylpyrrolidin-2-yl)methoxy)-7-(3-(pivaloyloxy)naphthalen-1-yl)pyrido[4,3-d]pyrimidin-4-yl)-2,5-diazabicyclo[2.2.2]octane-2-carboxylate synthesized according to Example 14, Step A-C substituting tert-butyl (1R,4R)-2,5-diazabicyclo[2.2.2]octane-2-carboxylate in place of 8-boc-3,8-diazabicyclo[3.2.1]octane.

Step A. tert-butyl (1R,4R)-5-(8-fluoro-7-(3-hydroxynaphthalen-1-yl)-2-(((S)-1-methylpyrrolidin-2-yl)methoxy)pyrido[4,3-d]pyrimidin-4-yl)-2,5-diazabicyclo[2.2.2]octane-2-carboxylate. To a solution of tert-butyl (1R,4R)-5-(8-fluoro-2-(((S)-1-methylpyrrolidin-2-yl)methoxy)-7-(3-(pivaloyloxy)naphthalen-1-yl)pyrido[4,3-d]pyrimidin-4-yl)-2,5-diazabicyclo[2.2.2]octane-2-carboxylate (7 mg, 0.01 mmol) in MeOH (0.3 ml) and water (0.1 ml) was added LiOH-H2O (1 mg, 0.03 mmol). The mixture was stirred at rt for 1 h. The mixture was concentrated in vacuo, partitioned between aq. saturated NH₄Cl and EtOAc and the layers separated. The combined organics were filtered through 1PS paper and evaporated in vacuo to give tert-butyl (1R,4R)-5-(8-fluoro-7-(3-hydroxynaphthalen-1-yl)-2-(((S)-1-methylpyrrolidin-2-yl)methoxy)pyrido[4,3-d]pyrimidin-4-yl)-2,5-diazabicyclo[2.2.2]octane-2-carboxylate (6 mg, 97 % yield). LCMS (MM-ES+APCI, Pos): m/z 615.3 (M+H).

Step B. 4-(4-((1R,4R)-2,5-diazabicyclo[2.2.2]octan-2-yl)-8-fluoro-2-(((S)-1-methylpyrrolidin-2-yl)methoxy)pyrido[4,3-d]pyrimidin-7-yl)naphthalen-2-ol. Synthesized according to Example 14, Step D substituting tert-butyl (1R,4R)-5-(8-fluoro-7-(3-hydroxynaphthalen-1-yl)-2-(((S)-1-methylpyrrolidin-2-yl)methoxy)pyrido[4,3-d]pyrimidin-4-yl)-2,5-diazabicyclo[2.2.2]octane-2-carboxylate in place of tert-butyl 5-(8-fluoro-7-(3-hydroxynaphthalen-1-yl)-2-(((S)-1-methylpyrrolidin-2-yl)methoxy)pyrido[4,3-d]pyrimidin-4-yl)-2,5-diazabicyclo[2.2.2]octane-2-carboxylate to give the title compound (3 mg, 35%). LCMS (MM-ES+APCI, Pos): m/z 515.2 (M+H).

### EXAMPLE 16

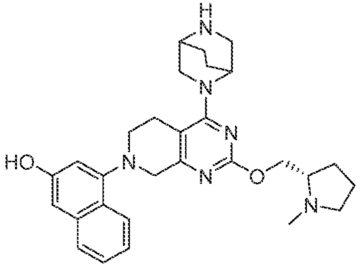

### 4-(4-(2,5-diazabicyclo[2.2.2]octan-2-yl)-2-(((S)-1-methylpyrrolidin-2-yl)methoxy)-5,8-dihydropyrido[3,4-d]pyrimidin-7(6H)-yl)naphthalen-2-ol

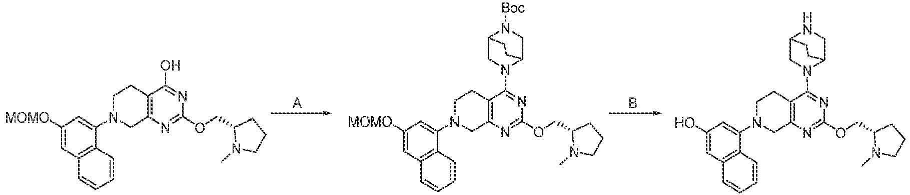

Step A. Tert-butyl 5-(7-(3-(methoxymethoxy)naphthalen-1-yl)-2-(((S)-1-methylpyrrolidin-2-yl)methoxy)-5,6,7,8-tetrahydropyrido[3,4-d]pyrimidin-4-yl)-2,5-diazabicyclo[2.2.2]octane-2-carboxylate. To a solution of (S)-7-(3-(methoxymethoxy)naphthalen-1-yl)-2-((1-methylpyrrolidin-2-yl)methoxy)-5,6,7,8-tetrahydropyrido[3,4-d]pyrimidin-4-ol (40 mg, 0.09 mmol) in dichloromethane (0.9 ml) at -40°C was added triethylamine (0.03 ml, 0.18 mmol) then triflic anhydride (0.02 ml, 0.098 mmol). The mixture was stirred at -40°C for 45 min. To this mixture was added tert-butyl 2,5-diazabicyclo[2.2.2]octane-2-carboxylate (56 mg, 0.27 mmol) and the mixture was stirred at 60°C for 2 h. The mixture was evaporated and purified by silica gel chromatography eluting with 0-20% MeOH/DCM with 0.5% NH₄OH to give tert-butyl 5-(7-(3-(methoxymethoxy)naphthalen-1-yl)-2-(((S)-1-methylpyrrolidin-2-yl)methoxy)-5,6,7,8-tetrahydropyrido[3,4-d]pyrimidin-4-yl)-2,5-diazabicyclo[2.2.2]octane-2-carboxylate (72 mg, 101 % yield). LCMS (MM-ES+APCI, Pos): m/z 645.4 (M+H).

Step B. 4-(4-(2,5-diazabicyclo[2.2.2]octan-2-yl)-2-(((S)-1-methylpyrrolidin-2-yl)methoxy)-5,8-dihydropyrido[3,4-d]pyrimidin-7(6H)-yl)naphthalen-2-ol. To a solution of tert-butyl 5-(7-(3-(methoxymethoxy)naphthalen-1-yl)-2-(((S)-1-methylpyrrolidin-2-yl)methoxy)-5,6,7,8-tetrahydropyrido[3,4-d]pyrimidin-4-yl)-2,5-diazabicyclo[2.2.2]octane-2-carboxylate (24 mg, 0.04 mmol) in MeOH (0.4 ml) was added 4.0 M hydrochloric acid solution in 1,4-dioxane (0.05 ml, 0.19 mmol). The mixture was stirred at room temperature for 18 hours. The mixture was evaporated and purified by C18 chromatography eluting with 5-95% ACN/H₂O with 0.1% TFA as modifier to afford 4-(4-(2,5-diazabicyclo[2.2.2]octan-2-yl)-2-(((S)-1-methylpyrrolidin-2-yl)methoxy)-5,8-dihydropyrido[3,4-d]pyrimidin-7(6H)-yl)naphthalen-2-ol as the TFA salt (14 mg, 75 % yield). LCMS (MM-ES+APCI, Pos): m/z 501.3 (M+H).

### EXAMPLE 17

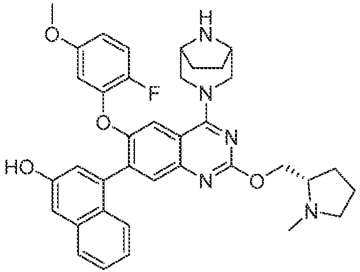

### 4-(4-((1R,5S)-3,8-diazabicyclo[3.2.1]octan-3-yl)-6-(2-fluoro-5-methoxyphenoxy)-2-(((S)-1-methylpyrrolidin-2-yl)methoxy)quinazolin-7-yl)naphthalen-2-ol bis(2,2,2-trifluoroacetate)

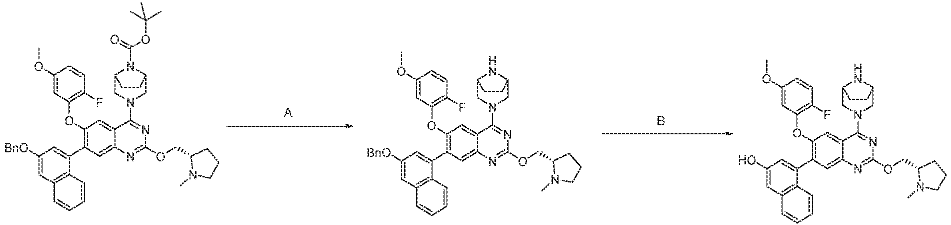

Tert-butyl (1R,5S)-3-(7-(3-(benzyloxy)naphthalen-1-yl)-6-(2-fluoro-5-methoxyphenoxy)-2-(((S)-1-methylpyrrolidin-2-yl)methoxy)quinazolin-4-yl)-3.8-diazabicyclo[3.2.1]octane-8-carboxylate. Synthesized according to Example 1, steps A-F substituting 2-fluoro-5-methoxyphenol in place of pyridine-3-ol in step A to afford tert-butyl (1R,5S)-3-(7-(3-(benzyloxy)naphthalen-1-yl)-6-(2-fluoro-5-methoxyphenoxy)-2-(((S)-1-methylpyrrolidin-2-yl)methoxy)quinazolin-4-yl)-3,8-diazabicyclo[3.2.1]octane-8-carboxylate (13 mg, 19%). LCMS (MM-ES+APCI, Pos): m/z 826.3 (M+H).

Step A: 7-(3-(benzyloxy)naphthalen-1-y1)-4-((1R,5S)-3,8-diazabicyclo[3.2.1]octan-3-yl)-6-(2-fluoro-5-methoxyphenoxy)-2-(((S)-1-methylpyrrolidin-2-yl)methoxy)quinazoline. Tert-butyl (1R,5S)-3-(7-(3-(benzyloxy)naphthalen-1-yl)-6-(2-fluoro-5-methoxyphenoxy)-2-(((S)-1-methylpyrrolidin-2-yl)methoxy)quinazolin-4-yl)-3,8-diazabicyclo[3.2.1]octane-8-carboxylate (.013 g, 0.016 mmol), dichloromethane (1 ml), and 4M HCl in Dioxane (1 ml, 4.0 mmol) were charged to a 25 mL pear shaped flask equipped with a stir bar. The mixture was stirred at room temperature for 90 minutes and the volatiles were removed in vacuo. The crude residue was purified by reverse-phase HPLC (Gilson, 5-95 % CH₃CN/H₂O with 0.1% TFA). Product containing fractions were pooled, frozen, and lyophilized overnight to furnish 7-(3-(benzyloxy)naphthalen-1-yl)-4-((1R,5S)-3,8-diazabicyclo[3.2.1]octan-3-yl)-6-(2-fluoro-5-methoxyphenoxy)-2-(((S)-1-methylpyrrolidin-2-yl)methoxy)quinazoline (5.0 mg, 43%). LCMS (MM-ES+APCI, Pos): m/z 726.3 (M+H).

Step B: 4-(4-((1R,5S)-3,8-diazabicyclo[3.2.1]octan-3-yl)-6-(2-fluoro-5-methoxyphenoxy)-2-(((S)-1-methylpyrrolidin-2-yl)methoxy)quinazolin-7-yl)naphthalen-2-ol bis(2,2,2-trifluoroacetate). 7-(3-(benzyloxy)naphthalen-1-yl)-4-((1R,5S)-3,8-diazabicyclo[3.2.1]octan-3-yl)-6-(2-fluoro-5-methoxyphenoxy)-2-(((S)-1-methylpyrrolidin-2-yl)methoxy)quinazoline (5.0 mg, 0.0080 mmol), methanol (0.1 ml), and dihydroxypalladium (0.0011 g, 0.0016 mmol) were charged to a 10 mL round bottom flask equipped with a stir bar. The mixture was placed under an N₂ atmosphere and stirred at room temperature. The mixture was purged with H₂ via balloon for 2 minutes. The mixture was stirred at room temperature for 1 hour. The mixture was sparged with nitrogen, diluted with MeOH, and filtered. The material was purified with a Gilson reverse-phase preparatory HPLC (5 to 95% H_{I}ACN with 0.1% TFA) to furnish 4-(4-((1R,5S)-3,8-diazabicyclo[3.2.1]octan-3-yl)-6-(2-fluoro-5-methoxyphenoxy)-2-(((S)-1-methylpyrrolidin-2-yl)methoxy)quinazolin-7-yl)naphthalen-2-ol bis(2,2,2-trifluoroacetate) (1.0 mg, 14 %). LCMS (MM-ES+APCI, Pos): m/z 636.3 (M+H).

### EXAMPLE 18

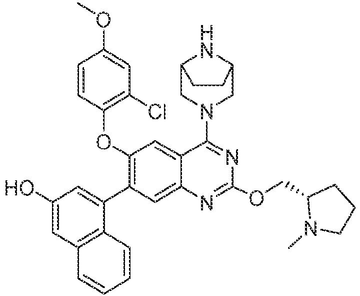

### 4-(4-((1R,5S)-3,8-diazabicyclo[3.2.1]octan-3-yl)-6-(2-chloro-4-methoxyphenoxy)-2-(((S)-1-methylpyrrolidin-2-yl)methoxy)quinazolin-7-yl)naphthalen-2-ol

Synthesized according to Example 17, substituting 2-chloro-4-methoxyphenol in place of 2-fluoro-5-methoxyphenol in step A to afford 4-(4-((1R,5S)-3,8-diazabicyclo[3.2.1]octan-3-yl)-6-(2-chloro-4-methoxyphenoxy)-2-(((S)-1-methylpyrrolidin-2-yl)methoxy)quinazolin-7-yl)naphthalen-2-ol bis(2,2,2-trifluoroacetate) (4.0 mg, 39%). LCMS (MM-ES+APCI, Pos): m/z 652.3 (M+H).

### EXAMPLE 19

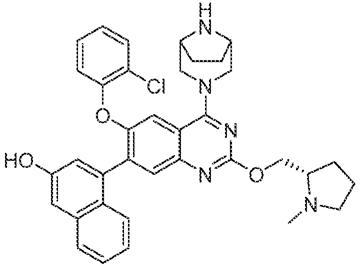

### 4-(4-((1R,5S)-3,8-diazabicyclo[3.2.1]octan-3-yl)-6-(2-chlorophenoxy)-2-(((S)-1-methylpyrrolidin-2-yl)methoxy)quinazolin-7-yl)naphthalen-2-ol

Synthesized according to Example 17, substituting 2-chlorophenol in place of 2-chloro-4-methoxyphenol in step A to afford 4-(4-((1R,5S)-3,8-diazabicyclo[3.2.1]octan-3-yl)-6-(2-chlorophenoxy)-2-(((S)-1-methylpyrrolidin-2-yl)methoxy)quinazolin-7-yl)naphthalen-2-ol bis(2,2,2-trifluoroacetate) (2.0 mg, 18%). LCMS (MM-ES+APCI, Pos): m/z 622.2 (M+H).

### EXAMPLE 20

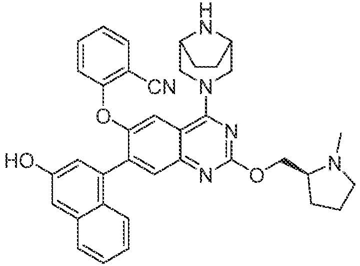

### 2-((4-((1R,5S)-3,8-diazabicyclo[3.2.1]octan-3-yl)-7-(3-hydroxynaphthalen-1-yl)-2-(((S)-1-methylpyrrolidin-2-yl)methoxy)quinazolin-6-yl)oxy)benzonitrile

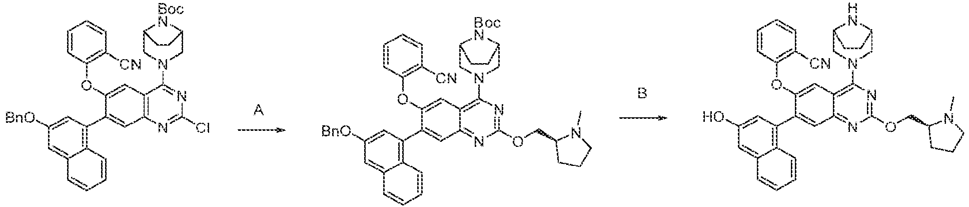

Step A: tert-butyl (1R,5S)-3-(7-(3-(benzyloxy)naphthalen-1-yl)-6-(2-cyanophenoxy)-2-(((S)-1-methylpyrrolidin-2-yl)methoxy)quinazolin-4-yl)-3,8-diazabicyclo[3.2.1]octane-8-carboxylate. A solution of tert-butyl (1R,5S)-3-(7-(3-(benzyloxy)naphthalen-1-yl)-2-chloro-6-(2-cyanophenoxy)quinazolin-4-yl)-3,8-diazabicyclo[3.2.1]octane-8-carboxylate (0.72 ml, 36 µmol), (S)-(1-methylpyrrolidin-2-yl)methanol (12 mg, 0.11 mmol), Cs₂CO₃ (35 mg, 0.11 mmol) and (2-dicyclohexylphosphino-2',6'-diisopropoxy-1,1'-biphenyl)[2-(2'-amino-1,1'-biphenyl)]palladium(II) methanesulfonate (6.0 mg, 7.2 µmol) in 1,4-dioxane (0.7 mL) was sparged with argon for 10 minutes. The reaction vessel was sealed and heated at 100°C for 18 hours. The reaction mixture was cooled to ambient temperature and diluted with 1,4-dioxane (4 mL). The mixture was filtered, concentrated under reduced pressure and purified by flash chromatography (0-10% DCM/MeOH with 0.1% ammonium hydroxide modifier) to afford tert-butyl (1R,5S)-3-(7-(3-(benzyloxy)naphthalen-1-yl)-6-(2-cyanophenoxy)-2-(((S)-1-methylpyrrolidin-2-yl)methoxy)quinazolin-4-yl)-3,8-diazabicyclo[3.2.1]octane-8-carboxylate (18 mg, 62 %) as a brown oil. LCMS (MM-ES+APCI, Pos): m/z 803.3 (M+H).

Step B: 2-((4-((1R,5S)-3,8-diazabicyclo[3.2.1]octan-3-yl)-7-(3-hydroxynaphthalen-1-yl)-2-(((S)-1-methylpyrrolidin-2-yl)methoxy)quinazolin-6-yl)oxy)benzonitrile. tert-butyl (1R,5S)-3-(7-(3-(benzyloxy)naphthalen-1-yl)-6-(2-cyanophenoxy)-2-(((S)-1-methylpyrrolidin-2-yl)methoxy)quinazolin-4-yl)-3,8-diazabicyclo[3.2.1]octane-8-carboxylate (18 mg, 22 µmol), was dissolved in DCM (0.5 mL) and cooled in an ice-water bath. 2,2,2-trifluoroacetic acid (0.5 mL) was added dropwise and the reaction was stirred while warming to ambient temperature. The reaction was concentrated under reduced pressure and redissolved in methanol (0.50 mL). To the reaction mixture was added Pd(OH)₂/C (16 mg, 11 µmol). The reaction mixture was sparged with nitrogen for 5 minutes. Hydrogen was added via balloon and the reaction mixture was stirred at ambient temperature for 15 minutes. The reaction mixture was sparged with nitrogen for 5 minutes, diluted with methanol (2 mL) and filtered. The filtrate was concentrated and purified by reverse phase HPLC (5-95% ACN/H₂O with 0.1% TFA modifier). The fractions containing the desired product were combined and lyophilized to afford 2-((4-((1R,5S)-3,8-diazabicyclo[3.2.1]octan-3-yl)-7-(3-hydroxynaphthalen-1-yl)-2-(((S)-1-methylpyrrolidin-2-yl)methoxy)quinazolin-6-yl)oxy)benzonitrile (3.9 mg, 21 %) as a yellow solid. LCMS (MM-ES+APCI, Pos): m/z 613.3 (M+H).

### EXAMPLE 21

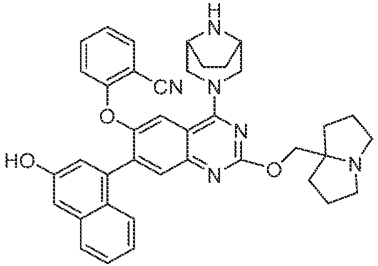

### 2-((4-((1R,5S)-3,8-diazabicyclo[3.2.1]octan-3-yl)-7-(3-hydroxynaphthalen-1-yl)-2-((tetrahydro-1H-pyrrolizin-7a(5H)-yl)methoxy)quinazolin-6-yl)oxy)benzonitrile

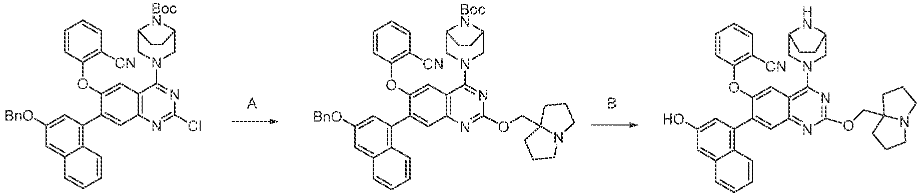

Step A: tert-butyl (1R,5S)-3-(7-(3-(benzyloxy)naphthalen-1-yl)-6-(2-cyanophenoxy)-2-((tetrahydro-1H-pyrrolizin-7a(5H)-yl)methoxy)quinazolin-4-yl)-3,8-diazabicyclo[3.2.1]octane-8-carboxylate. A solution of tert-butyl (1R,5S)-3-(7-(3-(benzyloxy)naphthalen-1-yl)-2-chloro-6-(2-cyanophenoxy)quinazolin-4-yl)-3,8-diazabicyclo[3.2.1]octane-8-carboxylate (31 mg, 43 µmol), Cs₂CO₃ (42 mg, 0.13 mmol), (tetrahydro-1H-pyrrolizin-7a(5H)-yl)methanol (18 mg, 0.13 mmol) and (2-Dicyclohexylphosphino-2',6'-diisopropoxy-1,1'-biphenyl)[2-(2'-amino-1,1'-biphenyl)]palladium(II) methanesulfonate (7.2 mg, 86 µmol) in 1,4-dioxane (1 mL) was sparged with argon for 10 minutes and the reaction vessel was sealed and heated at 100°C for 18 hours. The reaction was diluted with 1,4-dioxane (10 mL), filtered, concentrated and purified by flash chromatography eluting with (0-10% DCM/MeOH with 0.1% ammonium hydroxide modifier) to afford tert-butyl (1R,5S)-3-(7-(3-(benzyloxy)naphthalen-1-yl)-6-(2-cyanophenoxy)-2-((tetrahydro-1H-pyrrolizin-7a(5H)-yl)methoxy)quinazolin-4-yl)-3,8-diazabicyclo[3.2.1]octane-8-carboxylate (23 mg, 64 %) as a yellow oil. LCMS (MM-ES+APCI, Pos): m/z 829.5 (M+H).

Step B: 2-((4-((1R,5S)-3,8-diazabicyclo[3.2.1]octan-3-yl)-7-(3-hydroxynaphthalen-1-yl)-2-((tetrahydro-1H-pyrrolizin-7a(5H)-yl)methoxy)quinazolin-6-yl)oxy)benzonitrile. A solution of tert-butyl (1R,5S)-3-(7-(3-(benzyloxy)naphthalen-1-yl)-6-(2-cyanophenoxy)-2-((tetrahydro-1H-pyrrolizin-7a(5H)-yl)methoxy)quinazolin-4-yl)-3,8-diazabicyclo[3.2.1]octane-8-carboxylate (0.53 mg, 27 µmol) in dichloromethane (0.5 mL) was cooled to 0°C under nitrogen atmosphere. To the reaction mixture was added 2,2,2-trifluoroacetic acid (0.50 mL) and the reaction mixture was stirred at ambient temperature then concentrated. The residue was dissolved in MeOH (0.5 mL). To the reaction mixture was added Pd(OH)₂/C (11 mg, 8.0 µmol). The reaction mixture was sparged with nitrogen for 5 minutes, and then hydrogen was added via balloon. The reaction mixture was stirred for 15 minutes. The reaction was sparged with nitrogen for 5 minutes, diluted with methanol (2 mL) and filtered. The filtrate was concentrated and purified by reverse phase HPLC (5-95% ACN/H₂O with 0.1% TFA). The fractions containing the desired product were combined and lyophilized to afford 2-((4-((1R,5S)-3,8-diazabicyclo[3.2.1]octan-3-yl)-7-(3-hydroxynaphthalen-1-yl)-2-((tetrahydro- 1H-pyrrolizin-7a(5H)-yl)methoxy)quinazolin-6-yl)oxy)benzonitrile (11 mg, 50 % two steps) as a yellow solid. LCMS (MM-ES+APCI, Pos): m/z 639.4 (M+H).

### EXAMPLE 22

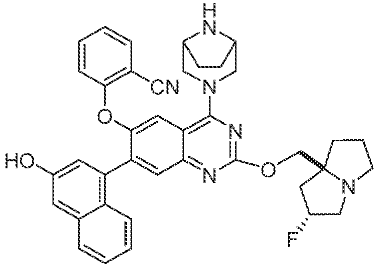

### 2-((4-((1R,5R)-3,8-diazabicyclo[3.2.1]octan-3-yl)-2-(((2R,7aS)-2-fluorotetrahydro-1H-pyrrolizin-7a(5H)-yl)methoxy)-7-(3-hydroxynaphthalen-1-yl)quinazolin-6-yl)oxy)benzonitrile (racemic, trans)

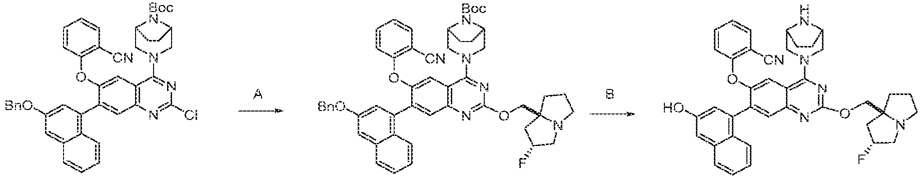

Step A: tert-butyl (1R,5S)-3-(7-(3-(benzyloxy)naphthalen-1-yl)-6-(2-cyanophenoxy)-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate. A solution of tert-butyl (1R,5S)-3-(7-(3-(benzyloxy)naphthalen-1-yl)-2-chloro-6-(2-cyanophenoxy)quinazolin-4-yl)-3,8-diazabicyclo[3.2.1]octane-8-carboxylate (32 mg, 44 µmol), ((2R,7aS)-2-fluorotetrahydro-1H-pyrrolizin-7a(5H)-yl)methanol (21 mg, 0.13 mmol), Cs₂CO₃ (43 mg, 0.13 mmol), (2-Dicyclohexylphosphino-2',6'-diisopropoxy-1,1'-biphenyl)[2-(2'-amino-1,1'-biphenyl)]palladium(II) methanesulfonate (7.4 mg, 9.0 µmol) in 1,4-dioxane (1 mL) was heated at 100°C for 18 hours and was cooled to ambient temperature. The mixture was diluted with EtOAc and washed with 10% aq. potassium carbonate. The organic phase was dried over Na₂SO₄, filtered, concentrated and purified by silica gel chromatography (0-10% DCM/MeOH with 0.1 % NH₄OH modifier) to afford tert-butyl (1R,5S)-3-(7-(3-(benzyloxy)naphthalen-1-yl)-6-(2-cyanophenoxy)-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (19 mg, 51%). LCMS (MM-ES+APCI, Pos): m/z 847.3 (M+H).

Step B: 2-((4-((1R,5R)-3,8-diazabicyclo[3.2.1]octan-3-yl)-2-(((2R,7aS)-2-fluorotetrahydro-1H-pyrrolizin-7a(5H)-yl)methoxy)-7-(3-hydroxynaphthalen-1-yl)quinazolin-6-yl)oxy)benzonitrile. tert-butyl (1R,5S)-3-(7-(3-(benzyloxy)naphthalen-1-yl)-6-(2-cyanophenoxy)-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (19 mg, 22 µmol), was dissolved in DCM (0.50 mL) and cooled in an ice/water bath. 2,2,2-trifluoroacetic acid (0.50 mL) was added dropwise and the reaction was stirred at ambient temperature. The reaction was concentrated under reduced pressure and dissolved in methanol (0.5 mL). To the reaction mixture was added Pd(OH)₂/C (9.5 mg, 6.7 µmol) and the reaction mixture was sparged with nitrogen for 5 minutes. Hydrogen was added via balloon and the reaction mixture was stirred at ambient temperature for 15 minutes. The reaction mixture was sparged with nitrogen for 5 minutes, diluted with methanol (2.0 mL) and filtered. The filtrate was concentrated and purified by reverse phase HPLC (5-95% ACN/H₂O with 0.1%TFA). The fractions containing the desired product were combined and lyophilized to afford 2-((4-((1R,5R)-3,8-diazabicyclo[3.2.1]octan-3-yl)-2-(((2R,7aS)-2-fluorotetrahydro-1H-pyrrolizin-7a(5H)-yl)methoxy)-7-(3-hydroxynaphthalen-1-yl)quinazolin-6-yl)oxy)benzonitrile (7.0 mg, 7.9 µmol, 35 %) as a white solid. LCMS (MM-ES+APCI, Pos): m/z 657.3 (M+H).

### EXAMPLE 23

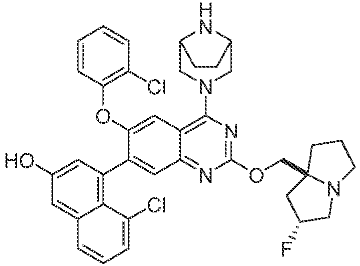

### 4-(4-((1R,5S)-3,8-diazabicyclo[3.2.1]octan-3-yl)-6-(2-chlorophenoxy)-2-(((2R,7aS)-2-fluorotetrahydro-1H-pyrrolizin-7a(5H)-yl)methoxy)quinazolin-7-yl)-5-chloronaphthalen-2-ol (racemic, trans)

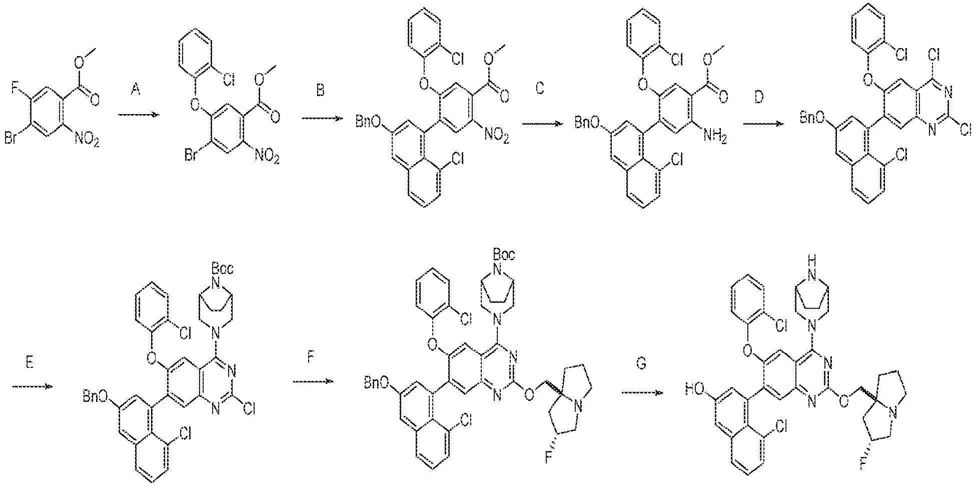

Step A: methyl 4-bromo-5-(2-chlorophenoxy)-2-nitrobenzoate. To a solution of methyl 4-bromo-5-fluoro-2-nitrobenzoate (1.0 g, 3.6 mmol) and 2-chlorophenol (0.46 g, 3.6 mmol) in DMSO (9 mL) was added potassium carbonate (1.0 g, 7.2 mmol). The reaction mixture was sparged with nitrogen and heated at 50°C for 2 hours. The reaction mixture was cooled to ambient temperature and partitioned between water (30 mL) and ethyl acetate (10 mL). The phases were separated and the aqueous was extracted with EtOAc (3 x 50 mL). The organic phases were combined and dried over Na₂SO₄, filtered, concentrated under reduced pressure and purified by silica gel chromatography (0-50% hexanes/EtOAc) to afford methyl 4-bromo-5-(2-chlorophenoxy)-2-nitrobenzoate (1.3 g, 97 %). 1H NMR (400 MHz, CDCl3) δ 8.33 (s, 1H), 7.57-7.51 (m, 1H), 7.42-7.34 (m, 1H), 7.34-7.27 (m, 1H), 7.21-7.15 (m, 1H), 6.75 (s, 1H), 3.85 (s, 3H).

Step B: methyl 4-(3-(benzyloxy)-8-chloronaphthalen-1-yl)-5-(2-chlorophenoxy)-2-nitrobenzoate. A mixture of methyl 4-bromo-5-(2-chlorophenoxy)-2-nitrobenzoate (0.30 g, 0.78 mmol), 2-(3-(benzyloxy)-8-chloronaphthalen-1-yl)-4,4,5,5-tetramethyl-1,3,2-dioxaborolane (0.46 g, 1.2 mmol), Pd(Ph₃P)₄ (0.14 g, 0.12 mmol), potassium carbonate (1.1 mL, 2.3 mmol, 2.0 M in water) in 1,4- dioxane (8 mL) was sparged with argon for 5 minutes and heated at 100°C for 48 hours. The reaction mixture was cooled to ambient temperature, diluted with EtOAc (50 mL), and filtered through a pad of celite. The organics were dried over Na₂SO₄, filtered and concentrated under reduced pressure. The reaction mixture was purified by silica gel chromatography (0-25% EtOAc/hexanes) to afford methyl 4-(3-(benzyloxy)-8-chloronaphthalen-1-yl)-5-(2-chlorophenoxy)-2-nitrobenzoate (0.24 g, 54 %) as a white solid. 1H NMR (400 MHz, CDCl₃) δ 8.06 (s, 1H), 7.72 (d, 1H), 7.49 (d, 2H), 7.45-7.39 (m, 4H), 7.39-7.31 (m, 3H), 7.29-7.24 (m,2H) , 7.20-7.11 (m, 2H), 6.71 (s, 1 H), 5.22 (s, 2H), 3.90 (s, 3H).

Step C: methyl 2-amino-4-(3-(benzyloxy)-8-chloronaphthalen-1-yl)-5-(2-chlorophenoxy)benzoate. To a solution of methyl 4-(3-(benzyloxy)-8-chloronaphthalen-1-yl)-5-(2-chlorophenoxy)-2-nitrobenzoate (0.24 g, 0.42 mmol) in THF (4 mL) were added a saturated ammonium chloride solution (1.0 mL) and zinc dust (0.27 g, 4.2 mmol). The reaction mixture was stirred at ambient temperature for 18 hours, diluted with water/EtOAc and filtered through GF/F filter paper. The filtrate was extracted with EtOAc. The organic phases were combined and dried over Na₂SO₄, filtered and concentrated under reduced pressure to afford methyl 2-amino-4-(3-(benzyloxy)-8-chloronaphthalen-1-yl)-5-(2-chlorophenoxy)benzoate (0.20 g, 88%) as a white solid. LCMS (MM-ES+APCI, Pos): m/z 544.1 (M+H).

Step D: 7-(3-(benzyloxy)-8-chloronaphthalen-1-yl)-2,4-dichloro-6-(2-chlorophenoxy)quinazoline. To an 0°C solution of methyl 2-amino-4-(3-(benzyloxy)-8-chloronaphthalen-1-yl)-5-(2-chlorophenoxy)benzoate (0.20 g, 0.37 mmol) in THF (1 mL) was added 2,2,2-trichloroacetyl isocyanate (73 mg, 0.39 mmol). The reaction mixture was stirred for 5 minutes and warmed to ambient temperature over 30 minutes. The reaction mixture was concentrated under reduce pressure. The solids were suspended in methanol (1 mL) and a solution of NH₃ in methanol (1.5 mL, 11 mmol) was added. The mixture was stirred at room temperature overnight and concentrated under reduced pressure. The reaction mixture was dissolved in POCl₃ (5 mL) and DIEA (0.17 mL, 0.13 mg, 0.97 mmol) was added. The reaction mixture was heated at reflux for 18 hours, concentrated under reduced pressure and diluted in EtOAc (40 mL). The organics were washed with 10% aq. K₂CO₃ (3 x 15 mL), brine (25 mL), dried over Na₂SO₄, filtered and concentrated to afford 7-(3-(benzyloxy)-8-chloronaphthalen-1-yl)-2,4-dichloro-6-(2-chlorophenoxy)quinazoline (0.22 g, 100%) that was used as crude in the next reaction. LCMS (MM-ES+APCI, Pos): m/z 591.0 (M+H).

Step E: tert-butyl (1R,5S)-3-(7-(3-(benzyloxy)-8-chloronaphthalen-1-yl)-2-chloro-6-(2-chlorophenoxy)quinazolin-4-yl)-3,8-diazabicyclo[3.2.1]octane-8-carboxylate. A solution of 7-(3-(benzyloxy)-8-chloronaphthalen-1-yl)-2,4-dichloro-6-(2-chlorophenoxy)quinazoline (0.20 g, 0.34 mmol), tert-butyl 3,8-diazabicyclo[3.2.1]octane-8-carboxylate (72 mg, 0.34 mmol), and DIEA (0.13 g, 1.0 mmol) in 1,4-dioxane (3 mL) was stirred at ambient temperature for 45 minutes. The reaction was diluted with water (50 mL) and extracted with EtOAc (3 x 15 mL). The organic phases were combined, washed with brine (15 mL), dried over Na₂SO₄, filtered and concentrated under reduced pressure. The residue was purified by flash chromatography (0-60% DCM/EtOAc) to afford tert-butyl (1R,5S)-3-(7-(3-(benzyloxy)-8-chloronaphthalen-1-yl)-2-chloro-6-(2-chlorophenoxy)quinazolin-4-yl)-3,8-diazabicyclo[3.2.1]octane-8-carboxylate (0.16 g, 62 %) as a white solid. LCMS (MM-ES+APCI, Pos): m/z 763.3 (M+H).

Step F: tert-butyl (1R,5S)-3-(7-(3-(benzyloxy)-8-chloronaphthalen-1-yl)-6-(2-chlorophenoxy)-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate. A mixture of tert-butyl (1R,5S)-3-(7-(3-(benzyloxy)-8-chloronaphthalen-1-yl)-2-chloro-6-(2-chlorophenoxy)quinazolin-4-yl)-3,8-diazabicyclo[3.2.1]octane-8-carboxylate (30 mg, 39 µmol), ((2R,7aS)-2-fluorotetrahydro-1H-pyrrolizin-7a(5H)-yl)methanol (19 mg, 0.12 mmol), Cs₂CO₃ (38 mg, 0.12 mmol), and (2-Dicyclohexylphosphino-2',6'-diisopropoxy-1,1'-biphenyl)[2-(2'-amino-1,1'-biphenyl)]palladium(II) methanesulfonate (6.5 mg, 7.8 µmol) in 1,4-dioxane (1 mL) was sparged with argon for 5 minutes and heated at 100°C overnight. The reaction mixture was diluted with 1,4-dioxane (5 mL), filtered, concentrated and purified by reverse phase chromatography (5-95% ACN/H₂O with 0.1%TFA modifier). The desired fractions were combined and diluted with aqueous saturated NaHCO₃ (15 mL) and extracted with 4:1 DCM/IPA (3 x 15 mL). The organic phases were dried over Na₂SO₄, filtered and concentrated to afford tert-butyl (1R,5S)-3-(7-(3-(benzyloxy)-8-chloronaphthalen-1-yl)-6-(2-chlorophenoxy)-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (13 mg, 37 % yield) as a pale-yellow oil. LCMS (MM-ES+APCI, Pos): m/z 890.3 (M+H).

Step G: 4-(4-((1R,5S)-3,8-diazabicyclo[3.2.1]octan-3-yl)-6-(2-chlorophenoxy)-2-(((2R,7aS)-2-fluorotetrahydro-1H-pyrrolizin-7a(5H)-yl)methoxy)quinazolin-7-yl)-5-chloronaphthalen-2-ol. To an 0°C solution of tert-butyl (1R,5S)-3-(7-(3-(benzyloxy)-8-chloronaphthalen-1-yl)-6-(2-chlorophenoxy)-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (13 mg, 15 µmol) in DCM (0.5 mL) was added 2,2,2-trifluoroacetic acid (0.5 mL). The reaction was stirred at ambient temperature, concentrated and dissolved in methanol (0.5 mL). To the reaction mixture was added Pd(OH)₂/C (5.1 mg, 3.6 µmol). The reaction was sparged with nitrogen for 5 minutes followed by addition of hydrogen via balloon and the reaction was stirred for 15 minutes. The reaction mixture was sparged with nitrogen for 5 minutes, diluted with methanol (2.0 mL) and filtered. The filtrate was concentrated and purified by reverse phase chromatography (5-60% ACN/H₂O with 0.1% TFA modifier). The desired fractions were combined and lyophilized to afford 4-(4-((1R,5S)-3,8-diazabicyclo[3.2.1]octan-3-yl)-6-(2-chlorophenoxy)-2-(((2R,7aS)-2-fluorotetrahydro-1H-pyrrolizin-7a(5H)-yl)methoxy)quinazolin-7-yl)-5-chloronaphthalen-2-ol (3.6 mg, 27 %) as a yellow solid. LCMS (MM-ES+APCI, Pos): m/z 700.2 (M+H).

### EXAMPLE 24

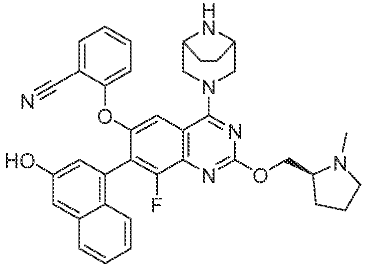

### 2-((4-((1R,5S)-3,8-diazabicyclo[3.2.1]octan-3-yl)-8-fluoro-7-(3-hydroxynaphthalen-1-yl)-2-(((S)-1-methylpyrrolidin-2-yl)methoxy)quinazolin-6-yl)oxy)benzonitrile

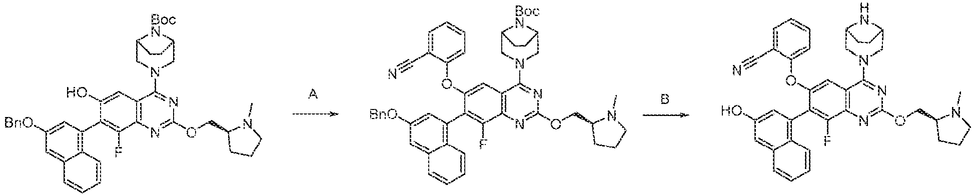

Step A: tert-butyl (1R,5S)-3-(7-(3-(benzyloxy)naphthalen-1-yl)-6-(2-cyanophenoxy)-8-fluoro-2-(((S)-1-methylpyrrolidin-2-yl)methoxy)quinazolin-4-yl)-3,8-diazabicyclo[3.2.1]octane-8-carboxylate. To a solution of tert-butyl (1R,5S)-3-(7-(3-(benzyloxy)naphthalen-1-yl)-8-fluoro-6-hydroxy-2-(((S)-1-methylpyrrolidin-2-yl)methoxy)quinazolin-4-yl)-3,8-diazabicyclo[3.2.1]octane-8-carboxylate (25 mg, 30 µmol) in DMSO (0.6 mL) were added K₂CO₃ (17 mg, 0.12 mmol) and 2-fluorobenzonitrile (13 µL, 0.12 mmol). The reaction mixture was heated at 70°C for 5 hours, cooled to ambient temperature, diluted with water (10 mL) and extracted with 4:1 DCM/IPA (3x 10 mL). The organic phases were combined, dried over Na₂SO₄, filtered and concentrated under reduced pressure. The residue was purified by reverse phase chromatography (5-95% AC/H₂O with 0.1% TFA additive) and the fractions containing product were lyophilized to afford tert-butyl (1R,5S)-3-(7-(3-(benzyloxy)naphthalen-1-yl)-6-(2-cyanophenoxy)-8-fluoro-2-(((S)-1-methylpyrrolidin-2-yl)methoxy)quinazolin-4-yl)-3,8-diazabicyclo[3.2.1]octane-8-carboxylate (7.0 mg, 28 %) as a brown solid. LCMS (MM-ES+APCI, Pos): m/z 821.3 (M+H).

Step B: 2-((4-((1R,5S)-3,8-diazabicyclo[3.2.1]octan-3-yl)-8-fluoro-7-(3-hydroxynaphthalen-1-yl)-2-(((S)-1-methylpyrrolidin-2-yl)methoxy)quinazolin-6-yl)oxy)benzonitrile. To a solution of tert-butyl (1R,5S)-3-(7-(3-(benzyloxy)naphthalen-1-yl)-6-(2-cyanophenoxy)-8-fluoro-2-(((S)-1-methylpyrrolidin-2-yl)methoxy)quinazolin-4-yl)-3,8-diazabicyclo[3.2.1]octane-8-carboxylate (9.0 mg, 11 µmol) in DCM (0.5 mL) was added 2,2,2-trifluoracetic acid (0.5 mL) at 0°C and the reaction mixture was stirred under nitrogen at ambient temperature for 30 minutes. The reaction mixture was concentrated under reduced pressure and dissolved in methanol (0.5 mL). To the reaction mixture was added Pd(OH)₂/C (6.2 mg, 4.4 µmol) and the reaction sparged with nitrogen. Hydrogen was added via balloon and the reaction mixture was stirred at ambient temperature for 30 minutes. The reaction was sparged with nitrogen for 5 minutes, diluted with methanol (2.0 mL) and filtered. The residue was purified by HPLC (5-95% ACN/H₂O with 0.1%TFA) and fractions containing product was lyophilized to afford 2-((4-((1R,5S)-3,8-diazabicyclo[3.2.1]octan-3-yl)-8-fluoro-7-(3-hydroxynaphthalen-1-yl)-2-(((S)-1-methylpyrrolidin-2-yl)methoxy)quinazolin-6-yl)oxy)benzonitrile (4.0 mg, 58 %) as a yellow solid. LCMS (MM-ES+APCI, Pos): m/z 631.3 (M+H).

### EXAMPLE 25

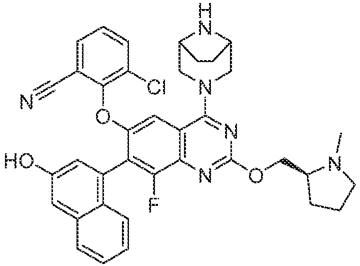

### 2-((4-((1R,5S)-3,8-diazabicyclo[3.2.1]octan-3-yl)-8-fluoro-7-(3-hydroxynaphthalen-1-yl)-2-(((S)-1-methylpyrrolidin-2-yl)methoxy)quinazolin-6-yl)oxy)-3-chlorobenzonitrile

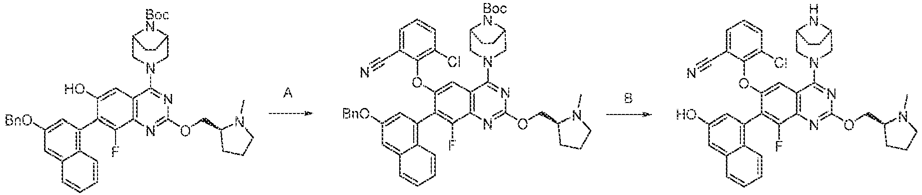

Step A: tert-butyl (1R,5S)-3-(7-(3-(benzyloxy)naphthalen-1-yl)-6-(2-chloro-6-cyanophenoxy)-8-fluoro-2-(((S)-1-methylpyrrolidin-2-yl)methoxy)quinazolin-4-yl)-3,8-diazabicyclo[3.2.1]octane-8-carboxylate. To a solution of tert-butyl (1R,5S)-3-(7-(3-(benzyloxy)naphthalen-1-yl)-8-fluoro-6-hydroxy-2-(((S)-1-methylpyrrolidin-2-yl)methoxy)quinazolin-4-yl)-3,8-diazabicyclo[3.2.1]octane-8-carboxylate (10 mg, 14 µmol) in DMSO (0.5 mL) were added K₂CO₃ (5.8 mg, 42 µmol) and 3-chloro-2-fluorobenzonitrile (6.5 mg, 42 µmol). The reaction mixture was sparged with argon and heated at 70°C for 2 hours. The reaction mixture was cooled to ambient temperature and purified by reverse phase chromatography (5-95% ACN/H₂O with 0.1%TFA) to afford tert-butyl (1R,5S)-3-(7-(3-(benzyloxy)naphthalen-1-yl)-6-(2-chloro-6-cyanophenoxy)-8-fluoro-2-(((S)-1-methylpyrrolidin-2-yl)methoxy)quinazolin-4-yl)-3,8-diazabicyclo[3.2.1]octane-8-carboxylate (11 mg, 91 %) as a pale-yellow solid after lyophilization. LCMS (MM-ES+APCI, Pos): m/z 855.3 (M+H).

Step B: 2-((4-((1R,5S)-3,8-diazabicyclo[3.2.1]octan-3-yl)-8-fluoro-7-(3-hydroxynaphthalen-1-yl)-2-(((S)-1-methylpyrrolidin-2-yl)methoxy)quinazolin-6-yl)oxy)-3-chlorobenzonitrile. To a solution of tert-butyl (1R,5S)-3-(7-(3-(benzyloxy)naphthalen-1-yl)-6-(2-chloro-6-cyanophenoxy)-8-fluoro-2-(((S)-1-methylpyrrolidin-2-yl)methoxy)quinazolin-4-yl)-3,8-diazabicyclo[3.2.1]octane-8-carboxylate (11 mg, 11 µmol) in DCM (0.5 mL) was added 2,2,2-trifluoracetic acid (0.5 mL) at 0°C and the reaction mixture was stirred at ambient temperature for 30 minutes. The reaction mixture was concentrated and dissolved in methanol (0.5 mL). To the mixture was added Pd(OH)₂/C (6.3 mg, 4.5 µmol) and the reaction mixture was sparged with nitrogen for 5 minutes. To the reaction was added hydrogen via balloon and the reaction mixture was stirred for 45 minutes. The reaction mixture was sparged with nitrogen for 5 minutes, diluted with methanol (2.0 mL) and filtered. The filtrate was concentrated and purified by HPLC (5-95% ACN/H₂O with 0.1% TFA). Fractions containing product were lyophilized to afford 2-((4-((1R,5S)-3,8-diazabicyclo[3.2.1]octan-3-yl)-8-fluoro-7-(3-hydroxynaphthalen-1-yl)-2-(((S)-1-methylpyrrolidin-2-yl)methoxy)quinazolin-6-yl)oxy)-3-chlorobenzonitrile (3.4 mg, 39 %) as a pale yellow solid. LCMS (MM-ES+APCI, Pos): m/z 665.3 (M+H).

### EXAMPLE 26

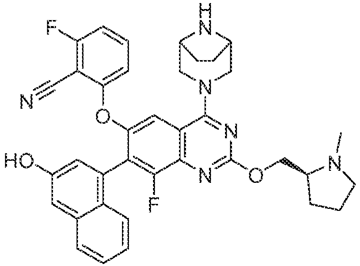

### 2-((4-((1R,5S)-3,8-diazabicyclo[3.2.1]octan-3-yl)-8-fluoro-7-(3-hydroxynaphthalen-1-yl)-2-(((S)-1-methylpyrrolidin-2-yl)methoxy)quinazolin-6-yl)oxy)-6-fluorobenzonitrile

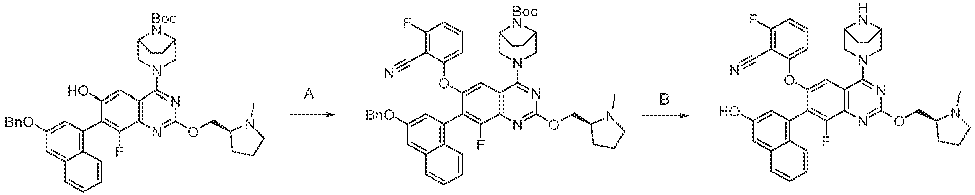

Step A: tert-butyl (1R,5S)-3-(7-(3-(benzyloxy)naphthalen-1-yl)-6-(2-cyano-3-fluorophenoxy)-8-fluoro-2-(((S)-1-methylpyrrolidin-2-yl)methoxy)quinazolin-4-yl)-3,8-diazabicyclo[3.2.1]octane-8-carboxylate. To a solution of tert-butyl (1R,5S)-3-(7-(3-(benzyloxy)naphthalen-1-yl)-8-fluoro-6-hydroxy-2-(((S)-1-methylpyrrolidin-2-yl)methoxy)quinazolin-4-yl)-3,8-diazabicyclo[3.2.1]octane-8-carboxylate (5.0 mg, 6.9 µmol) in DMSO (0.25 mL) were added K₂CO₃ (5.8 mg, 42 µmol) and 2,6-difluorobenzonitrile (3.9 mg, 28 µmol). The reaction mixture was sparged with argon, heated to 50°C for 3 hours and cooled to ambient temperature. The mixture was purified by reverse phase chromatography (5-95% ACN/H₂O with 0.1%TFA). Fractions containing product were lyophilized to afford tert-butyl (1R,5S)-3-(7-(3-(benzyloxy)naphthalen-1-yl)-6-(2-cyano-3-fluorophenoxy)-8-fluoro-2-(((S)-1-methylpyrrolidin-2-yl)methoxy)quinazolin-4-yl)-3,8-diazabicyclo[3.2.1]octane-8-carboxylate (6.3 mg, 95 %) as a white solid. LCMS (MM-ES+APCI, Pos): m/z 839.5 (M+H).

Step B: 2-((4-((1R,5S)-3,8-diazabicyclo[3.2.1]octan-3-yl)-8-fluoro-7-(3-hydroxynaphthalen-1-yl)-2-(((S)-1-methylpyrrolidin-2-yl)methoxy)quinazolin-6-yl)oxy)-6-fluorobenzonitrile. To an solution of tert-butyl (1R,5S)-3-(7-(3-(benzyloxy)naphthalen-1-yl)-6-(2-cyano-3-fluorophenoxy)-8-fluoro-2-(((S)-1-methylpyrrolidin-2-yl)methoxy)quinazolin-4-yl)-3,8-diazabicyclo[3.2.1]octane-8-carboxylate (16 mg, 17 µmol) in DCM (0.5 mL) was added 2,2,2-trifluoracetic acid (0.5 mL) at 0°C and the reaction mixture was stirred under nitrogen for 30 minutes. The mixture was concentrated and dissolved in methanol (0.5 mL). To the reaction mixture was added Pd(OH)₂/C (9.4 mg, 6.7 µmol) and the reaction mixture was sparged with nitrogen for 5 minutes. Hydrogen was introduced via balloon and the reaction mixture was stirred for 15 minutes. The reaction mixture was sparged with nitrogen for 5 minutes, diluted with methanol (2 mL), filtered and concentrated under reduced pressure. The residue was purified by HPLC (5-95% ACN/H₂O with 0.1% TFA). Fractions containing product were lyophilized to afford 2-((4-((1R,5S)-3,8-diazabicyclo[3.2.1]octan-3-yl)-8-fluoro-7-(3-hydroxynaphthalen-1-yl)-2-(((S)-1-methylpyrrolidin-2-yl)methoxy)quinazolin-6-yl)oxy)-6-fluorobenzonitrile (6.4 mg, 59%) as a white solid. LCMS (MM-ES+APCI, Pos): m/z 649.2 (M+H).

### EXAMPLE 27

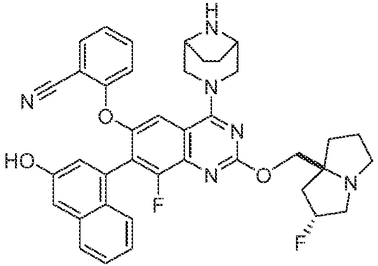

### 2-((4-((1R,5S)-3,8-diazabicyclo[3.2.1]octan-3-yl)-8-fluoro-2-(((2R,7aS)-2-fluorotetrahydro-1H-pyrrolizin-7a(5H)-yl)methoxy)-7-(3-hydroxynaphthalen-1-yl)quinazolin-6-yl)oxy)benzonitrile (racemic, trans)

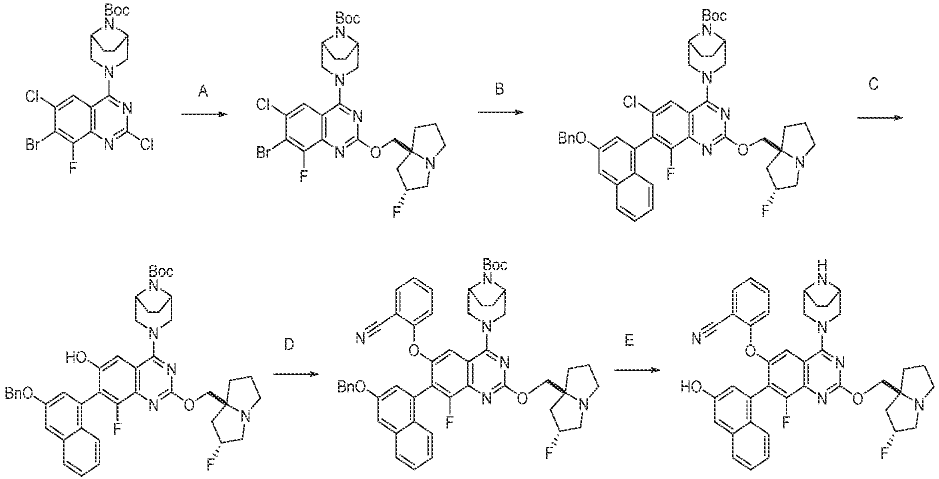

Step A: tert-butyl (1R,5S)-3-(7-bromo-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate. To a solution of tert-butyl (1R,5S)-3-(7-bromo-2,6-dichloro-8-fluoroquinazolin-4-yl)-3,8-diazabicyclo[3.2.1]octane-8-carboxylate (0.94 g, 1.9 mmol), ((2R,7aS)-2-fluorotetrahydro-1H-pyrrolizin-7a(5H)-yl)methanol (0.59 g, 3.7 mmol), DIEA (1.6 mL, 9.3 mmol) and 4 Å molecular sieve powder (0.4 g) in dioxane (10 mL) was heated at 90 °C for 88 hours. The reaction mixture was cooled to ambient temperature and filtered. The filtrate was partitioned between water (30 mL) and EtOAc (30 mL). The aqueous layer was extracted with EtOAc (2 x 30 mL). The combined organic phases were washed with brine (20 mL), dried over Na₂SO₄, filtered and concentrated. The residue was purified by silica gel chromatography (0-5% DCM/MeOH) to afford tert-butyl (1R,5S)-3-(7-bromo-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (0.39 g, 33%) as a beige foam. LCMS (MM-ES+APCI, Pos): m/z 628.1 (M+H).

Step B: tert-butyl (1R,5S)-3-(7-(3-(benzyloxy)naphthalen-1-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate. A solution of tert-butyl (1R,5S)-3-(7-bromo-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (80 mg, 0.13 mmol), 2-(3-(benzyloxy)naphthalen-1-yl)-4,4,5,5-tetramethyl-1,3,2-dioxaborolane (48 mg, 0.13 mmol), Pd(Ph₃P)₄ (29 mg, 0.025 mmol) in 1,4-dioxane (1 mL) and aqueous K₂CO₃ (0.25 mL, 0.50 mmol, 2.0 M) was sparged with argon for 5 minutes and then heated at 100°C for 18 hours. The reaction was diluted with 1,4-dioxane (2 mL), filtered through celite and concentrated under reduced pressure. The reaction mixture was purified by reverse phase chromatography (5-95% ACN/H₂O with 0.1%TFA). The desired fractions were combined and diluted with 10% aqueous K₂CO₃ (50 mL). The aqueous phase was extracted with 4:1 DCM/IPA (3x 20 mL). The organics were combined, dried over Na₂SO₄, filtered and concentrated under reduced pressure to afford tert-butyl (1R,5S)-3-(7-(3-(benzyloxy)naphthalen-1-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (62 mg, 62 %) as a brown solid. LCMS (MM-ES+APCI, Pos): m/z 728.3 (M+H).

Step C: tert-butyl (1R,5S)-3-(7-(3-(benzyloxy)naphthalen-1-yl)-8-fluoro-2-(((2R,7aS)-2-fluorotetrahydro-1H-pyrrolizin-7a(5H)-yl)methoxy)-6-hydroxyquinazolin-4-yl)-3,8-diazabicyclo[3.2.1]octane-8-carboxylate. A solution of tert-butyl (1R,5S)-3-(7-(3-(benzyloxy)naphthalen-1-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (20 mg, 26 µmol), di-tert-b'ty'(2',4',6'-triisopropy'-[1,1'-biphenyl]-2-yl)phosphane (13 mg, 31 µmol), KOH (5.7 mg, 0.10 mmol) and Pd(dba)₂ (9.4 mg, 10 µmol) in H₂O (1 mL) and 1,4-dioxane (1 mL) was sparged with argon for 5 minutes then heated to 100°C for 3 hours. The reaction was concentrated and purified by reverse phase chromatography (5-95% ACN/H₂O with 0.1%TFA). Fractions containing product were lyophilized to afford tert-butyl (1R,5S)-3-(7-(3-(benzyloxy)naphthalen-1-yl)-8-fluoro-2-(((2R,7aS)-2-fluorotetrahydro-1H-pyrrolizin-7a(5H)-yl)methoxy)-6-hydroxyquinazolin-4-yl)-3,8-diazabicyclo[3.2.1]octane-8-carboxylate (13 mg, 68 %) as a yellow solid. LCMS (MM-ES+APCI, Pos): m/z 764.3 (M+H).

Step D: tert-butyl (1R,5S)-3-(7-(3-(benzyloxvy)naphthalen-1-yl)-6-(2-cyanophenoxy)-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate. To a solution of tert-butyl (1R,5S)-3-(7-(3-(benzyloxy)naphthalen-1-yl)-8-fluoro-2-(((2R,7aS)-2-fluorotetrahydro-1H-pyrrolizin-7a(5H)-yl)methoxy)-6-hydroxyquinazolin-4-yl)-3,8-diazabicyclo[3.2.1]octane-8-carboxylate (13 mg, 17 µmol) in DMSO (0.5 mL) was added K₂CO₃ (14 mg, 0.10 mmol) and 2-fluorobenzonitrile (11 µL, 0.10 mmol). The reaction mixture was sparged with argon and heated at 70°C for 5 hours. The mixture was cooled to ambient temperature and purified by reverse phase chromatography (5-95% ACN/H₂O with 0.1% TFA). Fractions containing product were lyophilized to afford tert-butyl (1R,5S)-3-(7-(3-(benzyloxy)naphthalen-1-yl)-6-(2-cyanophenoxy)-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (3.8 mg, 25 % yield) as a yellow solid. LCMS (MM-ES+APCI, Pos): m/z 864.9 (M+H).

Step E: 2-((4-((1R,5S)-3,8-diazabicyclo[3.2.1]octan-3-yl)-8-fluoro-2-(((2R,7aS)-2-fluorotetrahydro-1H-pyrrolizin-7a(5H)-yl)methoxy)-7-(3-hydroxynaphthalen-1-yl)quinazolin-6-yl)oxy)benzonitrile. To an ice-cold solution of tert-butyl (1R,5S)-3-(7-(3-(benzyloxy)naphthalen-1-yl)-6-(2-cyanophenoxy)-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (3.3 mg, 3.8 µmol) in DCM (0.5 mL) was added 2,2,2-trifluoracetic acid (0.5 mL) and the reaction mixture was stirred at ambient temperature for 30 minutes. The reaction mixture was concentrated and dissolved in methanol (0.5 mL). To the reaction mixture was added Pd(OH)₂/C (5.4 mg, 3.8 µmol) and the reaction mixture was sparged with nitrogen for 5 minutes. Hydrogen was introduced via balloon and reaction mixture was stirred for 15 minutes. The reaction mixture was sparged with nitrogen for 5 minutes, diluted with methanol (2 mL) and filtered. The filtrate was concentrated and purified by HPLC (5-95% ACN/H₂O with 0.1% TFA). The fractions containing the desired product were combined and lyophilized to afford 2-((4-((1R,5S)-3,8-diazabicyclo[3.2.1]octan-3-yl)-8-fluoro-2-(((2R,7aS)-2-fluorotetrahydro-1H-pyrrolizin-7a(5H)-yl)methoxy)-7-(3-hydroxynaphthalen-1-yl)quinazolin-6-yl)oxy)benzonitrile (2.0 mg, 58 %) as a white solid. LCMS (MM-ES+APCI, Pos): m/z 675.3 (M+H).

### EXAMPLE 28

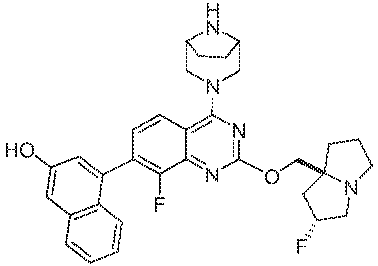

### 4-(4-((1R,5S)-3,8-diazabicyclo[3.2.1]octan-3-yl)-8-fluoro-2-(((2R,7aS)-2-fluorotetrahydro-1H-pyrrolizin-7a(5H)-yl)methoxy)quinazolin-7-yl)naphthalen-2-ol (trans, racemic)

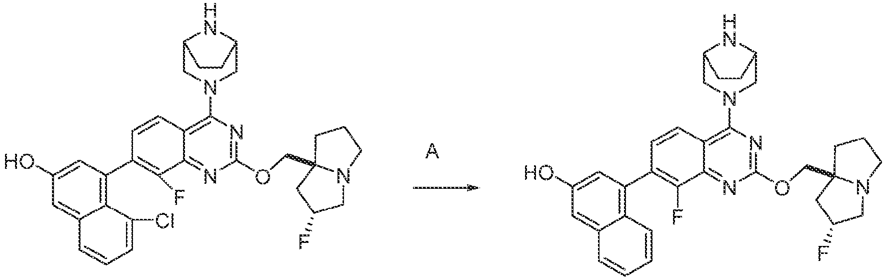

Step A: 4-(4-((1R,5S)-3,8-diazabicyclo[3.2.1]octan-3-yl)-8-fluoro-2-(((2R,7aS)-2-fluorotetrahydro-1H-pyrrolizin-7a(5H)-yl)methoxy)quinazolin-7-yl)naphthalen-2-ol. To a solution of 4-(4-((1R,5S)-3,8-diazabicyclo[3.2.1]octan-3-yl)-8-fluoro-2-(((2R,7aS)-2-fluorotetrahydro-1H-pyrrolizin-7a(5H)-yl)methoxy)quinazolin-7-yl)-5-chloronaphthalen-2-ol (8.0 mg, 9.8 µmol) in methanol (0.5 mL) was added Pd(OH)₂/C (5.5 mg, 3.9 µmol) and the reaction mixture was sparged with nitrogen for 5 minutes. Hydrogen was introduced via balloon and the reaction mixture was stirred at ambient temperature for 4 hours. The reaction mixture was sparged with nitrogen for 5 minutes, diluted with methanol (3.0 mL) and filtered. The filtrate was concentrated and purified by reverse-phase chromatography (5-95% ACN/H₂O with 0.1% TFA). Fractions containing product were lyophilized to afford 4-(4-((1R,5S)-3,8-diazabicyclo[3.2.1]octan-3-yl)-8-fluoro-2-(((2R,7aS)-2-fluorotetrahydro-1H-pyrrolizin-7a(5H)-yl)methoxy)quinazolin-7-yl)naphthalen-2-ol (2.2 mg, 40 %) as a white solid. LCMS (MM-ES+APCI, Pos): m/z 558.2 (M+H).

### EXAMPLE 29

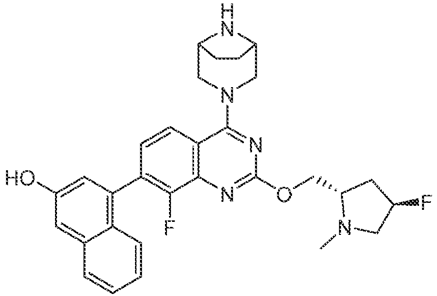

### 4-(4-((1R,5S)-3,8-diazabicyclo[3.2.1]octan-3-yl)-8-fluoro-2-(((2S,4R)-4-fluoro-1-methylpyrrolidin-2-yl)methoxy)quinazolin-7-yl)naphthalen-2-ol

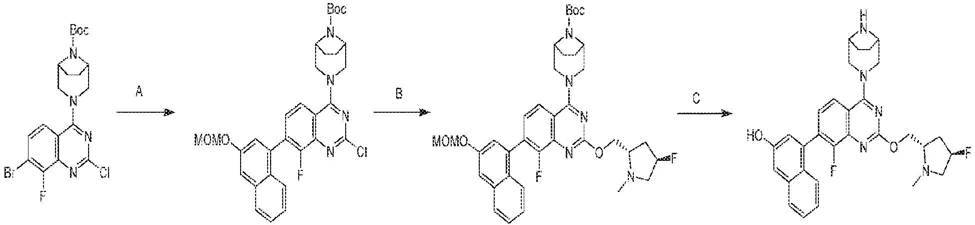

Step A: tert-butyl (1R,5S)-3-(2-chloro-8-fluoro-7-(3-(methoxymethoxy)naphthalen-1-yl)quinazolin-4-yl)-3,8-diazabicyclo[3.2.1]octane-8-carboxylate A mixture of tert-butyl 3-(7-bromo-2-chloro-8-fluoro-quinazolin-4-yl)-3,8-diazabicyclo[3.2.1]octane-8-carboxylate (300 mg, 636 µmol, 1.00 *eq),* 2-[3-(methoxymethoxy)-1-naphthyl]-4,4,5,5-tetramethyl-1,3,2-dioxaborolane (200 mg, 636 µmol, 1.00 *eq),* sodium carbonate (135 mg, 1.27 mmol, 2.00 *eq)* and ditert-butyl(cyclopentyl)phosphane;dichloropalladium;iron (82.9 mg, 127 µmol, 0.20 *eq)* in water (3.00 mL) and tetrahydrofuran (15.0 mL) was degassed and purged with nitrogen for 3 times, and then stirred at 15 °C for 16 hours under nitrogen atmosphere. The reaction mixture was extracted with ethyl acetate (30.0 mL × 3). The combined organic layers were washed with brine (20.0 mL × 2), dried, filtered and concentrated under reduced pressure to give a residue. The residue was purified by prep-TLC (SiO₂, DCM: MeOH = 10:1) to give the crude (340 mg, crude) as a yellow solid and used in the next step without further purification. LCMS [M+1]: 579.3.

Step B: tert-butyl (1R,5S)-3-(8-fluoro-2-(((2S,4R)-4-fluoro-1-methylpyrrolidin-2-yl)methoxy)-7-(3-(methoxymethoxy)naphthalen-1-yl)quinazolin-4-yl)-3,8-diazabicyclo[3.2.1]octane-8-carboxylate. A mixture of tert-butyl (1R,5S)-3-(2-chloro-8-fluoro-7-(3-(methoxymethoxy)naphthalen-1-yl)quinazolin-4-yl)-3,8-diazabicyclo[3.2.1]octane-8-carboxylate (170 mg, 294 µmol, 1.00 *eq),* [(2S,4R)-4-fluoro-1-methyl-pyrrolidin-2-yl]methanol (78.2 mg, 587 µmol, 2.00 *eq),* Pd(OAc)₂ (6.59 mg, 29.4 µmol, 0.10 *eq),* cesium carbonate (287 mg, 881 µmol, 3.00 *eq)* and BINAP (36.6 mg, 58.7 µmol, 0.20 *eq)* in toluene (10.0 mL) was degassed and purged with nitrogen for 3 times, and then stirred at 110 °C for 2 hours under nitrogen atmosphere. The reaction mixture was diluted with water 30.0 mL and extracted with ethyl acetate (50.0 mL × 3). The combined organic layers were washed with brine (20.0 mL × 2), dried, filtered and concentrated under reduced pressure to give a residue. The residue was purified by prep-TLC (SiO₂, Petroleum ether : Ethyl acetate = 0:1) to give the crude product (67.0 mg, crude) as a yellow oil and used in the next step without further purification. LCMS [M+1]: 676.4.

Step C: 4-(4-((1R,5S)-3,8-diazabicyclo[3.2.1]octan-3-yl)-8-fluoro-2-(((2S,4R)-4-fluoro-1-methylpyrrolidin-2-yl)methoxy)quinazolin-7-yl)naphthalen-2-ol. A mixture of tert-butyl 3-[8-fluoro-2-[[(2S,4R)-4-fluoro-1-methyl-pyrrolidin-2-yl]methoxy]-7-[3-(methoxymethoxy)-1-naphthyl]quinazolin-4-yl]-3,8-diazabicyclo[3.2.1]octane-8-carboxylate (57.0 mg, 84.4 µmol, 1.00 *eq)* in hydrochloric acid methanol (2 M, 4.00 mL) was degassed and purged with nitrogen for 3 times, and then the mixture was stirred at 20 °C for 1 hour under nitrogen atmosphere. The reaction mixture was concentrated under reduced pressure to give a residue. The residue was purified by prep-HPLC (HCl condition) to give the title compound (4.08 mg, 7.00 µmol, 8.30% yield, 97.5% purity, HCl) as a white solid. ¹H NMR (400 MHz, MeOD-*d*₄) δ = 8.13 (d, *J*=8.8 Hz, 1 H) 7.79 (d, *J*=8.4 Hz, 1 H) 7.64 (t, 7.6 Hz, 1 H)-7.43 - 7.51 (m, 2 H)-7.24 - 7.33 (m, 2 H) 7.16 (s, 1 H)-5.44 - 5.64 (m, 1 H)-4.95 - 5.24 (m, 4 H) 4.35 (br s, 3 H)-4.02 - 4.28 (m, 3 H)-3.57 - 3.80 (m, 1 H) 3.24 (s, 3 H)-2.69 - 2.85 (m, 1 H)-2.38 - 2.59 (m, 1 H) 2.20 (br s, 4 H). LCMS [M+1]: 532.4.

### EXAMPLE 30

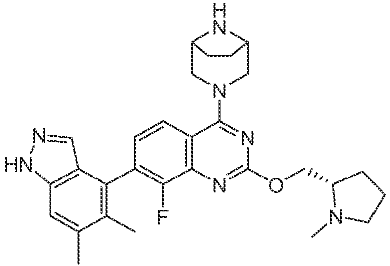

### 4-((1R,5S)-3,8-diazabicyclo[3.2.1]octan-3-yl)-7-(5,6-dimethyl-1H-indazol-4-yl)-8-fluoro-2-(((S)-1-methylpyrrolidin-2-yl)methoxy)quinazoline

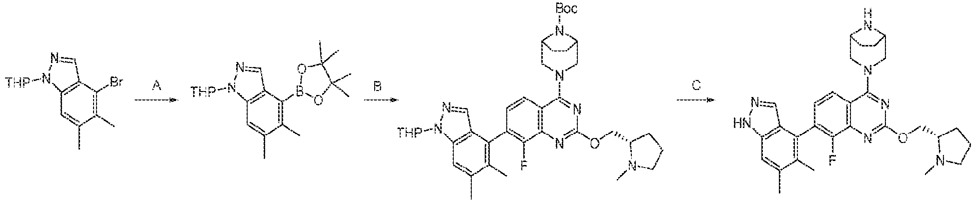

Step A: 5,6-dimethyl-1-(tetrahydro-2H-pyran-2-yl)-4-(4,4,5,5-tetramethyl-1,3,2-dioxaborolan-2-yl)-1H-indazole. A mixture of 4-bromo-5,6-dimethyl-1-tetrahydropyran-2-yl-indazole (200 mg, 647 µmol, 1.00 *eq),* 4,4,5,5-tetramethyl-2-(4,4,5,5-tetramethyl-1,3,2-dioxaborolan-2-yl)-1,3,2-dioxaborolane (246 mg, 970 µmol, 1.50 *eq),* potassium acetate (95.2 mg, 970 µmol, 1.50 *eq)* and Pd(dppf)Cl₂.CH₂Cl₂ (52.8 mg, 64.7 µmol, 0.10 *eq)* in dioxane (2.00 mL) was degassed and purged with nitrogen for 3 times, and then the mixture was stirred at 105 °C for 1 hour under nitrogen atmosphere. The reaction mixture was diluted with water (5.00 mL) and extracted with ethyl acetate (10.0 mL × 3). The combined organic layers were washed with brine (20.0 mL), dried, filtered and concentrated under reduced pressure to give a residue. The residue was purified by column chromatography (SiO₂, Petroleum ether : Ethyl acetate = 1:0 to 5:1) to give 5,6-dimethyl-1-tetrahydropyran-2-yl-4-(4,4,5,5-tetramethyl-1,3,2-dioxaborolan-2-yl)indazole (206 mg, 578 µmol, 89.4 % yield) as a yellow oil. ¹H NMR (400 MHz, CDCl₃) δ = 8.30 (s, 1H), 7.45 (s, 1H), 5.68 (dd, *J =* 2.8, 9.2 Hz, 1H),-4.04 - 3.96 (m, 1H),-3.77 - 3.69 (m, 1H), 2.55 (s, 3H), 2.41 (s, 3H),-2.21 - 2.12 (m, 1H),-2.08 - 2.00 (m, 1H),-1.83 - 1.72 (m, 2H),-1.68 - 1.51 (m, 2H), 1.42 (s, 12H).

Step B: tert-butyl (1R,5S)-3-(7-(5,6-dimethyl-1-(tetrahydro-2H-pyran-2-yl)-1H-indazol-4-yl)-8-fluoro-2-(((S)-1-methylpyrrolidin-2-yl)methoxy)quinazolin-4-yl)-3,8-diazabicyclo[3.2.1]octane-8-carboxylate. A mixture of tert-butyl (1R,5S)-3-(7-bromo-8-fluoro-2-(((S)-1-methylpyrrolidin-2-yl)methoxy)quinazolin-4-yl)-3,8-diazabicyclo[3.2.1]octane-8-carboxylate (50.0 mg, 90.8 µmol, 1.00 *eq),* 5,6-dimethyl-1-tetrahydropyran-2-yl-4-(4,4,5,5-tetramethyl-1,3,2-dioxaborolan-2-yl)indazole (64.7 mg, 182 µmol, 2.00 *eq),* Pd(dppf)Cl₂.CH₂Cl₂ (7.42 mg, 9.08 µmol, 0.10 *eq)* and cesium carbonate (59.2 mg, 182 µmol, 2.00 *eq)* in dioxane (1.00 mL) and water (0.20 mL) was degassed and purged with nitrogen for 3 times. The mixture was stirred at 105 °C for 2 hours under nitrogen atmosphere. The reaction mixture was concentrated under reduced pressure to give a residue. The residue was purified by prep-TLC (SiO₂, Dichloromethane : Methyl alcohol = 10:1) to give tert-butyl 3-[7-(5,6-dimethyl-1-tetrahydropyran-2-yl-indazol-4-yl)-8-fluoro-2-[[(2*S*)-1-methylpyrrolidin-2-yl]methoxy]quinazolin-4-yl]-3,8-diazabicyclo[3.2.1]octane-8-carboxylate (33.0 mg, 35.7 µmol, 39.3 % yield, 75.7 % purity) as a brown solid. LCMS [M+1] = 701.2.

Step C: 4-((1R,5S)-3,8-diazabicyclo[3.2.1]octan-3-yl)-7-(5,6-dimethyl-1H-indazol-4-yl)-8-fluoro-2-(((S)-1-methylpyrrolidin-2-yl)methoxy)quinazoline. A solution of tert-butyl (1R,5S)-3-(7-(5,6-dimethyl-1-(tetrahydro-2H-pyran-2-yl)-1H-indazol-4-yl)-8-fluoro-2-(((S)-1-methylpyrrolidin-2-yl)methoxy)quinazolin-4-yl)-3,8-diazabicyclo[3.2.1]octane-8-carboxylate (30.0 mg, 32.5 µmol, 1.00 *eq)* in HCl/MeOH (2.00 M, 3.00 mL, 185 *eq)* was stirred at 15 °C for 12 hours. The reaction mixture was concentrated under reduced pressure to give a residue. The residue was purified by prep-HPLC (TFA condition) to give 4-(3,8-diazabicyclo[3.2.1]octan-3-yl)-7-(5,6-dimethyl-1*H*-indazol-4-yl)-8-fluoro-2-[[(2*S*)-1-methylpyrrolidin-2-yl]methoxy]quinazoline (14.9 mg, 23.4 µmol, 72.0 % yield, 99.0 % purity, TFA) as an Off-white solid. ¹H NMR (400 MHz, MeOD) δ = 7.99 (br d, *J =* 8.8 Hz, 1H), 7.54 (br s, 1H), 7.50 (s, 1H),-7.47 - 7.41 (m, 1H), 4.77 (br d, *J* = 10.8 Hz, 1H), 4.29 (br s, 2H), 3.94 (br d, *J =* 13.2 Hz, 3H), 3.77 (br s, 1H), 3.28 (br s, 2H), 3.13 (br s, 3H), 2.52 (s, 3H), 2.44 (br dd, *J* = 8.8, 15.2 Hz, 2H), 2.22 (br s, 4H), 2.20 (br s, 3H),-2.17 - 2.04 (m, 4H). LCMS [M+1] = 516.5

### EXAMPLE 31

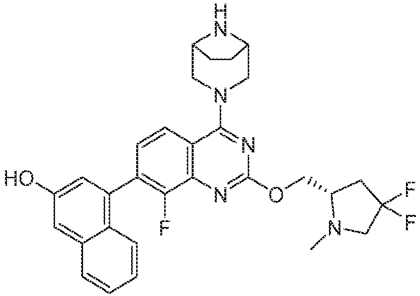

### 4-(4-((1R,5S)-3,8-diazabicyclo[3.2.1]octan-3-yl)-2-(((S)-4,4-difluoro-1-methylpyrrolidin-2-yl)methoxy)-8-fluoroquinazolin-7-yl)naphthalen-2-ol

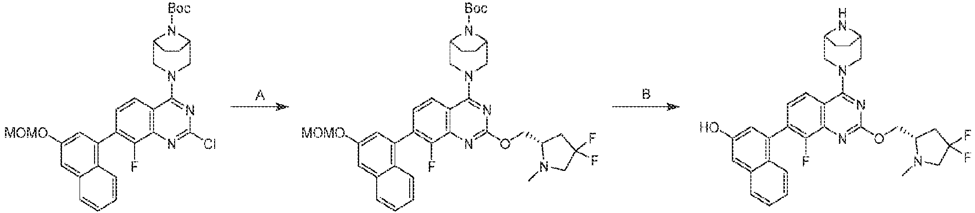

Step A: tert-butyl (1R,5S)-3-(2-(((S)-4,4-difluoro-1-methylpyrrolidin-2-yl)methoxy)-8-fluoro-7-(3-(methoxymethoxy)naphthalen-1-yl)quinazolin-4-yl)-3,8-diazabicyclo[3.2.1]octane-8-carboxylate. A mixture of tert-butyl 3-[2-chloro-8-fluoro-7-[3-(methoxymethoxy)-1-naphthyl]quinazolin-4-yl]-3,8-diazabicyclo[3.2.1]octane-8-carboxylate (170 mg, 294 µmol, 1.00 *eq),* [(2*S*)-4,4-difluoro-1-methyl-pyrrolidin-2-yl]methanol (88.8 mg, 587 µmol, 2.00 *eq),* Pd(OAc)₂ (6.59 mg, 29.4 µmol, 0.10 *eq),* cesium carbonate (287 mg, 881 µmol, 3.00 *eq)* and BINAP (36.6 mg, 58.7 µmol, 0.20 *eq)* in toluene (10.0 mL) was degassed and purged with nitrogen for 3 times, and then the mixture was stirred at 110 °C for 2 hours under nitrogen atmosphere. The reaction mixture was diluted with water 30.0 mL and extracted with ethyl acetate (40.0 mL × 3). The combined organic layers were washed with brine (20.0 mL × 2), dried, filtered and concentrated under reduced pressure to give a residue. The residue was purified by prep-TLC (SiO₂, Petroleum ether : Ethyl acetate = 0:1) to give the crude product tert-butyl 3-[2-[[(2S)-4,4-difluoro-1-methyl-pyrrolidin-2-yl]methoxy]-8-fluoro-7-[3-(methoxymethoxy)-1-naphthyl]quinazolin-4-yl]-3,8-diazabicyclo[3.2.1]octane-8-carboxylate (70 mg, crude) as a brown oil and used into the next step without further purification. LCMS [M+1]: 694.3

Step B: 4-(4-((1R,5S)-3,8-diazabicyclo[3.2.1]octan-3-y1)-2-(((S)-4,4-difluoro-1-methylpyrrolidin-2-yl)methoxy)-8-fluoroquinazolin-7-yl)naphthalen-2-ol. A mixture of tert-butyl 3-[2-[[(2*S*)-4,4-difluoro-1-methyl-pyrrolidin-2-yl]methoxy]-8-fluoro-7-[3-(methoxymethoxy)-1-naphthyl]quinazolin-4-yl]-3,8-diazabicyclo[3.2.1]octane-8-carboxylate (65.0 mg, 93.7 µmol, 1.00 *eq)* in hydrochloric acid methanol (2 M, 4.00 mL) was degassed and purged with nitrogen for 3 times, and then the mixture was stirred at 20 °C for 1 hour under nitrogen atmosphere. The reaction mixture was concentrated under reduced pressure to give a residue. The residue was purified by prep-HPLC (HCl condition) to give 4-[4-(3,8-diazabicyclo[3.2.1]octan-3-yl)-2-[[(2S)-4,4-difluoro-1-methyl-pyrrolidin-2-yl]methoxy]-8-fluoro-quinazolin-7-yl]naphthalen-2-ol (11.9 mg, 19.6 µmol, 20.9% yield, 96.3% purity, HCl) as a yellow solid. ¹H NMR (400 MHz, MeOD-*d*₄) δ = 8.15 (d, *J*=8.8 Hz, 1 H) 7.79 (d, *J*=8.4 Hz, 1 H) 7.65 (t, *J*=7.2 Hz, 1 H)-7.43 - 7.52 (m, 2 H)-7.23 - 7.33 (m, 2 H) 7.16 (s, 1 H)-4.97 - 5.28 (m, 3 H) 4.48-4.60 (m, 1 H) 4.30-4.41 (m, 3 H)-4.13 - 4.30 (m, 3 H) 3.85-4.00 (m, 1 H) 3.25 (s, 3 H)-3.02 - 3.16 (m, 1 H)-2.72 - 2.96 (m, 1 H) 2.21 (br s, 4 H). LCMS [M+1]: 550.4

### EXAMPLE A

### KRas G12D Surface Plasmon Resonance (SPR) Binding Assay

This Example illustrates that exemplary compounds of the present invention bind to KRas G12D as measured by surface plasmon resonance (SPR).

Briefly, 1L of 1.05X HBS-Mg buffer (262.5mM BioUltra Hepes, pH 7.5, 157.5mM NaCl, 105mM MgCl₂, 0.525mM TCEP, 0.0305% Brij-35) was prepared and filter sterilized using a 0.22µm bottle top filter. Approximately 50mL of 1.05X HBS-Mg buffer was removed and saved for future dilutions. A 50mL aliquot of DMSO (Sigma Aldrich DMSO Lot. #SHBK2079) was added and continued to stir for 10 minutes, creating the final 1.0X HBS-Mg buffer (250mM BioUltra Hepes pH 7.5, 150mM NaCl, 100mM MgCl₂, 0.5mM TCEP, 0.03% Brij-35).

Biacore T200 instrument was primed using 1.0X HBS-Mg buffer before docking a GE Streptavidin (SA) chip and then primed two additional times prior to beginning the immobilization step. All immobilized protein mixtures were created using 3-5mg/mL Biotinylated Avidin-tagged KRAS protein using the following immobilization settings: SA chip type, 1 flow cells per cycle, 720 second contact time, and 5µL/min flow rate. Normalization of the detector was also performed during the immobilization step using the GE BiaNormalize solution.

All compounds were diluted to 10mM in 100% DMSO prior to being diluted 20X in 1.05X buffer. Another 10X dilution was created using 1.0X buffer prior to performing a series of 3X dilutions to create a compound concentration curve using the following assay settings: 20C analysis temperature, General Settings=10Hz data collection rate and multi-detection; Assay Steps=all set to LMW kinetics; Cycle Types=LMW kinetics (60s contact time, 120s dissociation time, 100µL/min flow rate, extra wash after injection with 50% DMSO, flow path 1,2,3,4); Flow path detection=2-1, 4-3). Data evaluation was performed using the Biacore T200 Evaluation software and data fit to 1:1 binding model.

The results for exemplary compounds of Formula (I) are shown in Table 1.

**Table 1**

| Determination of KRas G12D K_{D} for Exemplary Compounds of Formula (I) | | | |
|---|---|---|---|
| **Example No.** | **K_{D} (nM)** | **Example No.** | **K_{D} (nM)** |
| 2 | 8 | 13 | 78 |
| 3 | > 1000000 | 14 | 36 |
| 7 | 9 | 15 | 93 |
| 8 | 3 | 16 | 300 |
| 10 | 19 | 29 | 5 |
| 11 | 16 | 30 | 152 |
| 12 | 110 | 31 | 121 |

### EXAMPLE B

### KRas G12D Binding Assay

This Example illustrates that exemplary compounds of the present invention bind to KRas G12D and are capable of displacing a labeled tracer ligand occupying the KRas G12D binding site.

The ability of a compound to bind to KRAS G12D was measured using a TR-FRET displacement assay. Biotinylated GDP-loaded recombinant human KRAS G12D (corresponding to amino acids 1:169, produced at Array BioPharma) was incubated with a custom-made Cy5 labelled tracer, europium labelled streptavidin and compound (2% DMSO final) in buffer (50mM HEPES [pH 7.5], 5mM MgCl₂, 0.005% Tween-20 & 1 mM DTT). After a 60 minute incubation at 22°C, the reaction was measured using a PerkinElmer EnVision multimode plate reader via TR-FRET dual wavelength detection, and the percent of control (POC) calculated using a ratiometric emission factor. 100 POC is determined using no test compound and 0 POC is determined using a concentration of control compound that completely inhibits binding of the tracer to KRAS. The POC values were fit to a 4-parameter logistic curve and the IC₅₀ value was determined as the concentration where the curve crosses 50 POC.

The results for exemplary compounds of Formula (I) are shown in Table 2.

**Table 2**

| Binding to KRas G12D by Exemplary Compounds of Formula (I) | | | |
|---|---|---|---|
| **Example No.** | **IC₅₀ (nM)** | **Example No.** | **IC₅₀ (nM)** |
| 1 | 7.0 | 18 | 11.5 |
| 2 | 6.1 | 19 | 1.3 |
| 3 | 0.5 | 20 | 1.6 |
| 4 | 0.7 | 21 | 1.0 |
| 5 | 1.4 | 22 | 1.7 |
| 6 | 3.5 | 23 | 6.6 |
| 7 | 11.2 | 24 | 1.4 |
| 8 | 4.3 | 25 | 5.3 |
| 9 | 186.7 | 26 | 7.4 |
| 10 | 22.9 | 27 | 3.6 |
| 11 | 10.4 | 28 | 0.6 |
| 13 | 61.9 | 29 | 1.8 |
| 14 | 30.8 | 30 | 57.0 |
| 16 | 152.5 | 31 | 296.7 |
| 17 | 8.0 | | |

### EXAMPLE C

### Inhibition of KRas G12D-mediated Phosphorylation of ERK by Exemplary Compounds of Formula (I)

This Example illustrates that exemplary compounds of the present invention inhibit the phosphorylation of ERK downstream of KRAS G12D.

AGS cells (ATCC CRL-1739) expressing G12D were grown in DMEM medium supplemented with 10% fetal bovine serum, 10mM HEPES, and Penicillin/Streptomycin. Cells were plated in tissue culture treated 96 well plates at a density of 40,000 cells/well and allowed to attach for 12-14 hours. Diluted compounds were then added in a final concentration of 0.5% DMSO. After 3 hours, the medium was removed, 150 µl of 4.0% formaldehyde was added and the plates incubated at room temperature for 20 minutes. The plates were washed with PBS, and permeabilized with 150µL of ice cold 100% methanol for 10 minutes. Non-specific antibody binding to the plates was blocked using 100 µL Licor blocking buffer (Li-Cor Biotechnology, Lincoln NE) for 1 hour at room temperature.

The amount of phosho-ERK was determined using an antibody specific for the phosphorylated form of ERK and compared to the amount of GAPDH. Primary antibodies used for the detection were added as follows: Phospho-ERK(Cell Signaling cs-9101) diluted 1:500 and GAPDH(Millipore MAB374) diluted 1:5000 in Licor block + 0.05%Tween 20. The plates were incubated for 2 hours at room temperature. The plates were washed with PBS + 0.05% Tween 20.

Secondary antibodies used to visualize primary antibodies were added as follows: Anti-rabbit-680 diluted 1:1000 and Anti-mouse-800 diluted 1:1000 both in Licor block +0.05% Tween20, and were incubated for 1 hour at room temperature. The plates were washed with PBS +0.05% Tween 20. A 100µl aliquot of PBS was added to each well and the plates were read on a Li-Cor Odyssey CLX plate reader phospho-ERK(Thr202/Tyr204) signal was normalized to the GAPDH signal for each well and percent of DMSO control values were calculated. IC50 values were generated using a 4-parameter fit of the dose response curve

The results for exemplary compounds of Formula (I) are shown in Table 3.

**Table 3**

| Inhibition of KRas G12D-mediated Phosphorylation of ERK by Exemplary Compounds of Formula (I) | | | |
|---|---|---|---|
| **Example No.** | **IC₅₀ (nM)** | **Example No.** | **IC₅₀ (nM)** |
| 1 | 8391 | 17 | 5556 |
| 2 | 4545 | 18 | 1371 |
| 3 | 95 | 19 | 1705 |
| 4 | 21 | 20 | 2156 |
| 5 | 42 | 21 | 1269 |
| 6 | 1800 | 22 | 140 |
| 7 | 1417 | 23 | 264 |
| 8 | 271 | 24 | 377 |
| 9 | 6786 | 25 | 784 |
| 10 | 5662 | 26 | 3541 |
| 11 | 2844 | 27 | 44 |
| 12 | 5556 | 28 | 171 |
| 13 | 5556 | 29 | 779 |
| 14 | 8607 | 30 | 5093 |
| 15 | 7121 | 31 | 8501 |
| 16 | 5948 | | |

## Claims

1. A compound of Formula (I): 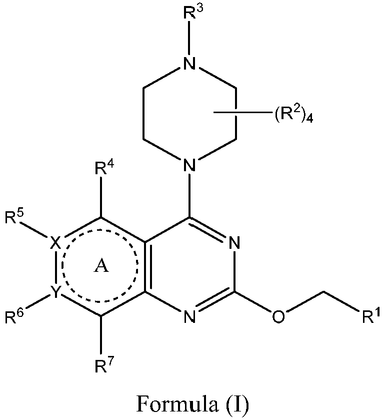 or a pharmaceutically acceptable salt thereof, wherein:
A is phenyl, heteroaryl or heterocyclyl;
X is N, C or CH;
Y is N, C or CH;
where if X is N, then Y is not N, and where if Y is N, then X is not N; 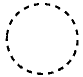
represents one or more optionally-present double bonds;
R¹ is heterocyclyl, optionally substituted with one or more substituents independently selected from: hydroxy, halogen, C1-C3 haloalkyl, C1-C3 alkoxy and cyano;
two R² are each independently hydrogen, hydroxy, halogen, C1-C3 haloalkyl, C1-C3 alkyl, C1-C3 alkoxy, (C1-C3 alkoxy)-C1-C3 alkyl, C1-C3 alkyl-N(R⁸)₂, cyano, C1-C3 cyanoalkyl, C2 - C4 cyanoalkenyl, C1 - C3 hydroxyalkyl, HC(=O)-, -CO₂R⁸, or - CO²N(R⁸)₂, where R⁸ is hydrogen or C1-C3 alkyl, and
two R² are hydrogen, two R² on the same carbon atom join to form a cycloalkyl ring, or two R² join to form an ethylene bridge to form a [3.2.1] or [2.2.2] ring system, where said ethylene bridge forms a [2.2.2] ring system if X is N and ring A is heteroaryl, and where said ethylene bridge forms a [2.2.2] ring system if Y is N and ring A is heterocyclyl;
R³ is hydrogen or C1-C3 alkyl optionally substituted with one or more substituents independently selected from: halogen, C1-C3 alkoxy and cyano;
R⁴ is hydrogen;
R⁵ is absent or is selected from hydrogen, halogen, -O-phenyl and -O-pyridyl, where said phenyl and said pyridyl are optionally substituted with one or more substituents independently selected from: hydroxy, halogen, C1-C3 haloalkyl, C1-C3 alkyl, C1-C3 alkoxy and cyano;
R⁶ is phenyl, naphthyl or indazolyl, optionally substituted with one or more substituents independently selected from: hydroxy, halogen, C1-C3 haloalkyl, C1-C3 alkyl, C1-C3 alkoxy and cyano; and
R⁷ is hydrogen or halogen.

2. The compound or salt of claim 1, wherein Formula (I) is represented by Formula (I-A): 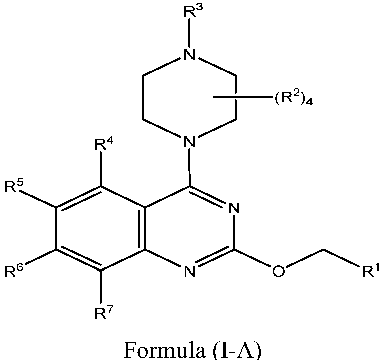
where R⁵ must be present, or wherein Formula (I) is represented by Formula (I-B): 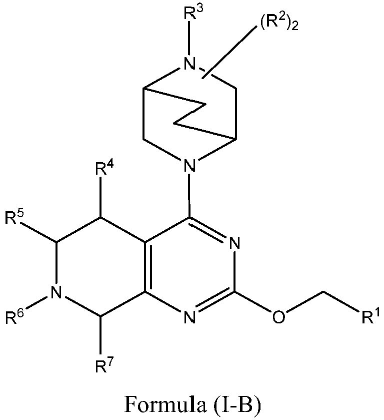
where R⁵ must be present.

3. The compound or salt of claim 1, wherein R¹ is hexahydro-1H-pyrrolizinyl optionally substituted with one or more substituents independently selected from: hydroxy, halogen, C1-C3 haloalkyl, C1-C3 alkoxy and cyano optionally wherein said hexahydro-1H-pyrrolizinyl is optionally substituted with a halogen, optionally wherein R¹ is 2-fluorohexahydro-1H-pyrrolizinyl.

4. The compound or salt of claim 1, wherein R¹ heterocyclyl is pyrrolidine.

5. The compound or salt of claim 1, wherein two R² are hydrogen, and two R² join to form an ethylene bridge to form a [3.2.1] ring system.

6. The compound or salt of claim 1, wherein two R² are hydrogen, and two R² join to form an ethylene bridge to form a [2.2.2] ring system.

7. The compound or salt of claim 1, wherein two R² are hydrogen and two R² join to form an alkylene bridge to form a [3.2.1] or [2.2.2] ring system.

8. The compound or salt of claim 6, wherein X is N and A is heteroaryl.

9. The compound or salt of claim 6, wherein Y is N and ring A is heterocyclyl.

10. The compound or salt of claim 7, wherein X and Y are both C or CH.

11. The compound or salt of claim 1, wherein R³ is hydrogen or a halogen-substituted C1-C3 alkyl.

12. The compound or salt of claim 1, wherein R⁵ is absent; halogen; -O-phenyl where phenyl is optionally substituted with one or more substituents independently selected from: halogen, C1-C3 alkoxy and cyano; or -O-pyridyl.

13. The compound or salt of claim 1, wherein R⁶ is phenyl optionally substituted with one or more substituents independently selected from: hydroxy and halogen; naphthyl optionally substituted with halogen, hydroxy, or both halogen and hydroxy; or indazolyl optionally substituted with one or more C1-C3 alkyl.

14. The compound or salt of claim 1, wherein R⁷ is hydrogen or halogen.

15. A compound according to claim 1 selected from: 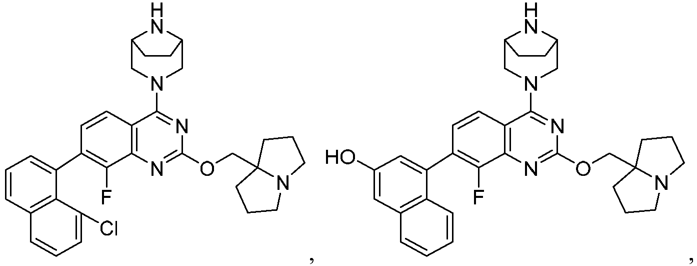 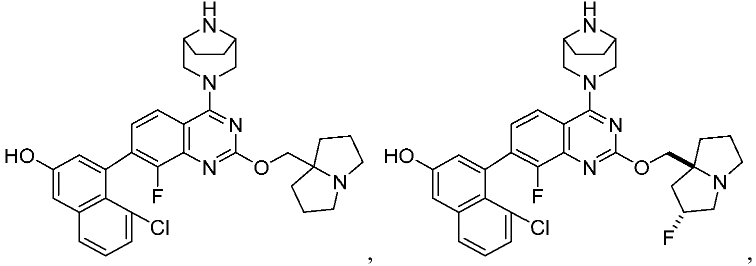 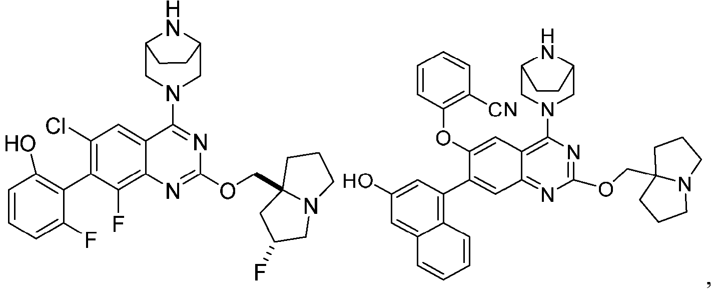 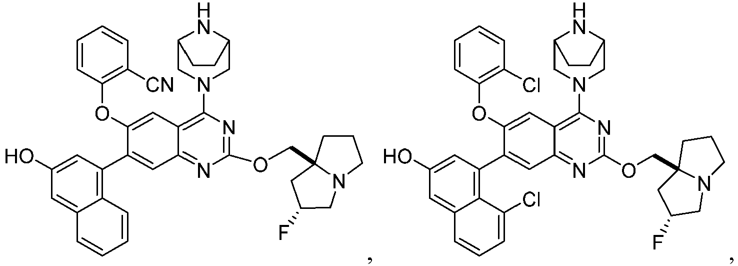 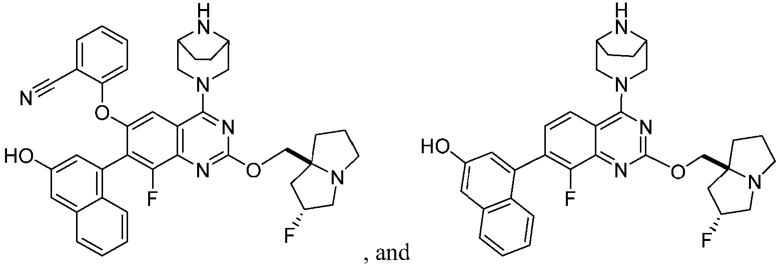 or a pharmaceutically acceptable salt thereof.

16. A compound selected from 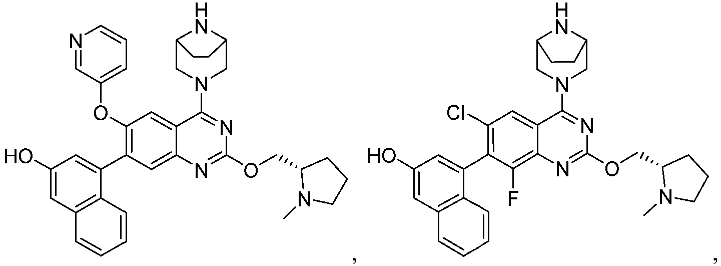 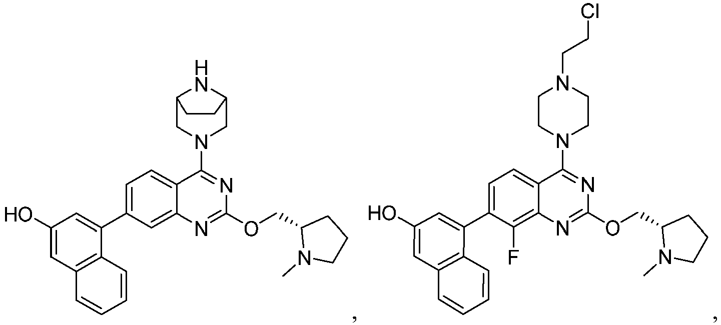 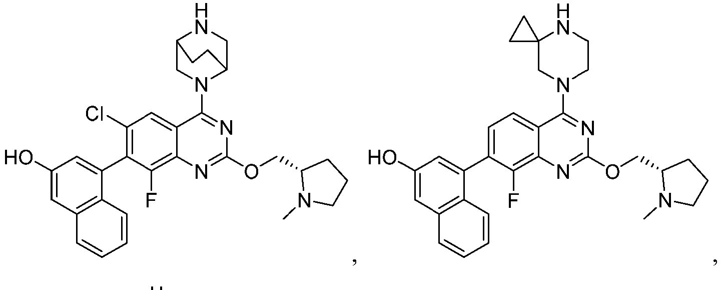 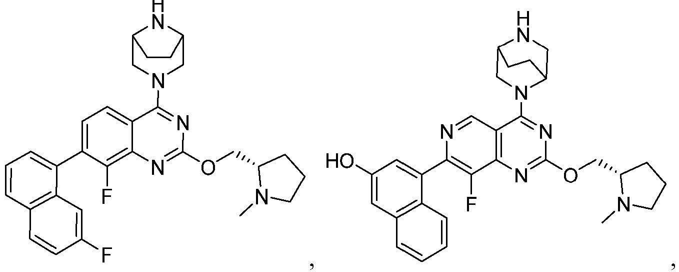 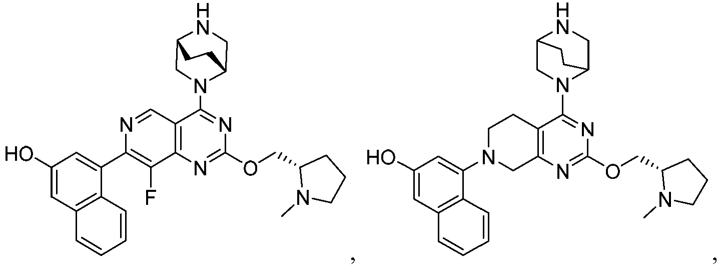 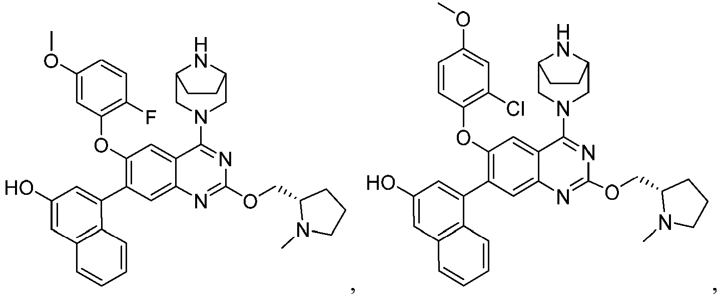 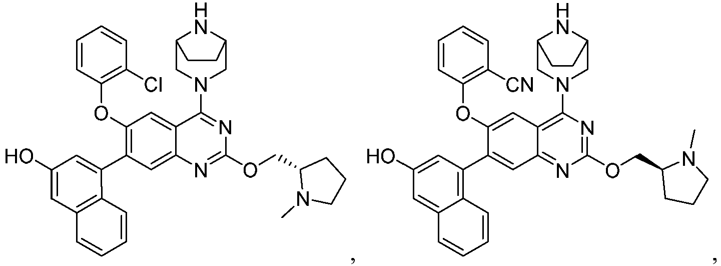 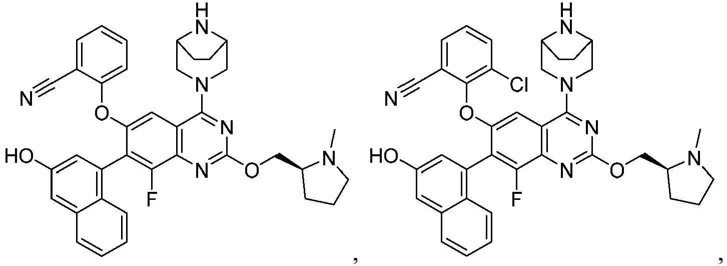 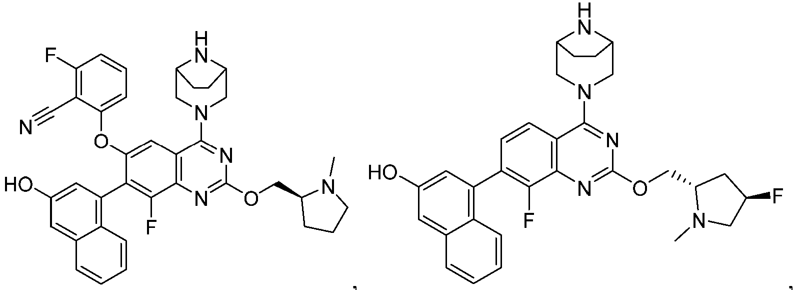 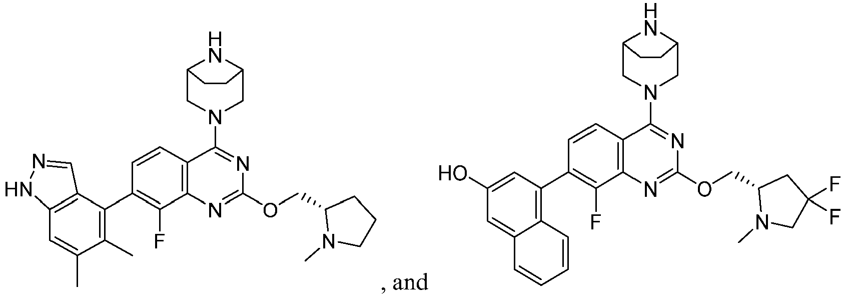 or a pharmaceutically acceptable salt thereof.

17. A pharmaceutical composition, comprising a therapeutically effective amount of a compound of any one of claims 1-16 or a pharmaceutically acceptable salt thereof, and a pharmaceutically acceptable excipient.

18. An in vitro method for inhibiting KRas G12D activity in a cell, comprising contacting the cell in which inhibition of KRas G12D activity is desired with an effective amount of a compound of according to any one of claims 1-16 or a pharmaceutically acceptable salt thereof, or the pharmaceutical composition of claim 17.

19. A compound according to any one of claims 1-16 or a pharmaceutically acceptable salt thereof, or the pharmaceutical composition of claim 17 for use in a method for treating cancer comprising administering to a patient having cancer a therapeutically effective amount of the compound or a pharmaceutically acceptable salt thereof, or the pharmaceutical composition.

20. The compound, pharmaceutically acceptable salt thereof, or pharmaceutical composition for use according to claim 19, wherein the cancer is selected from the group consisting of Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Biliary tract: gall bladder carcinoma, ampullary carcinoma, cholangiocarcinoma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial 'carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); Hematologic: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); Skin: malignant melanoma, basal cell carcinoma, squamous cell carcinoma, Kaposi's sarcoma, moles dysplastic nevi, lipoma, angioma, dermatofibroma, keloids, psoriasis; and Adrenal glands: neuroblastoma.

21. The compound, pharmaceutically acceptable salt thereof, or pharmaceutical composition for use according to claim 19, wherein the cancer is a KRas G12D-associated cancer.

22. The compound, pharmaceutically acceptable salt thereof, or pharmaceutical composition for use according to claim 19, wherein the cancer is non-small cell lung cancer, small cell lung cancer, colorectal cancer, rectal cancer or pancreatic cancer.

23. The compound, pharmaceutically acceptable salt thereof, or pharmaceutical composition for use according to claim 19, wherein the therapeutically effective amount of the compound is between about 0.01 to 100 mg/kg per day, optionally wherein the therapeutically effective amount of the compound is between about 0.1 to 50 mg/kg per day.

24. A compound according to any one of claims 1-16 or a pharmaceutically acceptable salt thereof, or the pharmaceutical composition of claim 17 for use in a method for treating cancer in a patient in need thereof, the method comprising (a) determining that the cancer is associated with a KRas G12D mutation (e.g., a KRas G12D-associated cancer); and (b) administering to the patient a therapeutically effective amount of the compound or the pharmaceutically acceptable salt thereof, or the pharmaceutical composition.

## Patentansprüche

1. Verbindung der Formel (I): 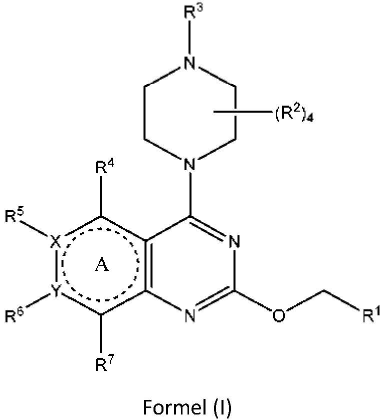 oder ein pharmazeutisch verträgliches Salz davon, wobei:
A Phenyl, Heteroaryl oder Heterocyclyl ist;
X N, C oder CH ist;
Y N, C oder CH ist;
wobei, wenn X N ist, dann Y nicht N ist, und wenn Y N ist, dann X nicht N ist; 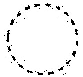
eine oder mehrere optional vorhandene Doppelbindungen darstellt;
R¹ Heterocyclyl ist, das optional mit einem oder mehreren Substituenten substituiert ist, unabhängig ausgewählt aus: Hydroxy, Halogen, C1-C3-Halogenalkyl, C1-C3-Alkoxy und Cyano;
zwei R² jeweils unabhängig Wasserstoff, Hydroxy, Halogen, C1-C3-Halogenalkyl, C1-C3-Alkyl, C1-C3-Alkoxy, (C1-C3-Alkoxy)-C1-C3-alkyl, C1-C3-Alkyl-N(R⁸)₂, Cyano, C1-C3-Cyanoalkyl, C2-C4-Cyanoalkenyl, C1-C3-Hydroxyalkyl, HC(=O)-, -CO₂R⁸ oder -CO²N(R⁸)₂ sind, wobei R⁸ Wasserstoff oder C1-C3-Alkyl ist, und
zwei R² Wasserstoff sind, sich zwei R² an dem gleichen Kohlenstoffatom verbinden, um einen Cycloalkylring zu bilden, oder sich zwei R² verbinden, um eine Ethylenbrücke zu bilden, um ein [3.2.1]- oder [2.2.2]-Ringsystem zu bilden, wobei die Ethylenbrücke ein [2.2.2]-Ringsystem bildet, wenn X N ist und Ring A Heteroaryl ist, und wobei die Ethylenbrücke ein [2.2.2]-Ringsystem bildet, wenn Y N ist und Ring A Heterocyclyl ist;
R³ Wasserstoff oder C1-C3-Alkyl ist, optional substituiert mit einem oder mehreren Substituenten, unabhängig ausgewählt aus: Halogen, C1-C3-Alkoxy und Cyano;
R⁴ Wasserstoff ist;
R⁵ fehlt oder aus Wasserstoff, Halogen, -O-Phenyl und -O-Pyridyl ausgewählt ist, wobei das Phenyl und das Pyridyl optional mit einem oder mehreren Substituenten substituiert sind, die unabhängig ausgewählt sind aus: Hydroxy, Halogen, C1-C3-Halogenalkyl, C1-C3-Alkyl, C1-C3-Alkoxy und Cyano;
R⁶ Phenyl, Naphthyl oder Indazolyl ist, optional substituiert mit einem oder mehreren Substituenten, unabhängig ausgewählt aus: Hydroxy, Halogen, C1-C3-Halogenalkyl, C1-C3-Alkyl, C1-C3-Alkoxy und Cyano; und
R⁷ Wasserstoff oder Halogen ist.

2. Verbindung nach Anspruch 1, wobei Verbindung (I) durch Formel (I-A) dargestellt wird: 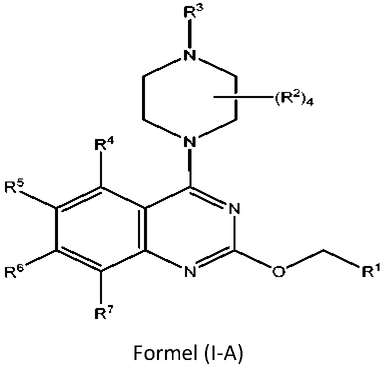
wobei R⁵ vorhanden sein muss oder wobei Formel (I) durch Formel (I-B) dargestellt wird: 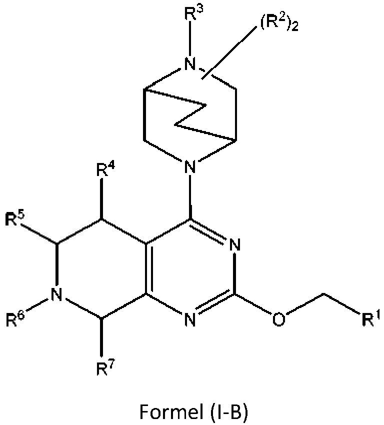
wobei R⁵ vorhanden sein muss.

3. Verbindung oder Salz nach Anspruch 1, wobei R¹ Hexahydro-1H-pyrrolizinyl ist, optional substituiert mit einem oder mehreren Substituenten, unabhängig ausgewählt aus: Hydroxy, Halogen, C1-C3-Halogenalkyl, C1-C3-Alkoxy und Cyano, optional wobei das Hexahydro-1H-pyrrolizinyl optional mit einem Halogen substituiert ist, wobei R¹ optional 2-Fluorhexahydro-1H-pyrrolizinyl ist.

4. Verbindung oder Salz nach Anspruch 1, wobei das R¹-Heterocyclyl Pyrrolidin ist.

5. Verbindung oder Salz nach Anspruch 1, wobei zwei R² Wasserstoff sind und sich zwei R² verbinden, um eine Ethylenbrücke zu bilden, um ein [3.2.1]-Ringsystem zu bilden.

6. Verbindung oder Salz nach Anspruch 1, wobei zwei R² Wasserstoff sind und sich zwei R² verbinden, um eine Ethylenbrücke zu bilden, um ein [2.2.2]-Ringsystem zu bilden.

7. Verbindung oder Salz nach Anspruch 1, wobei zwei R² Wasserstoff sind und sich zwei R² verbinden, um eine Alkylenbrücke zu bilden, um ein [3.2.1]- oder [2.2.2]-Ringsystem zu bilden.

8. Verbindung oder Salz nach Anspruch 6, wobei X N ist und A Heteroaryl ist.

9. Verbindung oder Salz nach Anspruch 6, wobei Y N ist und Ring A Heterocyclyl ist.

10. Verbindung oder Salz nach Anspruch 7, wobei X und Y beide C oder CH sind.

11. Verbindung oder Salz nach Anspruch 1, wobei R³ Wasserstoff oder ein halogensubstituiertes C1-C3-Alkyl ist.

12. Verbindung oder Salz nach Anspruch 1, wobei R⁵ fehlt; Halogen; -O-Phenyl, wobei das Phenyl optional mit einem oder mehreren Substituenten substituiert ist, unabhängig ausgewählt aus: Halogen, C1-C3-Alkoxy und Cyano; oder -O-Pyridyl.

13. Verbindung oder Salz nach Anspruch 1, wobei R⁶ Phenyl ist, optional substituiert mit einem oder mehreren Substituenten, unabhängig ausgewählt aus: Hydroxy und Halogen; Naphthyl, optional substituiert mit Halogen, Hydroxy oder sowohl Halogen als auch Hydroxy; oder Indazolyl, optional substituiert mit einem oder mehreren C1-C3-Alkyl.

14. Verbindung oder Salz nach Anspruch 1, wobei R⁷ Wasserstoff oder Halogen ist.

15. Verbindung nach Anspruch 1, ausgewählt aus: 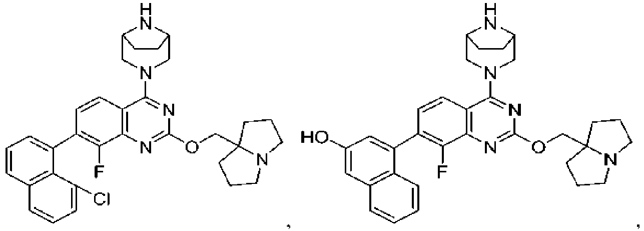 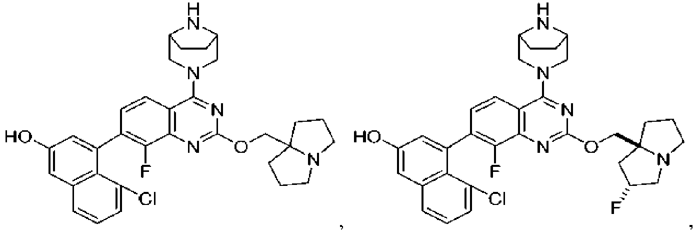 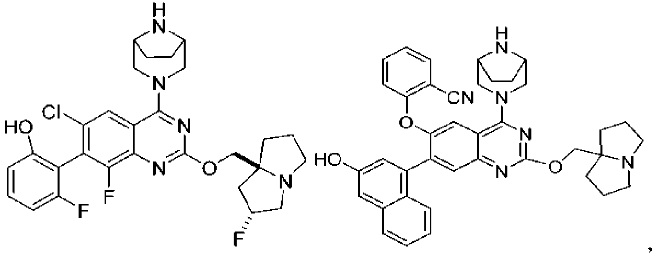 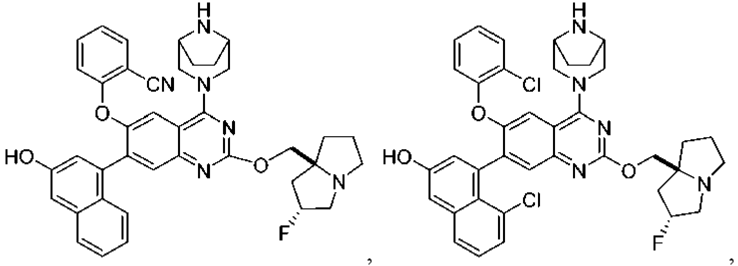 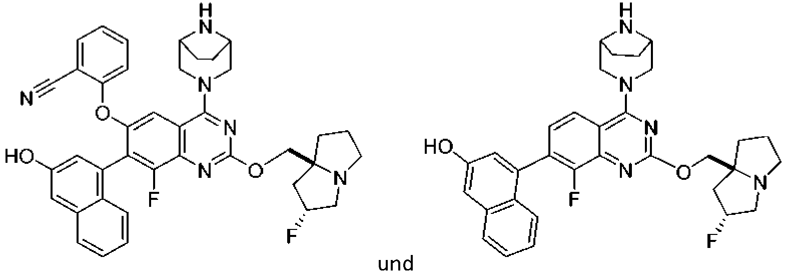 oder ein pharmazeutisch verträgliches Salz davon.

16. Verbindung, ausgewählt aus 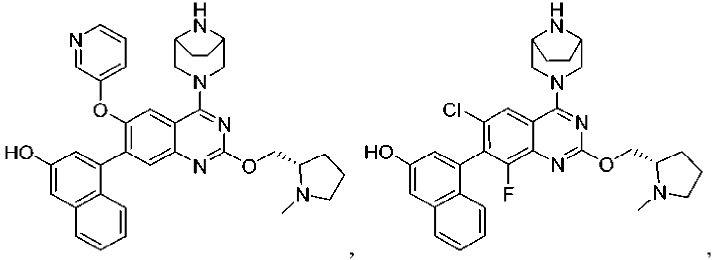 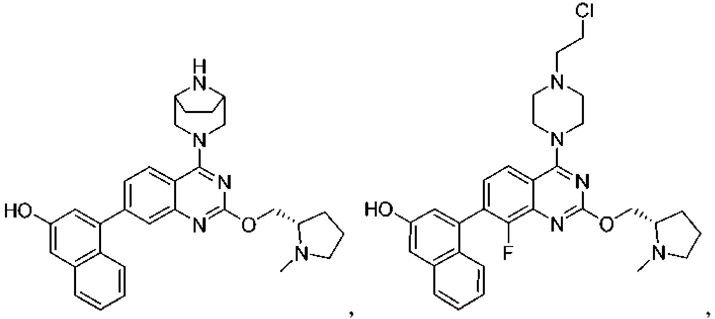 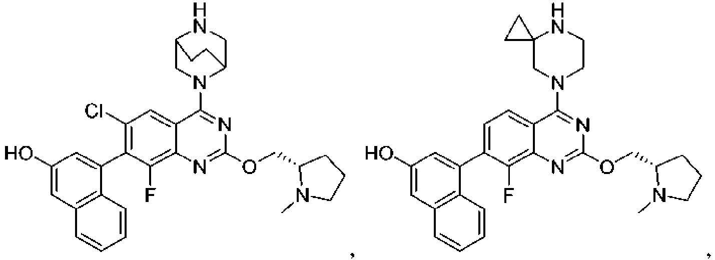 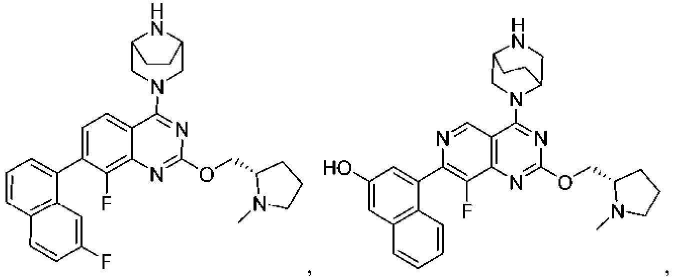 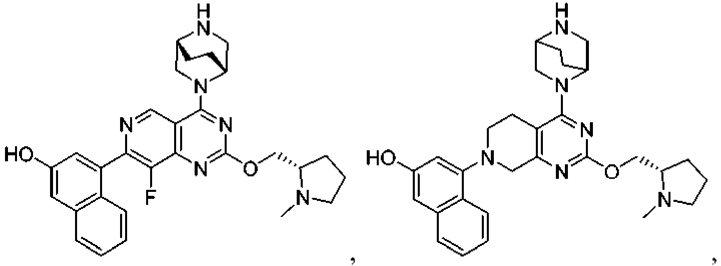 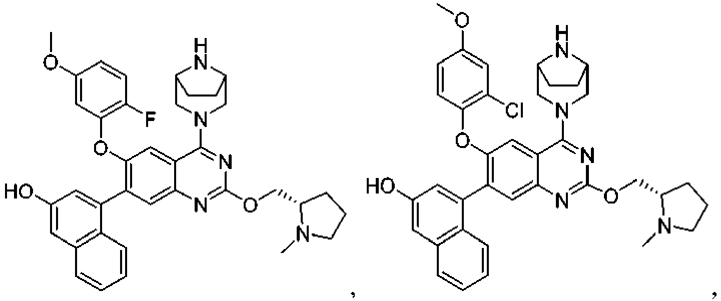 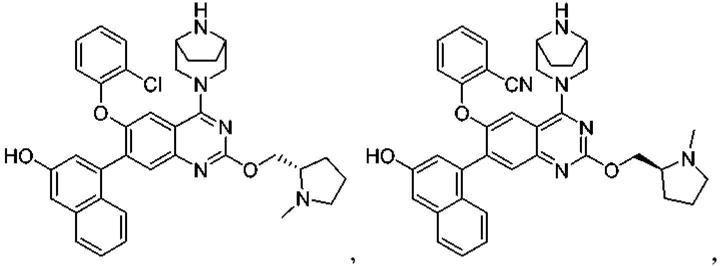 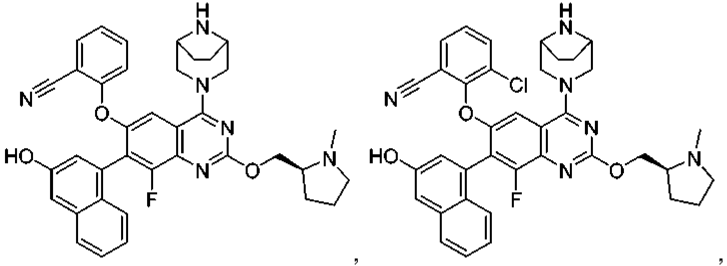 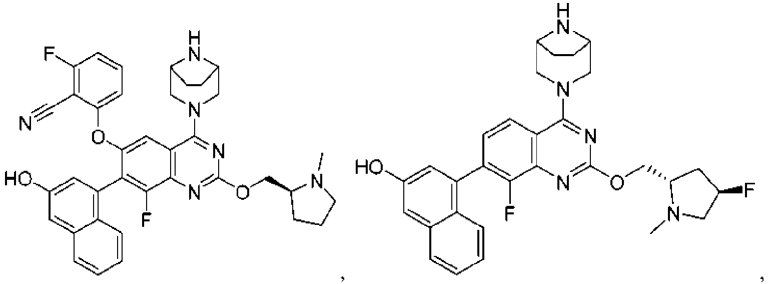 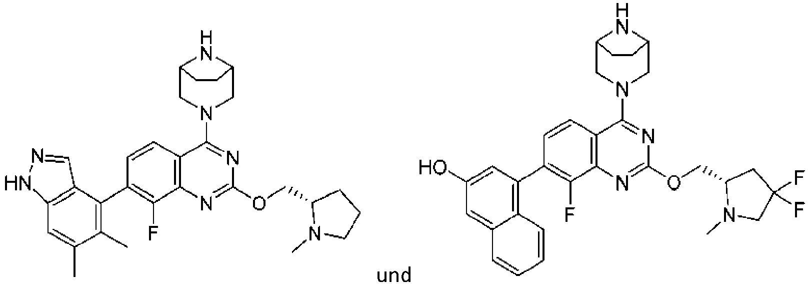 oder ein pharmazeutisch verträgliches Salz davon.

17. Pharmazeutische Zusammensetzung, umfassend eine therapeutisch wirksame Menge einer Verbindung nach einem der Ansprüche 1-16 oder ein pharmazeutisch verträgliches Salz davon und einen pharmazeutisch verträglichen Hilfsstoff.

18. In-vitro-Verfahren zum Hemmen von KRas-G12D-Aktivität in einer Zelle, umfassend In-Kontakt-Bringen der Zelle, in der eine Hemmung der KRas-G12D-Aktivität erwünscht ist, mit einer wirksamen Menge einer Verbindung nach einem der Ansprüche 1-16 oder einem pharmazeutisch verträglichen Salz davon oder der pharmazeutischen Zusammensetzung nach Anspruch 17.

19. Verbindung nach einem der Ansprüche 1-16 oder ein pharmazeutisch verträgliches Salz davon oder die pharmazeutische Zusammensetzung nach Anspruch 17 zur Verwendung in einem Verfahren zum Behandeln von Krebs, umfassend Verabreichen einer therapeutisch wirksamen Menge der Verbindung oder eines pharmazeutisch verträglichen Salzes davon oder der pharmazeutischen Zusammensetzung an einen Patienten mit Krebs.

20. Verbindung, pharmazeutisch verträgliches Salz davon oder pharmazeutische Zusammensetzung zu Verwendung nach Anspruch 19, wobei der Krebs aus der Gruppe ausgewählt ist, bestehend aus das Herz betreffend: Sarkom (Angiosarkom, Fibrosarkom, Rhabdomyosarkom, Liposarkom), Myxom, Rhabdomyom, Fibrom, Lipom und Teratom; Lunge: bronchogenem Karzinom (Plattenepithelkarzinom, undifferenziertem kleinzelligem, undifferenziertem großzelligem, Adenokarzinom), alveolarem (bronchiolärem) Karzinom, bronchialem Adenom, Sarkom, Lymphom, chondromatösem Hamartom, Mesotheliom; gastrointestinal: Speiseröhre (Plattenepithelkarzinom, Adenokarzinom, Leiomyosarkom, Lymphom), Magen (Karzinom, Lymphom, Leiomyosarkom), Bauchspeicheldrüse (duktalem Adenokarzinom, Insulinom, Glukagonom, Gastrinom, Karzinoidtumoren, Vipom), Dünndarm (Adenokarzinom, Lymphom, Karzinoidtumoren, Kaposi-Sarkom, Leiomyom, Hämangiom, Lipom, Neurofibrom, Fibrom), Dickdarm (Adenokarzinom, tubulärem Adenom, villösem Adenom, Hamartom, Leiomyom); Urogenitaltrakt: Niere (Adenokarzinom, Wilms-Tumor (Nephroblastom), Lymphom, Leukämie), Blase und Harnröhre (Plattenepithelkarzinom, Übergangszellkarzinom, Adenokarzinom), Prostata (Adenokarzinom, Sarkom), Hoden (Seminom, Teratom, embryonalem Karzinom, Teratokarzinom, Chorionkarzinom, Sarkom, Interstitialzellkarzinom, Fibrom, Fibroadenom, adenomatoiden Tumoren, Lipom), Leber: Hepatom (hepatozellulärem Karzinom), Cholangiokarzinom, Hepatoblastom, Angiosarkom, hepatozellulärem Adenom, Hämangiom; Gallengang: Gallenblasenkarzinom, ampullärem Karzinom, Cholangiokarzinom; Knochen: osteogenem Sarkom (Osteosarkom), Fibrosarkom, malignem fibrösem Histiozytom, Chondrosarkom, Ewing-Sarkom, malignem Lymphom (Retikulumzellsarkom), multiplem Myelom, malignem Riesenzelltumorchordom, Osteochonfrom (osteokartilaginären Exostosen), benignem Chondrom, Chondroblastom, Chondromyxofibrom, osteoidem Osteom und Risenzelltumoren; Nervensystem: Schädel (Osteom, Hämangiom, Granulom, Xanthom, Osteitis deformans), Hirnhäute (Meningeom, Meningiosarkom, Gliomatose), Gehirn (Astrozytom, Medulloblastom, Gliom, Ependymom, Germinom (Pinealom), Glioblastom multiforme, Oligodendrogliom, Schwannom, Retinoblastom, angeborenen Tumoren), Neurofibrom, Meningeom, Gliom, Sarkom des Rückenmarks); gynäkologisch: Uterus (Endometriumkarzinom (serösem Zystadenokarzinom, muzinösem Zystadenokarzinom, nicht klassifiziertem Karzinom), Granulosazelltumoren, Sertoli-Leydig-Zelltumoren, Dysgerminom, malignem Teratom), Vulva (Plattenepithelkarzinom, intraepithelialem Karzinom, Adenokarzinom, Fibrosarkom, Melanom), Vagina (Klarzellkarzinom, Plattenepithelkarzinom, botryoidem Sarkom (embryonalem Rhabdomyosarkom), Eileiter (Karzinom); hämatologisch: Blut (myeloischer Leukämie (akut und chronisch), akuter lymphoblastischer Leukämie, chronischer lymphatischer Leukämie, myeloproliferativen Krankheiten, multiplem Myelom, myelodysplastischem Syndrom), Hodgkin-Krankheit, Non-Hodgkin-Lymphom (malignem Lymphom); Haut: malignem Melanom, Basalzellkarzinom, Plattenepithelkarzinom, Kaposi-Sarkom, dysplastischen Nävi, Lipom, Angiom, Dermatofibrom, Keloiden, Psoriasis; und Nebennieren: Neuroblastom.

21. Verbindung, pharmazeutisch verträgliches Salz davon oder pharmazeutische Zusammensetzung zur Verwendung nach Anspruch 19, wobei der Krebs ein KRas-G12D-assoziierter Krebs ist.

22. Verbindung, pharmazeutisch verträgliches Salz davon oder pharmazeutische Zusammensetzung zur Verwendung nach Anspruch 19, wobei der Krebs nicht kleinzelliger Lungenkrebs, kleinzelliger Lungenkrebs, Kolorektalkrebs, Rektumkrebs oder Bauchspeicheldrüsenkrebs ist.

23. Verbindung, pharmazeutisch verträgliches Salz davon oder pharmazeutische Zusammensetzung zur Verwendung nach Anspruch 19, wobei die therapeutisch wirksame Menge der Verbindung zwischen etwa 0,01 bis 100 mg/kg pro Tag beträgt, wobei die therapeutisch wirksame Menge der Verbindung optional zwischen etwa 0,1 bis 50 mg/kg pro Tag beträgt.

24. Verbindung nach einem der Ansprüche 1-16 oder ein pharmazeutisch verträgliches Salz davon oder die pharmazeutische Zusammensetzung nach Anspruch 17 zur Verwendung in einem Verfahren zum Behandeln von Krebs bei einem Patienten, der dies benötigt, wobei das Verfahren (a) Bestimmen, dass der Krebs mit einer KRas-G12D-Mutation assoziiert ist (z. B. ein KRas-G12D-assoziierter Krebs); und (b) Verabreichen einer therapeutisch wirksamen Menge der Verbindung oder des pharmazeutisch verträglichen Salzes davon oder der pharmazeutischen Zusammensetzung an den Patienten umfasst.

## Revendications

1. Composé de Formule (I) : 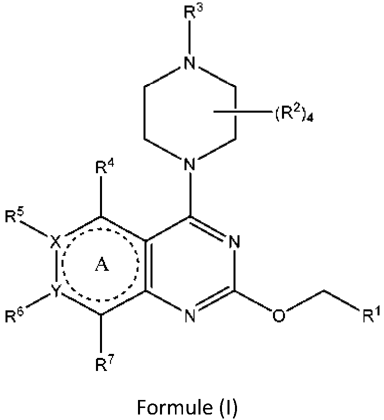 ou sel pharmaceutiquement acceptable de celui-ci, dans lequel :
A est un phényle, hétéroaryle ou hétérocyclyle ;
X est un N, C ou CH ;
Y est un N, C ou CH ;
dans lequel, si X est N, alors Y n'est pas N, et dans lequel si Y est N, alors X n'est pas N ; 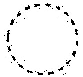
représente une ou plusieurs doubles liaisons éventuellement présentes ;
R¹ est un hétérocyclyle, éventuellement substitué par un ou plusieurs substituants choisis indépendamment parmi un : hydroxy, halogène, haloalkyle en C1-C3, alcoxy en C1-C3 et cyano ;
deux groupes R² sont chacun indépendamment un hydrogène, un hydroxy, un halogène, un haloalkyle en C1-C3, un alkyle en C1-C3, un alcoxy en C1-C3, un (alcoxy)-alkyle en C1-C3, un alkyl en C1-C3-N(R⁸)₂, un cyano, un cyanoalkyle en C1-C3, un cyanoalcényle en C2 - C4, un hydroxyalkyle en C1 - C3, un HC(=O)-, un -CO₂R⁸, ou - CO²N(R⁸)₂, dans lequel R⁸ est un hydrogène ou un groupe alkyle en C1-C3, et
deux groupes R² sont un hydrogène, deux groupes R² portés par le même atome de carbone se lient pour former un cycle cycloalkyle, ou deux groupes R² se lient pour former un pont éthylène afin de former un système cyclique [3.2.1] ou [2.2.2], dans lequel ledit pont éthylène forme un système cyclique [2.2.2] si X est N et le cycle A est un hétéroaryle, et dans lequel ledit pont éthylène forme un système cyclique [2.2.2] si Y est N et le cycle A est un hétérocyclyle ;
R³ est un hydrogène ou un alkyle en C1-C3 éventuellement substitué par un ou plusieurs substituants choisis indépendamment parmi un : halogène, alcoxy en C1-C3 et cyano ;
R⁴ est un hydrogène ;
R⁵ est absent ou est choisi parmi l'hydrogène, l'halogène, le -O-phényle et le -O-pyridyle, dans lequel ledit phényle et ledit pyridyle sont éventuellement substitués par un ou plusieurs substituants choisis indépendamment parmi un : hydroxy, halogène, haloalkyle en C1-C3, alkyle en C1-C3, alcoxy en C1-C3 et cyano ;
R⁶ est un phényle, un naphtyle ou un indazolyle, éventuellement substitué par un ou plusieurs substituants choisis indépendamment parmi un : hydroxy, halogène, haloalkyle en C1-C3, alkyle en C1-C3, alcoxy en C1-C3 et cyano ; et
R⁷ représente un hydrogène ou un halogène.

2. Composé ou sel selon la revendication 1, dans lequel la formule (I) est représentée par la formule (I-A) : 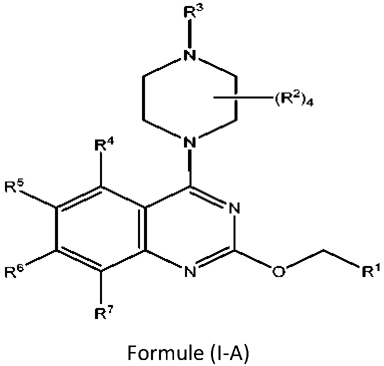
dans lequel R⁵ doit être présent, ou dans lequel la formule (I) est représentée par la formule (I-B) : 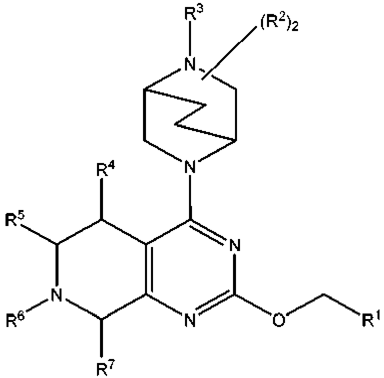 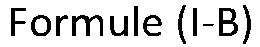
dans laquelle R⁵ doit être présent.

3. Composé ou sel selon la revendication 1, dans lequel R¹ est un hexahydro-1H-pyrrolizinyle éventuellement substitué par un ou plusieurs substituants choisis indépendamment parmi un : hydroxy, halogène, haloalkyle en C1-C3, alcoxy en C1-C3 et cyano, éventuellement dans lequel ledit hexahydro-1H-pyrrolizinyle est éventuellement substitué par un halogène, éventuellement dans lequel R¹ est un 2-fluorohexahydro-1H-pyrrolizinyle.

4. Composé ou sel selon la revendication 1, dans lequel R¹ hétérocyclyle est une pyrrolidine.

5. Composé ou sel selon la revendication 1, dans lequel deux R² représentent des atomes d'hydrogène, et deux R² se lient pour former un pont éthylène afin de former un système cyclique [3.2.1].

6. Composé ou sel selon la revendication 1, dans lequel deux R² représentent des atomes d'hydrogène, et deux R² se lient pour former un pont éthylène afin de former un système cyclique [2.2.2].

7. Composé ou sel selon la revendication 1, dans lequel deux R² représentent des atomes d'hydrogène et deux R² se lient pour former un pont alcylène afin de former un système cyclique [3.2.1] ou [2.2.2].

8. Composé ou sel selon la revendication 6, dans lequel X est un N et A est un hétéroaryle.

9. Composé ou sel selon la revendication 6, dans lequel Y est un N et le cycle A est un hétérocyclyle.

10. Composé ou sel selon la revendication 7, dans lequel X et Y sont tous deux un C ou CH.

11. Composé ou sel selon la revendication 1, dans lequel R³ est un hydrogène ou un alkyle en C1-C3 substitué par un halogène.

12. Composé ou sel selon la revendication 1, dans lequel R⁵ est absent ; un halogène ; un -O-phényle dans lequel le phényle est éventuellement substitué par un ou plusieurs substituants choisis indépendamment parmi un : halogène, alcoxy en C1-C3 et cyano ; ou un -O-pyridyle.

13. Composé ou sel selon la revendication 1, dans lequel R⁶ est un phényle éventuellement substitué par un ou plusieurs substituants choisis indépendamment parmi un : hydroxy et halogène ; un naphtyle éventuellement substitué par un halogène, un hydroxy ou à la fois un halogène et un hydroxy ; ou un indazolyle éventuellement substitué par un ou plusieurs alkyles en C1-C3.

14. Composé ou sel selon la revendication 1, dans lequel R⁷ est un hydrogène ou un halogène.

15. Composé selon la revendication 1 choisi parmi : 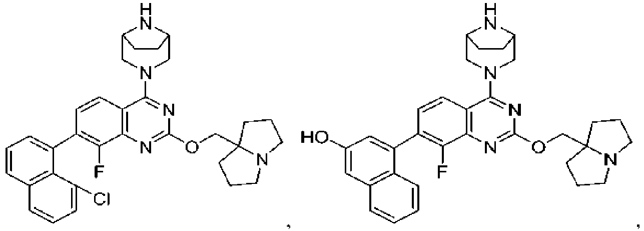 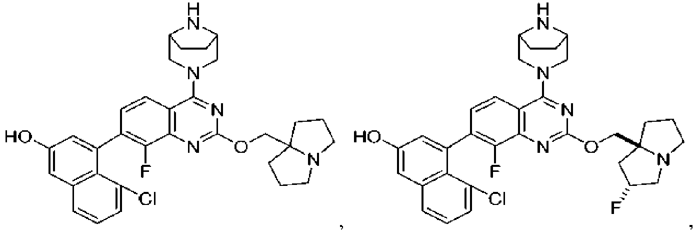 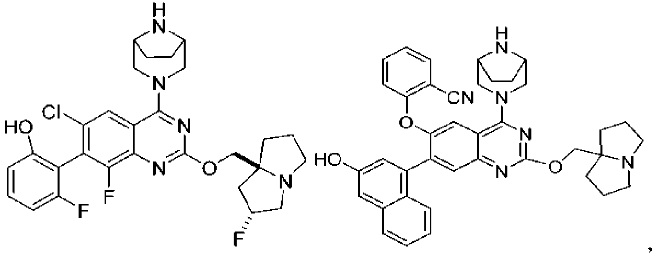 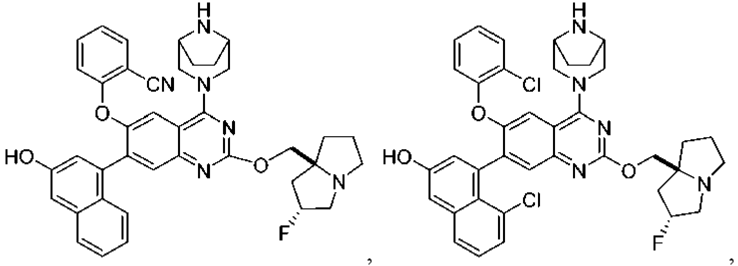 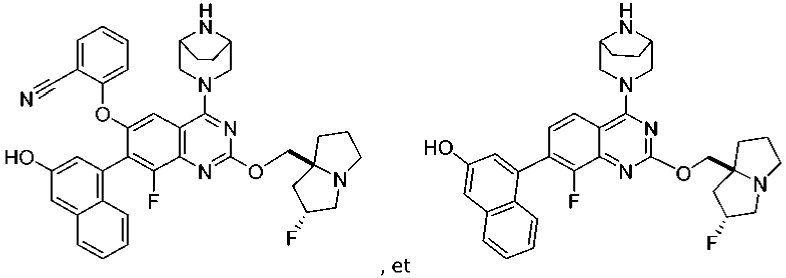 ou sel pharmaceutiquement acceptable de celui-ci.

16. Composé sélectionné parmi 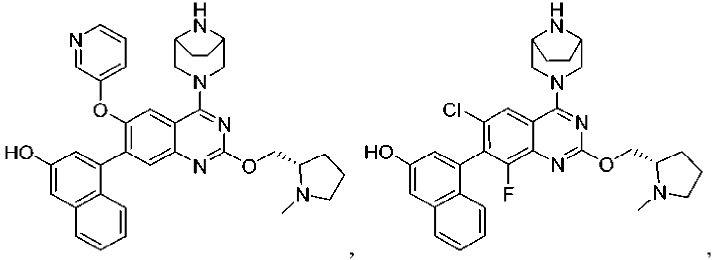 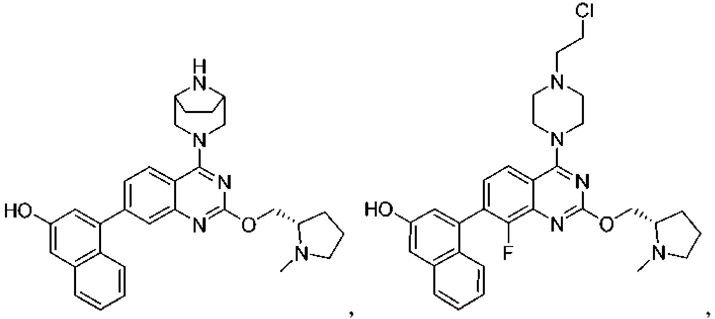 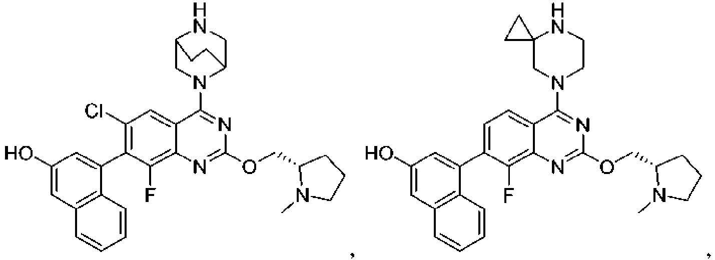 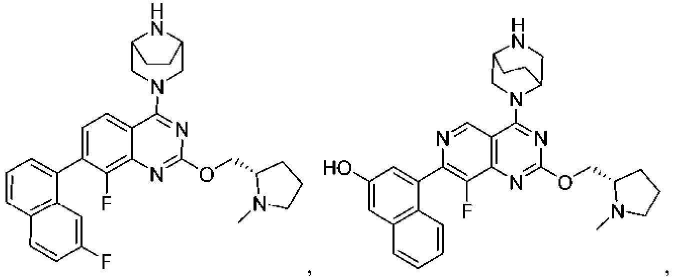 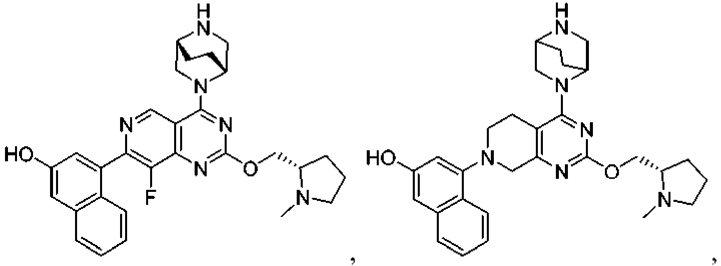 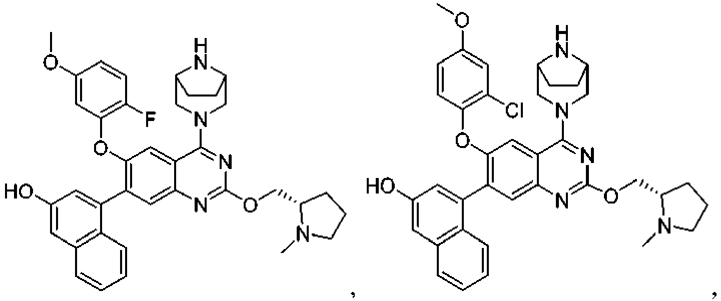 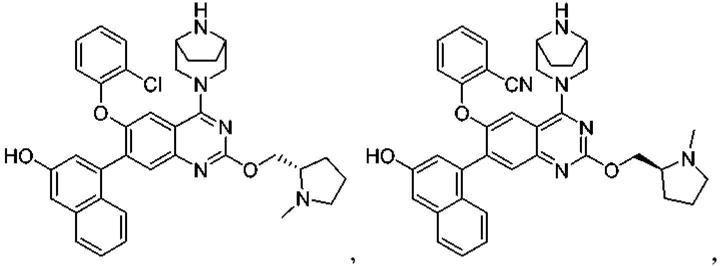 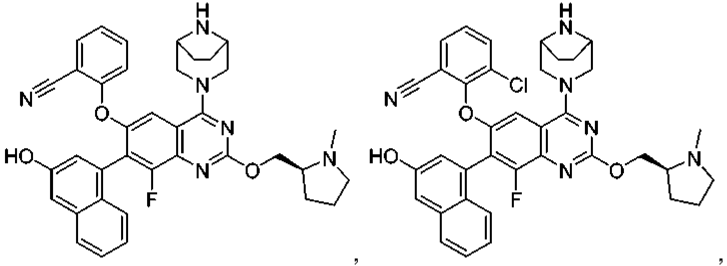 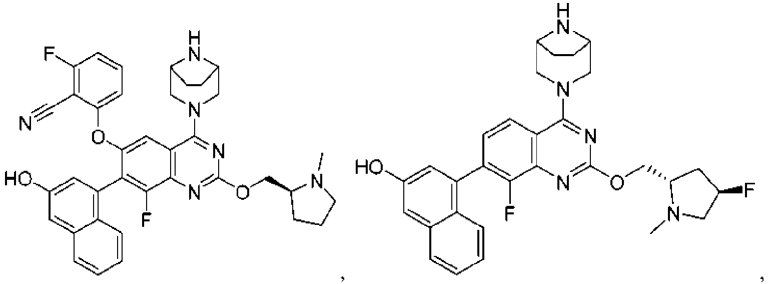 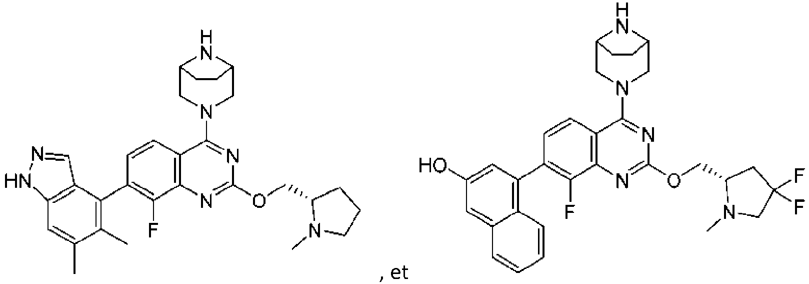 ou sel pharmaceutiquement acceptable de celui-ci.

17. Composition pharmaceutique, comprenant une quantité thérapeutiquement efficace d'un composé selon l'une quelconque des revendications 1 à 16 ou un sel ou solvate pharmaceutiquement acceptable de celui-ci, et un excipient pharmaceutiquement acceptable.

18. Procédé in vitro d'inhibition de l'activité KRas G12D dans une cellule, comprenant la mise en contact de la cellule dans laquelle l'inhibition de l'activité KRas G12D est souhaitée avec une quantité efficace d'un composé selon l'une quelconque des revendications 1 à 16 ou un sel pharmaceutiquement acceptable de celui-ci, ou de la composition pharmaceutique selon la revendication 17.

19. Composé selon l'une quelconque des revendications 1 à 16 ou un sel pharmaceutiquement acceptable de celui-ci, ou composition pharmaceutique selon la revendication 17 pour une utilisation dans un procédé de traitement du cancer comprenant l'administration à un patient atteint de cancer d'une quantité thérapeutiquement efficace du composé ou d'un sel pharmaceutiquement acceptable de celui-ci, ou de la composition pharmaceutique.

20. Composé, sel pharmaceutiquement acceptable de celui-ci, ou composition pharmaceutique pour une utilisation selon la revendication 19, dans lequel le cancer est sélectionné dans le groupe consistant d'un cancer cardiaque : sarcome (angiosarcome, fibrosarcome, rhabdomyosarcome, liposarcome), un myxome, un rhadomyome, un fibrome, un lipome et un tératome ; un carcinome bronchogénique (épidermoïde, à petites cellules indifférencié, à grandes cellules indifférencié, adénocarcinome), un carcinome alvéolaire (bronchiolaire), un adénome bronchique, un sarcome, un lymphome, un hémartome chrondromateux, un mésothéliome ; un cancer gastrointestinal : de l'œsophage (carcinome épidermoïde, un adénocarcinome, un léiomyosarcome, un lymphome), un cancer de l'estomac, (carcinome, lymphome, léiomyosarcome), un cancer du pancréas (un adénocarcinome canalaire, un insulinome, un glucagonome, un gastrinome, une tumeur carcinoïde, un vipome), un cancer de l'intestin grêle (adénocarcinome, lymphome, tumeur carcinoïde, sarcome de Kaposi, léiomyome, hémangiome, lipome, neurofibrome, fibrome), un cancer du colon (adécarcinome, adénome tubulaire, adénome villeux, hamartome, léiomyome) ; cancer des voies génito-urinaires : un cancer du rein (adénocarcinome, une tumeur de Wilms (néphroblastome), un lymphome, une leucémie), un cancer de la vessie et de l'urètre (carcinome épidermoïde, carcinome transitionnel, adénocarcinome), un cancer de la prostate (adénocarcinome, sarcome), un cancer des testicules (séminome, tératome, carcinome embryonnaire, tératocarcinome, choriocarcinome, sarcome du testicule, carcinome interstitiel, fibrome, fibroadénome, tumeurs adénomatoïdes, lipome) ; un cancer du foie : hépatome (carcinome hépatocellulaire), cholangiocarcinome, hépatoblastome, angiosarcome, adénome hépatocellulaire, hémangiome ; voies biliaires : carcinome de la vésicule biliaires, carcinome ampullaire, cholangiocarcinome ; cancer des os : sarcome ostéogénique (ostéosarcome), fibrosarcome, histiocytome fibreux mailn, chrondrosarcome, sarcome d'Ewing, lymphome malin (sarcome à cellules réticulaires), myélome multiple, chordome à tumeur cellulaire géante maline, ostéochonfrome (exostoses cartilagineuses), chrondome bénin, chondroblastome, chondromyxofibrome, ostéome ostéoïde et tumeurs cellulaires génates ; cancer du système nerveux : crâne (ostéome, hémangiome, granulome, xanthome, une ostéite déformante), des méninges (méningiome, méningiosarcome, gliomatose), du cerveau (astrocytome, médulloblastome, gliome, épendymome, germinome (pinéalome), glioblastome multiforme, oligodendrogliome, schwannome, rétinoblastome, tumeurs congénitales), un neurofibrome de la moelle épinière, méningiome, gliome, sarcome) ; cancer gynécologique : de l'utérus (carcinome de l'endomètre (cystadénocarcinome séreux, cystadénocarcinome mucineux, carcinome non classé), des tumeurs à cellules de la granulosa-thécale, des tumeurs à cellules de Sertoli-Leydig, un dysgerminome, un tératome malin), du vagin (carcinome à cellules claires, carcinome épidermoïde, carcinome, sarcome botryoïde (rhabdomyosarcome embryonnaire), un cancer des trompes de Fallope (carcinome) ; Cancer hématologique : du sang (leucémie myéloïde (aiguë et chronique), leucémie lymphoblastique aiguë, leucémie lymphoïde chronique, maladies myéloprolifératives, myélome multiple, syndrome myélodysplasique), maladie de Hodgkin, lymphome non-Hodgkinien (lymphome malin) ; cancer de la peau : mélanome malin, carcinome des cellules basales, carcinome des cellules squameuses, sarcome de Kaposi, naevus dysplasiques, lipome, angiome, dermatofibrome, chéloïdes, psoriasis ; et cancer des glandes surrénales : neuroblastome.

21. Composé, son sel pharmaceutiquement acceptable ou composition pharmaceutique à utiliser selon la revendication 19, dans lequel le cancer est un cancer associé à KRas G12D.

22. Composé ou sel pharmaceutiquement acceptable de celui-ci ou composition pharmaceutique destiné à être utilisé selon la revendication 19, dans lequel le cancer est un cancer du poumon non à petites cellules, un cancer du poumon à petites cellules, un cancer colorectal, un cancer rectal ou un cancer du pancréas.

23. Composé, son sel pharmaceutiquement acceptable ou composition pharmaceutique à utiliser selon la revendication 19, dans lequel la quantité thérapeutiquement efficace du composé est comprise entre environ 0,01 et 100 mg/kg par jour, éventuellement dans lequel la quantité thérapeutiquement efficace du composé est comprise entre environ 0,1 et 50 mg/kg par jour.

24. Composé selon l'une quelconque des revendications 1 à 16 ou sel pharmaceutiquement acceptable de celui-ci, ou composition pharmaceutique selon la revendication 17 pour une utilisation dans un procédé de traitement du cancer chez un patient qui en a besoin, le procédé comprenant (a) la détermination que le cancer est associé à une mutation KRas G12D (par exemple, un cancer associé à KRas G12D) ; et (b) l'administration au patient d'une quantité thérapeutiquement efficace du composé ou du sel pharmaceutiquement acceptable de celui-ci, ou de la composition pharmaceutique.
